# EUROPEAN PATENT APPLICATION

(11) **EP 4 717 089 A2**
(43) Date of publication of application: **01.04.2026**
(21) Application number: 26152348.4
(22) Date of filing: 28.07.2022
(51) Int. Cl.: A01P 1/00

(54) **MICROBIOCIDAL PYRAZOLE DERIVATIVES**

(30) Priority: 02.08.2021 EP 21189099; 02.02.2022 EP 22154772
(62) Divisional of application: 22757954.7
(71) Applicant: Syngenta Crop Protection AG, 4058 Basel (CH)
(72) Inventor: EDMUNDS, Andrew, 4332 Stein (CH); SCARBOROUGH, Christopher Charles, 4332 Stein (CH); WILLIAMS, Simon, 4332 Stein (CH); JUNG, Pierre Joseph Marcel, 4332 Stein (CH); FINKBEINER, Peter, 4332 Stein (CH); POULIOT, Martin, 4332 Stein (CH); GROSHEVA, Daria, 4332 Stein (CH); SUESSE, Lars, 4332 Stein (CH); BRUNOT, Guillaume, 4332 Stein (CH); BONVALOT, Damien, 4332 Stein (CH)
(74) Representative: SYNGENTA IP

(57) **Abstract**

A compound of formula (I): 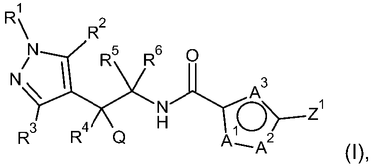 wherein the substituents are as defined in claim 1, and the agrochemically acceptable salts, stereoisomers, enantiomers, tautomers and N-oxides of those compounds, which can be used as fungicides.

## Description

The present invention relates to microbiocidal pyrazole derivatives, e.g. as active ingredients, which have microbiocidal activity, in particular fungicidal activity. The invention also relates to preparation of these pyrazole derivatives, to intermediates useful in the preparation of these pyrazole derivatives, to the preparation of these intermediates, to agrochemical compositions which comprise at least one of the pyrazole derivatives, to preparation of these compositions and to the use of the pyrazole derivatives or compositions in agriculture or horticulture for controlling or preventing infestation of plants, harvested food crops, seeds or non-living materials by phytopathogenic microorganisms, in particular fungi.

According to a first aspect of the present invention, there is provided a compound of formula (I): 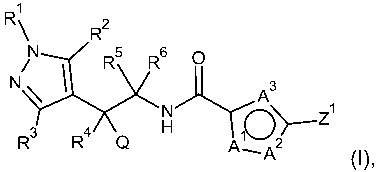 wherein
R¹ is selected from the group consisting of hydrogen, C₁-C₄ alkyl, C₂-C₄ alkenyl, C₂-C₄ alkynyl and C₃-C₆ cycloalkyl;
R² is selected from the group consisting of hydrogen, halogen, C₁-C₄ alkyl, C₂-C₄ alkenyl, C₂-C₄ alkynyl, C₁-C₄ haloalkyl, C₃-C₆ cycloalkyl, C₁-C₄ alkylcarbonyl, N-C₁-C₄ alkoxy-C₁-C₄ alkyl-carbonimidoyl, N-hydroxy-C₁-C₄ alkyl-carbonimidoyl and C₁-C₄ alkoxycarbonyl;
R³ is selected from the group consisting of hydrogen, halogen and C₁-C₄ alkyl;
R⁴ is selected from the group consisting of hydrogen, halogen, C₁-C₄ alkyl, cyano, C₁-C₄ alkylcarbonyl, C₁-C₄ alkoxycarbonyl, C₁-C₄ alkylaminocarbonyl and di(C₁-C₄ alkylamino)carbonyl;
R⁵ and R⁶ are independently selected from the group consisting of hydrogen and C₁-C₄ alkyl;
A¹, A² and A³ are independently selected from the group consisting of CR⁷, N, NR⁸, O and S, with the proviso that at least one of A¹, A² and A³ is selected from N, O and S, and that no more than one of A¹, A² and A³ is O or S;
R⁷ and R⁸ are independently selected from the group consisting of hydrogen, C₁-C₄ alkyl, C₂-C₄ alkenyl and C₂-C₄ alkynyl;
Q is selected from the group consisting of Q¹, Q², Q³, Q⁴ and Q⁵: 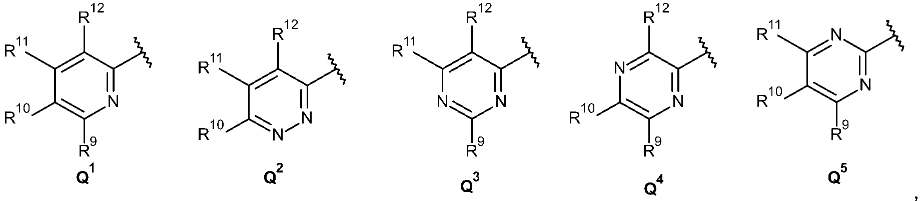
wherein
R⁹, R¹⁰, R¹¹ and R¹² are independently selected from the group consisting of hydrogen, halogen, amino, hydroxy, carboxylic acid, cyano, C₁-C₄ alkyl, C₁-C₄ haloalkyl, C₁-C₄ alkoxy, C₁-C₄ haloalkoxy, C₂-C₄ alkenyloxy, C₂-C₄ alkynyloxy, C₁-C₄ alkylsulfanyl, C₁-C₄ alkylsulfinyl, C₁-C₄ alkylsulfonyl, C₁-C₄ alkoxy-C₁-C₄ alkyl, N-C₁₋₄alkylamino, N,N-diC₁₋₄alkylamino, C₁-C₆ alkoxycarbonyl, C₁-C₄ alkylcarbonyl, N-C₁-C₄ alkoxy-C₁-C₄alkyl-carbonimidoyl, N-hydroxy-C₁-C₄alkyl-carbonimidoyl, C₁-C₄alkylaminocarbonyl, di(C₁-C₄ alkylamino)carbonyl), C₁-C₄ alkylcarbonylamino, C₁-C₄ alkylsulfonylamino, trifluoromethylsulfonyloxy, phenyl, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl and C₁-C₄ alkoxy; and
Z¹ is selected from the group consisting of C₁-C₄ alkyl, phenyl, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl, C₁-C₄ alkoxy, C₁-C₄ haloalkoxy, C₁-C₄ alkylsulfanyl, C₁-C₄ alkylsulfinyl, C₁-C₄ alkylsulfonyl and C₂-C₄ alkynyl;
or an agrochemically acceptable salt, stereoisomer, enantiomer, tautomer or N-oxide thereof.

Surprisingly, it has been found that the compounds of formula (I) have, for practical purposes, a very advantageous level of biological activity for protecting plants against diseases that are caused by fungi.

According to a second aspect of the invention, there is provided an agrochemical composition comprising a fungicidally effective amount of a compound of formula (I) according to the invention. Such an agricultural composition may further comprise at least one additional active ingredient and/or an agrochemically-acceptable diluent or carrier.

According to a third aspect of the invention, there is provided a method of controlling or preventing infestation of useful plants by phytopathogenic microorganisms, wherein a fungicidally effective amount of a compound of formula (I) according to the invention, or a composition comprising the compound of formula (I), is applied to the plants, to parts thereof or the locus thereof.

According to a fourth aspect of the invention, there is provided the use of a compound of formula (I) according to the invention as a fungicide. According to this particular aspect of the invention, the use may exclude methods for the treatment of the human or animal body by surgery or therapy.

As used herein, the term "halogen" or "halo" refers to fluorine (fluoro), chlorine (chloro), bromine (bromo) or iodine (iodo), preferably fluorine, chlorine or bromine.

As used herein, amino means a -NH₂ group.

As used herein, cyano means a -CN group.

As used herein, the term "hydroxyl" or "hydroxy" means an -OH group.

As used herein, the term "carboxylic acid" means a -COOH group.

As used herein, oxo means an =O group, e.g., sulfinyl (-S(O)-) or sulfonyl (-S(O)₂-) oxygen.

As used herein, the term "C₁-C₄ alkyl" refers to a straight or branched hydrocarbon chain radical consisting solely of carbon and hydrogen atoms, containing no unsaturation, having from one to four carbon atoms, and which is attached to the rest of the molecule by a single bond. The terms "C₁-C₃ alkyl" and "C₁-C₂ alkyl" are to be construed accordingly. Examples of C₁-C₄alkyl include, but are not limited to, methyl, ethyl, *n-*propyl, 1-methylethyl (isopropyl), *n*-butyl, and 1,1-dimethylethyl (*t*-butyl). A "C₁-C₄ alkylene" group refers to the corresponding definition of C₁-C₄alkyl, except that such radical is attached to the rest of the molecule by two single bonds. Examples of C₁-C₄ alkylene, are -CH₂- and -CH₂CH₂-.

As used herein, the term "C₂-C₄ alkenyl" refers to a straight or branched hydrocarbon chain radical group consisting solely of carbon and hydrogen atoms, containing at least one double bond that can be of either the (E) or (Z) configuration, having from two to four carbon atoms, which is attached to the rest of the molecule by a single bond. The term "C₃-C₄ alkenyl" is to be construed accordingly. Examples of C₂-C₄ alkenyl include, but are not limited to, ethenyl and prop-1-enyl.

As used herein, the term "C₂-C₄ alkynyl" refers to a straight or branched hydrocarbon chain radical group consisting solely of carbon and hydrogen atoms, containing at least one triple bond, having from two to four carbon atoms, and which is attached to the rest of the molecule by a single bond. The term "C₃-C₄ alkynyl" is to be construed accordingly. Examples of C₃-C₄ alkynyl include, but are not limited to, ethynyl, prop-1-ynyl, propargyl (prop-2-ynyl), but-1-ynyl and 3-methyl-but-1-ynyl.

As used herein, the term "C₁-C₄ haloalkyl" refers to a C₁-C₄alkyl radical as generally defined above substituted by one or more of the same or different halogen atoms. Examples of C₁-C₄ haloalkyl include, but are not limited to fluoromethyl, fluoroethyl, chloroethyl, difluoromethyl, dichloroethyl, trifluoromethyl, fluoropropyl, chloropropyl, difluoropropyl, dichloropropyl, trifluoropropyl, trichloropropyl, 2,2-difluoroethyl, 2,2-dichloroethyl, 2,2,2-trifluoroethyl, and 3,3,3-trifluoropropyl.

As used herein, the term "C₁-C₄ alkoxy" refers to a radical of the formula RₐO- where Rₐ is a C₁-C₄ alkyl radical as generally defined above. The terms "C₁-C₃ alkoxy" and "C₁-C₂ alkoxy" are to be construed accordingly. Examples of C₁-C₄ alkoxy include, but are not limited to, methoxy, ethoxy, propoxy, iso-propoxy, and *t*-butoxy.

As used herein, the term "C₁-C₄ alkoxy-C₁-C₄ alkyl" refers to radical of the formula R_{b}-O-Rₐ- where R_{b} is a C₁-C₄ alkyl radical as generally defined above, and Rₐ is a C₁-C₄ alkylene radical as generally defined above.

As used herein, the term "C₁-C₄ alkylcarbonyl" refers to a radical of the formula -C(O)Rₐ where Rₐ is a C₁-C₄ alkyl radical as generally defined above.

As used herein, the term "C₁-C₄ alkoxycarbonyl" refers to a radical of the formula -C(O)ORₐ where Rₐ is a C₁-C₄ alkyl radical as generally defined above.

As used herein, the term "N-C₁-C₄alkylamino" refers to a radical of the formula -NH-Rₐ where Rₐ is a C₁-C₄ alkyl radical as defined above.

As used herein, the term "N,N-diC₁-C₄alkylamino" refers to a radical of the formula -N(Rₐ)Rₐ where each Rₐ is a C₁-C₄ alkyl radical, which may be the same or different, as defined above.

As used herein, the term "C₁-C₄ alkylaminocarbonyl" refers to a radical of the formula -C(O)NHRₐ where Rₐ is a C₁-C₄alkyl radical as generally defined above.

As used herein, the term "di(C₁-C₄ alkylamino)carbonyl" refers to a radical of the formula -C(O)NRₐ(Rₐ) where each Rₐ is a C₁-C₄alkyl radical, which may be the same or different, as generally defined above.

As used herein, the term "C₁-C₄ alkylsulfonylamino" refers to a radical of the formula -NH-S(O)₂Rₐ where Rₐ is a C₁-C₄alkyl radical as generally defined above.

As used herein, the term "C₂-C₄ alkenyloxy" refers to a radical of the formula -ORₐ where Rₐ is a C₂-C₄ alkenyl radical as generally defined above.

As used herein, the term "C₂-C₄ alkynyloxy" refers to a radical of the formula -ORₐ where Rₐ is a C₂-C₄ alkynyl radical as generally defined above.

As used herein, the term "N-C₁-C₄ alkoxy-C-C₁-C₄ alkyl-carbonimidoyl" refers to a radical of the formula - C(Rₐ)=NO(R_{b}) where Rₐ is a C₁-C₄ alkyl radical as generally defined above, and R_{b} is a C₁-C₄ alkyl radical as generally defined above.

As used herein the term "N-hydroxy-C-C₁-C₄ alkyl-carbonimidoyl" refers to a radical of the formula - C(Rₐ)=NOH where Rₐ is a C₁-C₄ alkyl radical as generally defined above.

As used herein, the term "C₃-C₆ cycloalkyl" refers to a stable, monocyclic ring radical which is saturated or partially unsaturated and contains 3 to 6 carbon atoms. The terms "C₃-C₄ cycloalkyl" and "C₃-C₅ cycloalkyl" are to be construed accordingly. Examples of C₃-C₆ cycloalkyl include, but are not limited to, cyclopropyl, cyclobutyl, cyclopentyl, cyclohexyl, cyclopenten-1-yl, cyclopenten-3-yl, and cyclohexen-3-yl.

As used herein, the term "C₃-C₆ cycloalkylC₁-C₄alkyl" refers to a C₃-C₆ cycloalkyl ring as defined above attached to the rest of the molecule by a C₁-C₄alkylene radical as defined above. Examples of C₃-C₆ cycloalkylC₁-C₄alkyl include, but are not limited to, cyclopropyl-methyl, cyclobutyl-ethyl, and cyclopentylmethyl.

Examples of a 5- or 6-membered heteroaryl ring, which comprise 1 to 4 heteroatoms independently selected from nitrogen, oxygen and sulfur, include, but are not limited to, pyridyl, pyrimidyl, pyrrolyl, pyrazolyl, furyl, thienyl, imidazolyl, isoxazolyl, oxazolyl, thiazolyl, isothiazolyl, triazolyl, oxadiazolyl, thiadiazolyl, tetrazolyl, pyrazinyl, pyridazinyl and triazinyl.

Where substituents are indicated as being "optionally substituted", this means that they may or may not carry one or more identical or different substituents, e.g., one, two or three R^{x} substituents. For example, C₁-C₆alkyl substituted by 1, 2 or 3 halogens, may include, but not be limited to, -CH₂Cl, -CHCl₂, -CCl₃, - CH₂F, -CHF₂, -CF₃, -CH₂CF₃ or -CF₂CH₃ groups. As another example, C₁-C₆alkoxy substituted by 1, 2 or 3 halogens, may include, but not be limited to, CH₂ClO-, CHCl₂O-, CCl₃O-, CH₂FO-, CHF₂O-, CF₃O-, CF₃CH₂O- or CH₃CF₂O- groups.

The compounds of formula (I) or the intermediate compounds of formula (III) according to the invention, which have at least one basic centre, can form, for example, acid addition salts, for example with strong inorganic acids such as mineral acids, for example perchloric acid, sulfuric acid, nitric acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄-alkane- or arylsulfonic acids which are unsubstituted or substituted, for example by halogen, for example methane- or p-toluenesulfonic acid.

The compounds of formula (I) or the intermediate compounds of formula (III) according to the invention, which have at least one acidic group, can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

The presence of one or more possible asymmetric carbon atoms in a compound of formula (I) according to the invention means that the compounds may occur in chiral isomeric forms, i.e., enantiomeric or diastereomeric forms. Also, atropisomers may occur as a result of restricted rotation about a single bond. Formula (I) is intended to include all those possible isomeric forms and mixtures thereof. The present invention includes all those possible isomeric forms and mixtures thereof for a compound of formula (I) according to the invention. Likewise, a compound of formula (I) is intended to include all possible tautomers (including lactam-lactim tautomerism and keto-enol tautomerism) where present. The present invention includes all possible tautomeric forms for a compound of formula (I) according to the invention.

In each case, the compounds of formula (I) according to the invention are in free form, in oxidized form as an N-oxide, in covalently hydrated form, or in salt form, e.g., an agronomically usable or agrochemically acceptable salt form. N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991. N-oxides can be prepared by reacting a compound of formula (I) with a suitable oxidizing agent, for example the H₂O₂/urea adduct, in the presence of an acid anhydride, e.g. trifluoroacetic anhydride. Such oxidations are known from the literature, for example from J. Med. Chem. 1989, 32 (12), 2561-73, or WO2000/15615. The compounds of formula (I) according to the invention also include hydrates, which may be formed during salt formation.

The following list provides definitions, including preferred definitions, for substituents R¹, R², R³, R⁴, R⁵, R⁶, R⁷, R⁸, R⁹, R¹⁰, R¹¹, R¹², A¹, A², A³, Q (Q¹, Q², Q³, Q⁴ and Q⁵) and Z¹ with reference to the compounds of formula (I) of the present invention. For any one of these substituents, any of the definitions given below may be combined with any definition of any other substituent given below or elsewhere in this document.

In an embodiment of the invention, R¹ is C₁-C₄ alkyl. Preferably, R¹ is methyl, ethyl or isopropyl. More preferably, R¹ is methyl.

In an embodiment of the invention, R² is selected from the group consisting of hydrogen, halogen, C₁-C₄ alkyl, C₃-C₆ cycloalkyl, C₁-C₄ alkylcarbonyl, N-C₁-C₄ alkoxy-C₁-C₄ alkyl-carbonimidoyl and N-hydroxy-C₁-C₄ alkyl-carbonimidoyl. Preferably, R² is selected from the group consisting of hydrogen, halogen, methyl, ethyl, cyclopropyl, C₁-C₂ alkylcarbonyl, N-C₁-C₂ alkoxy-C₁-C₂ alkyl-carbonimidoyl and N-hydroxy-C₁-C₂ alkyl-carbonimidoyl. More preferably, R² is selected from the group consisting of hydrogen, fluorine, chlorine, bromine, methyl, ethyl, cyclopropyl, acetyl, -C(CH₃)=NOCH₃, -C(CH₃)=NOCH₂CH₃ and - C(CH₃)=NOH. Even more preferably, R² is selected from the group consisting of hydrogen, fluorine, chlorine and methyl.

In an embodiment of the invention, R³ is selected from the group consisting of hydrogen, fluorine, chlorine and methyl. More preferably, R³ is hydrogen.

In an embodiment of the invention, R⁴ is selected from the group consisting of hydrogen, halogen, C₁-C₄ alkyl, cyano and C₁-C₄ alkoxycarbonyl. Preferably, R⁴ is selected from the group consisting of hydrogen, chlorine, fluorine, methyl, ethyl, isopropyl, cyano and -CO₂Me. More preferably R⁴ is selected from the group consisting of hydrogen, cyano or C₁-C₄ alkyl. Even more preferably, R⁴ is selected from the group consisting of hydrogen, methyl, ethyl, isopropyl and cyano. Even more preferably, R⁴ is hydrogen or methyl.

In an embodiment of the invention, R⁵ and R⁶ are independently selected from the group consisting of hydrogen, methyl and ethyl. Preferably, R⁵ and R⁶ are independently selected from the group consisting of hydrogen and methyl. More preferably, R⁵ and R⁶ are hydrogen.

In an embodiment of the invention, Q is selected from the group consisting of Q¹, Q², Q³ and Q⁴: 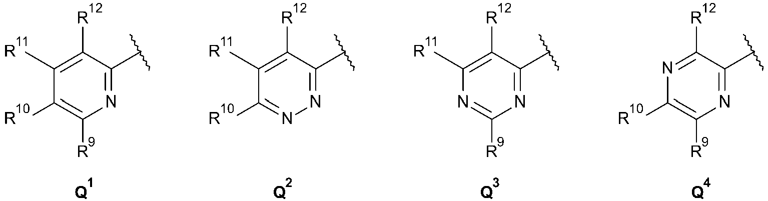 wherein R⁹, R¹⁰, R¹¹ and R¹² are as defined above.

Preferably, Q is selected from the group consisting of Q¹, Q² and Q³: 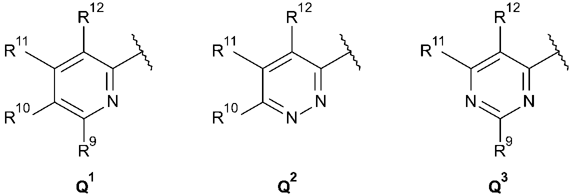 wherein R⁹, R¹⁰, R¹¹ and R¹² are as defined above.

More preferably, Q is Q¹: 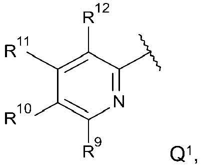 wherein R⁹, R¹⁰, R¹¹ and R¹² are as defined above.

In an embodiment of the invention, R⁹ and R¹⁰ are independently selected from the group consisting of hydrogen, halogen, amino, hydroxy, carboxylic acid, cyano, C₁-C₃ alkyl, C₁-C₂ haloalkyl, C₁-C₄ alkoxy, C₁-C₃ haloalkoxy, C₂-C₃ alkenyloxy, C₂-C₃ alkynyloxy, C₁-C₂ alkylsulfanyl, C₁-C₂ alkylsulfinyl, C₁-C₂ alkylsulfonyl, C₁-C₂ alkoxy-C₁-C₂ alkyl, N-C₁-C₄alkylamino, N,N-diC₁-C₄alkylamino, C₁-C₃ alkoxycarbonyl, C₁-C₂ alkylcarbonyl, N-C₁-C₂ alkoxy-C₁-C₂ alkyl-carbonimidoyl, N-hydroxy-C₁-C₂ alkyl-carbonimidoyl, C₁-C₂ alkylaminocarbonyl, di(C₁-C₂ alkylamino)carbonyl, C₁-C₂ alkylcarbonylamino, C₁-C₂ alkylsulfonylamino, trifluoromethylsulfonyloxy, phenyl, 2-cyanophenyl, 3-cyanophenyl, 4-cyanophenyl, 2-methylphenyl, 3-methylphenyl, 4-methylphenyl, 3-chloropyrrol-1-yl, 4-(trifluoromethyl)pyrazol-1-yl, 3-(trifluoromethyl)pyrazol-1-yl, 3-cyanopyrazol-1-yl, 4-cyanopyrazol-1-yl, 5-chloropyrazol-1-yl, 4-chloropyrazol-1-yl, 3-chloropyrazol-1-yl, 5-fluoropyrazol-1-yl, 4-fluoropyrazol-1-yl, 3-fluoropyrazol-1-yl, 3,5-dimethylpyrazol-1-yl, 5-methylpyrazol-1-yl, 4-methylpyrazol-1-yl, 3-methylpyrazol-1-yl, pyrazol-1-yl, cyclopropyl and 1-cyanocyclopropyl.

In another embodiment of the invention, R⁹ and R¹⁰ are independently selected from the group consisting of hydrogen, halogen, amino, carboxylic acid, cyano, C₁-C₃ alkyl, C₁-C₄ alkoxy, C₁-C₄ haloalkyl, C₁-C₄ alkoxycarbonyl, C₁-C₂ alkylsulfonyl, C₁-C₄ alkylsulfonylamino, C₁-C₄ alkylcarbonylamino, di(C₁-C₄ alkylamino)carbonyl, N-C₁-C₂ alkoxy-C-C₁-C₂ alkyl-carbonimidoyl, C₁-C₄ alkylcarbonyl and C₁-C₄ alkylcarbonylamino, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl and C₁-C₄ alkoxy

In still another embodiment of the invention, R⁹ and R¹⁰ are independently selected from the group consisting of hydrogen, halogen, amino, carboxylic acid, cyano, C₁-C₃ alkyl, C₁-C₄ alkoxy, C₁-C₄ haloalkyl, C₁-C₄ alkoxycarbonyl, C₁-C₂ alkylsulfonyl, C₁-C₄ alkylsulfonylamino, C₁-C₄ alkylcarbonylamino, di(C₁-C₄ alkylamino)carbonyl, N-C₁-C₂ alkoxy-C-C₁-C₂ alkyl-carbonimidoyl, C₁-C₄ alkylcarbonyl and C₁-C₄ alkylcarbonylamino, 5- or 6-membered heteroaryl and C₃-C₆ cycloalkyl, wherein the 5- or 6-membered heteroaryl comprises 1 or 2 heteroatoms individually selected from N, O and S, and wherein any of said 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl and C₁-C₄ alkoxy. Preferably, R⁹ and R¹⁰ are independently selected from the group consisting of hydrogen, chlorine, fluorine, bromine, amino, carboxylic acid, cyano, methyl, ethyl, trifluoromethyl, difluoromethyl, difluoromethoxy, 2,2-difluoroethoxy, 2,2,2-trifluoroethoxy, methoxy, ethoxy, propoxy, allyloxy, prop-2-ynoxy, methylsulfanyl, methylsulfinyl, methylsulfonyl, ethylsulfonyl, dimethylamino, methoxymethyl, ethoxymethyl, 2-methoxyethoxymethyl, methoxycarbonyl, ethoxycarbonyl, tert-butoxycarbonyl, acetyl, propanoyl, - C(CH₃)=NOCH₃, -C(CH₃)=NOCH₂CH₃, -C(CH₃)=NOH, methylaminocarbonyl, di(methylamino)carbonyl, methylcarbonylamino, methylsulfonylamino, trifluoromethylsulfonyloxy, phenyl, 2-cyanophenyl, 3-cyanophenyl, 4-cyanophenyl, 2-methylphenyl, 3-methylphenyl, 4-methylphenyl, 3-chloropyrrol-1-yl, 4-(trifluoromethyl)pyrazol-1-yl, 3-(trifluoromethyl)pyrazol-1-yl, 3-cyanopyrazol-1-yl, 4-cyanopyrazol-1-yl, 5-chloropyrazol-1-yl, 4-chloropyrazol-1-yl, 3-chloropyrazol-1-yl, 5-fluoropyrazol-1-yl, 4-fluoropyrazol-1-yl, 3-fluoropyrazol-1-yl, 3,5-dimethylpyrazol-1-yl, 5-methylpyrazol-1-yl, 4-methylpyrazol-1-yl, 3-methylpyrazol-1-yl, pyrazol-1-yl, cyclopropyl and 1-cyanocyclopropyl.

More preferably, R⁹ and R¹⁰ are independently selected from the group consisting of hydrogen, chlorine, fluorine, bromine, amino, carboxylic acid, cyano, methyl, ethyl, trifluoromethyl, difluoromethyl, difluoromethoxy, 2,2-difluoroethoxy, 2,2,2-trifluoroethoxy, methoxy, ethoxy, propoxy, allyloxy, prop-2-ynoxy, methylsulfanyl, methylsulfinyl, methylsulfonyl, ethylsulfonyl, dimethylamino, methoxymethyl, ethoxymethyl, 2-methoxyethoxymethyl, methoxycarbonyl, ethoxycarbonyl, tert-butoxycarbonyl, acetyl, propanoyl, -C(CH₃)=NOCH₃, -C(CH₃)=NOCH₂CH₃, -C(CH₃)=NOH, methylaminocarbonyl, di(methylamino)carbonyl, methylcarbonylamino, methylsulfonylamino and trifluoromethylsulfonyloxy.

In one embodiment of the invention, R¹¹ is selected from the group consisting of hydrogen, halogen, hydroxy, cyano, C₁-C₄ alkyl, C₁-C₄ alkoxy, C₂-C₄ alkenyloxy, C₁-C₄ alkylcarbonyl and N-C₁-C₄ alkoxy-C₁-C₄ alkyl-carbonimidoyl.

In another embodiment of the invention, R¹¹ is selected from the group consisting of hydrogen, halogen, hydroxy, cyano, C₁-C₄ alkyl and C₁-C₄ alkoxy. Preferably, R¹¹ is selected from the group consisting of hydrogen, chlorine, fluorine, bromine, hydroxy, cyano, methyl and methoxy.

In one embodiment R¹² is selected from the group consisting of hydrogen, halogen, C₁-C₄ alkyl, C₁-C₄ alkoxyl, C₁-C₄ alkylcarbonyl and N-C₁-C₄ alkoxy-C₁-C₄ alkyl-carbonimidoyl.

In another embodiment R¹² is selected from the group consisting of hydrogen, halogen, C₁-C₄ alkyl and C₁-C₄ alkoxyl. In still another embodiment of the invention, R¹² is selected from the group consisting of hydrogen, halogen and C₁-C₄ alkyl. Preferably, R¹² is selected from the group consisting of hydrogen, chlorine, fluorine, bromine and methyl.

In an embodiment of the invention, Q is selected from the group consisting of Q¹, Q², Q³ and Q⁴: 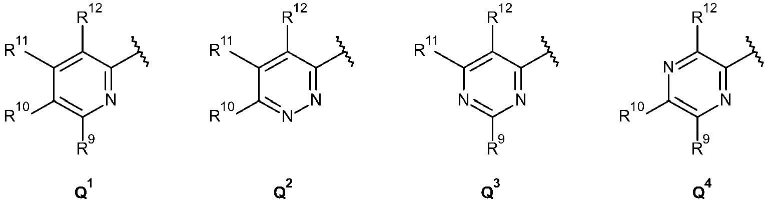 wherein R⁹ and R¹⁰ are independently selected from the group consisting of hydrogen, halogen, amino, hydroxy, carboxylic acid, cyano, C₁-C₃ alkyl, C₁-C₂ haloalkyl, C₁-C₄ alkoxy, C₁-C₃ haloalkoxy, C₂-C₃ alkenyloxy, C₂-C₃ alkynyloxy, C₁-C₂ alkylsulfanyl, C₁-C₂ alkylsulfinyl, C₁-C₂ alkylsulfonyl, C₁-C₂ alkoxy-C₁-C₂ alkyl, N-C₁₋₄alkylamino, N,N-diC₁₋₄alkylamino, C₁-C₃ alkoxycarbonyl, C₁-C₂ alkylcarbonyl, N-C₁-C₂ alkoxy-C₁-C₂ alkyl-carbonimidoyl, N-hydroxy-C₁-C₂ alkyl-carbonimidoyl, C₁-C₂ alkylaminocarbonyl, di(C₁-C₂ alkylamino)carbonyl, C₁-C₂-alkylcarbonylamino, C₁-C₂-alkylsulfonylamino, trifluoromethylsulfonyloxy, phenyl, 2-cyanophenyl, 3-cyanophenyl, 4-cyanophenyl, 2-methylphenyl, 3-methylphenyl, 4-methylphenyl, 3-chloropyrrol-1-yl, 4-(trifluoromethyl)pyrazol-1-yl, 3-(trifluoromethyl)pyrazol-1-yl, 3-cyanopyrazol-1-yl, 4-cyanopyrazol-1-yl, 5-chloropyrazol-1-yl, 4-chloropyrazol-1-yl, 3-chloropyrazol-1-yl, 5-fluoropyrazol-1-yl, 4-fluoropyrazol-1-yl, 3-fluoropyrazol-1-yl, 3,5-dimethylpyrazol-1-yl, 5-methylpyrazol-1-yl, 4-methylpyrazol-1-yl, 3-methylpyrazol-1-yl, pyrazol-1-yl, cyclopropyl and 1-cyanocyclopropyl; R¹¹ is selected from the group consisting of hydrogen, halogen, hydroxy, cyano, C₁-C₄ alkyl and C₁-C₄ alkoxy; and R¹² is selected from the group consisting of hydrogen, halogen and C₁-C₄ alkyl.

Preferably, Q is selected from the group consisting of Q¹, Q² and Q³: 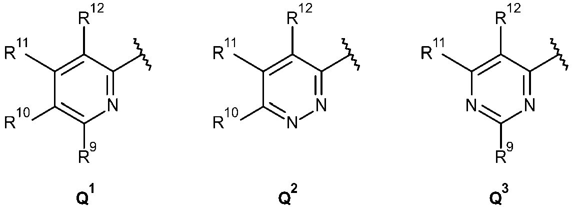 wherein R⁹ and R¹⁰ are independently selected from the group consisting of hydrogen, chlorine, fluorine, bromine, amino, carboxylic acid, cyano, methyl, ethyl, trifluoromethyl, difluoromethyl, difluoromethoxy, 2,2-difluoroethoxy, 2,2,2-trifluoroethoxy, methoxy, ethoxy, propoxy, allyloxy, prop-2-ynoxy, methylsulfanyl, methylsulfinyl, methylsulfonyl, ethylsulfonyl, dimethylamino, methoxymethyl, ethoxymethyl, 2-methoxyethoxymethyl, methoxycarbonyl, ethoxycarbonyl, tert-butoxycarbonyl, acetyl, propanoyl, - C(CH₃)=NOCH₃, -C(CH₃)=NOCH₂CH₃, -C(CH₃)=NOH, methylaminocarbonyl, di(methylamino)carbonyl, methylcarbonylamino, methylsulfonylamino and trifluoromethylsulfonyloxy; R¹¹ is selected from the group consisting of hydrogen, chlorine, fluorine, bromine, hydroxy, cyano, methyl and methoxy; and R¹² is selected from the group consisting of hydrogen, chlorine, fluorine, bromine and methyl.

More preferably, Q is Q¹: 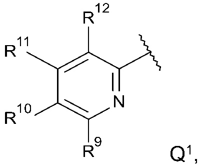 wherein R⁹ and R¹⁰ are independently selected from the group consisting of hydrogen, chlorine, fluorine, bromine, amino, carboxylic acid, cyano, methyl, ethyl, trifluoromethyl, difluoromethyl, difluoromethoxy, 2,2-difluoroethoxy, 2,2,2-trifluoroethoxy, methoxy, ethoxy, propoxy, allyloxy, prop-2-ynoxy, methylsulfanyl, methylsulfinyl, methylsulfonyl, ethylsulfonyl, dimethylamino, methoxymethyl, ethoxymethyl, 2-methoxyethoxymethyl, methoxycarbonyl, ethoxycarbonyl, tert-butoxycarbonyl, acetyl, propanoyl, - C(CH₃)=NOCH₃, -C(CH₃)=NOCH₂CH₃, -C(CH₃)=NOH, methylaminocarbonyl, di(methylamino)carbonyl, methylcarbonylamino, methylsulfonylamino and trifluoromethylsulfonyloxy; R¹¹ is selected from the group consisting of hydrogen, chlorine, fluorine, bromine, hydroxy, cyano, methyl and methoxy; and R¹² is selected from the group consisting of hydrogen, chlorine, fluorine, bromine and methyl.

In an embodiment of the invention, A¹, A² and A³ are independently selected from the group consisting of CR⁷, N, O and S, with the proviso that at least one of A¹, A² and A³ is selected from N, O and S, and that no more than one of A¹, A² and A³ is O or S.

In an embodiment of the invention, R⁷ is hydrogen or methyl.

In an embodiment of the invention, R⁸ is hydrogen or methyl.

In an embodiment of the invention, Z¹ is selected from the group consisting of 1-methylpyrazol-4-yl, 2,3,4-trifluorophenyl, 2,3-difluorophenyl, 3,4-difluorophenyl, 2,4,6-trifluorophenyl, 2,4-difluorophenyl, 2,5-difluorophenyl, 2-fluoro-4-methoxy-phenyl, 2-fluoro-4-methylsulfonyl-phenyl, 2-fluorophenyl, 3-fluorophenyl, 4-fluorophenyl, 2-furyl, 2-thienyl, 3-thienyl, 2-methylphenyl, 3-methylphenyl, 4-methylphenyl, 2-chlorophenyl, 3-chlorophenyl, 4-chlorophenyl, 3-methoxyphenyl, 4-ethynyl-2-fluoro-phenyl, 4-fluoro-2-methoxy-phenyl, cyclopropyl, 1-methylcyclopropyl, cyclobutyl, cyclohexyl, cyclopentyl, methyl, n-propyl and phenyl. Preferably, Z¹ is selected from the group consisting of 1-methylpyrazol-4-yl, 2,4,6-trifluorophenyl, 2,4-difluorophenyl, 2,5-difluorophenyl, 2-chlorophenyl, 2-fluorophenyl, 2-furyl, 2-methylphenyl, 2-thienyl, 3,4-difluorophenyl, 3-chlorophenyl, 3-fluorophenyl, 3-methylphenyl, 3-thienyl, 4-fluoro-2-methoxy-phenyl, 4-fluorophenyl, 4-methylphenyl, cyclobutyl, cyclohexyl, cyclopentyl, methyl and phenyl. More preferably, Z¹ is selected from the group consisting of 1-methylpyrazol-4-yl, 2,4,6-trifluorophenyl, 2,4-difluorophenyl, 2-fluorophenyl, 2-furyl, 2-methylphenyl, 2-thienyl, 3,4-difluorophenyl, 3-chlorophenyl, 3-thienyl, 4-fluoro-2-methoxy-phenyl, 4-fluorophenyl, cyclobutyl, cyclohexyl, cyclopentyl and methyl. Even more preferably, Z¹ is selected from the group consisting of 2,4-difluorophenyl, 2-fluorophenyl, 4-fluorophenyl and phenyl.

In an embodiment of the invention, the compound of formula (I) may be a compound of formula (II): 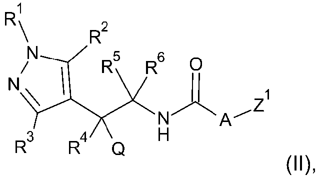 wherein R¹, R², R³, R⁴, R⁵, R⁶, Q (Q¹, Q², Q³, Q⁴ and Q⁵ ) and Z¹ are as defined for the compounds of formula (I) according to the present invention, and A is selected from the group consisting of: 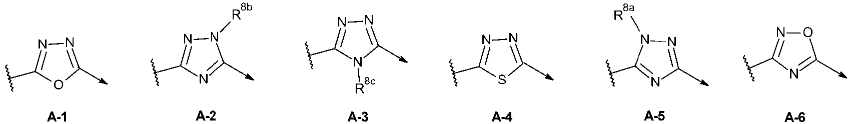 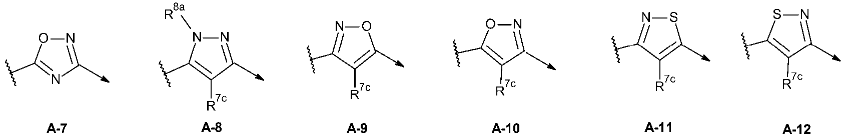 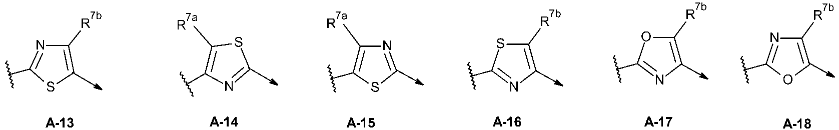 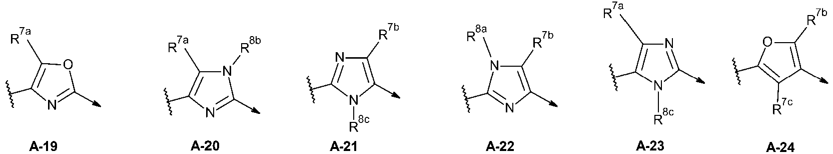 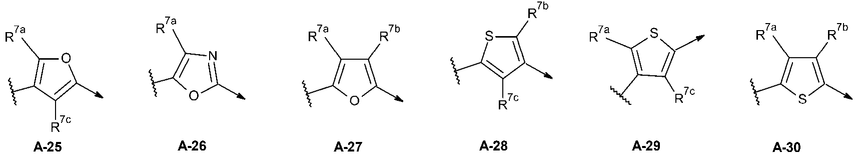 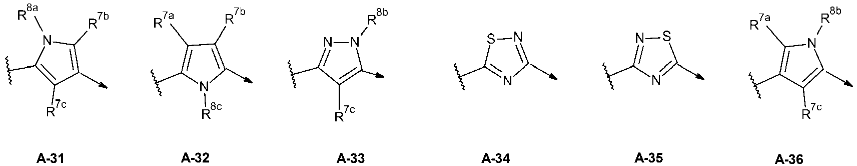 wherein the dotted line indicates the bond to the C(=O) group, and the arrow indicates the bond to the Z¹ group, and R^{7a}, R^{7b}, R^{7c}, R^{8a}, R^{8b} and R^{8c} are independently selected from the group consisting of hydrogen, C₁-C₄ alkyl, C₂-C₄ alkenyl and C₂-C₄ alkynyl.

In an embodiment of the invention, R^{7a}, R^{7b}, R^{7c}, R^{8a}, R^{8b} and R^{8c} are independently selected from the group consisting of hydrogen and methyl.

In another embodiment of the invention, R^{7a}, R^{7b}, R^{7c}, R^{8a}, R^{8b} and R^{8c} are hydrogen.

In another embodiment of the invention, R^{7a}, R^{7b}, R^{7c}, R^{8a}, R^{8b} and R^{8c} are methyl.

Preferably, in the compound of formula (II) each of R^{7a}, R^{7b} and R^{7c} is hydrogen and each of R^{8a}, R^{8b} and R^{8c} is methyl.

In an embodiment of the invention, in the compound of formula (II) A is selected from the group consisting of A-1, A-4, A-6, A-7, A-9, A-10, A-13 or A-15: 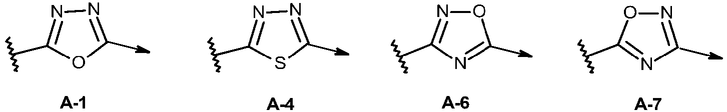 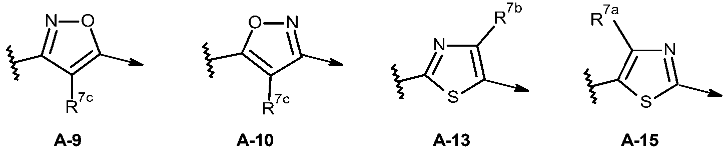 wherein the dotted line indicates the bond to the C(=O) group, and the arrow indicates the bond to the Z¹ group, and R^{7a}, R^{7b} and R^{7c} are independently selected from hydrogen and methyl.

In another embodiment of the invention, in the compound of formula (II) A is selected from the group consisting of A-1, A-4, A-6, A-7, A-9, A-10, A-13 or A-15: 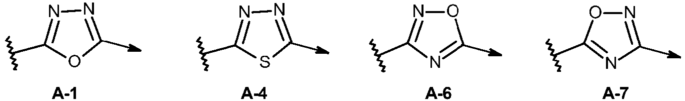 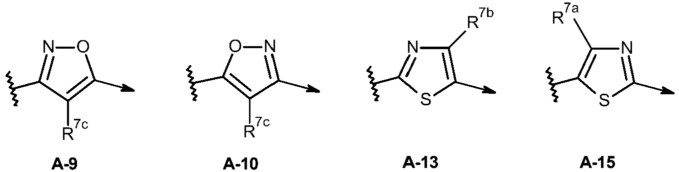 wherein the dotted line indicates the bond to the C(=O) group, and the arrow indicates the bond to the Z¹ group, and R^{7a}, R^{7b} and R^{7c} are hydrogen.

In another embodiment of the invention, in the compound of formula (II) A is selected from the group consisting of A-1, A-4, A-9, A-10, A-13 or A-15: 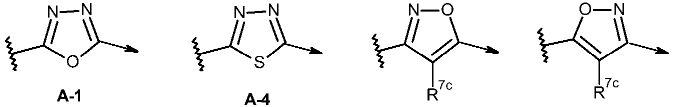 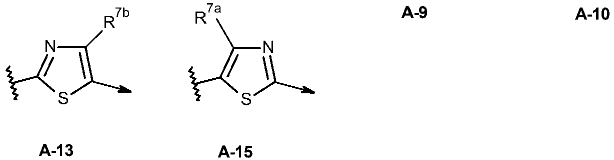 wherein the dotted line indicates the bond to the C(=O) group, and the arrow indicates the bond to the Z¹ group, and R^{7a}, R^{7b} and R^{7c} are hydrogen.

In another embodiment of the invention, in the compound of formula (II) A is selected from the group consisting of A-1, A-4, A-9 or A-10: 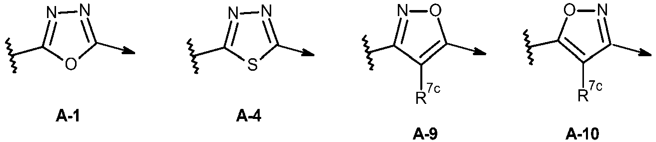 wherein the dotted line indicates the bond to the C(=O) group, and the arrow indicates the bond to the Z¹ group, and R^{7c} is hydrogen.

In another embodiment of the invention, in the compound of formula (II) A is selected from the group consisting of A-4, A-9 or A-10: 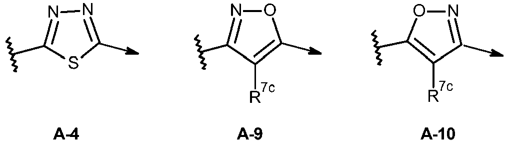 wherein the dotted line indicates the bond to the C(=O) group and the arrow indicates the bond to the Z¹ group, and R^{7c} is hydrogen.

In an embodiment of the invention, the compound of formula (II) may be a compound of formula (II-A) wherein A is A-9: 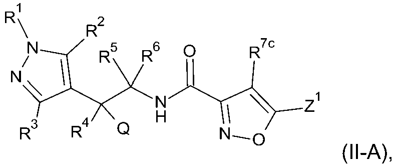 wherein R¹, R², R³, R⁴, R⁵, R⁶, R^{7c}, Q and Z¹ are as defined for the compounds of formula (I) according to the present invention.

In a variant of this embodiment of the invention, the compound of formula (II) may be a compound of formula (II-A) wherein Q is Q¹.

Preferably, in the compound of formula (II-A) of the invention,
R¹ is C₁-C₄ alkyl, preferably methyl, ethyl or isopropyl,
R² is selected from the group consisting of hydrogen, fluorine, chlorine and methyl,
R³ is selected from the group consisting of hydrogen, fluorine, chlorine and methyl,
R⁴ is selected from the group consisting of hydrogen, methyl, ethyl, isopropyl and cyano,
R⁵ and R⁶ are independently selected from the group consisting of hydrogen, methyl and ethyl,
R^{7c} is hydrogen or methyl, and
Q and Z¹ are as defined for the compounds of formula (I) according to the present invention.

In another embodiment of the invention, the compound of formula (II) may be a compound of formula (II-B) wherein A is A-10: 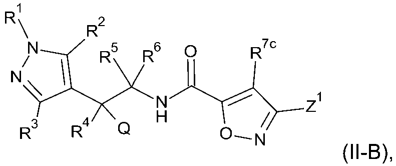 wherein R¹, R², R³, R⁴, R⁵, R⁶, R^{7c}, Q and Z¹ are as defined for the compounds of formula (I) according to the present invention.

In a variant of this embodiment of the invention, the compound of formula (II) may be a compound of formula (II-B) wherein Q is Q¹.

Preferably, in the compound of formula (II-B) of the invention,
R¹ is C₁-C₄ alkyl, preferably methyl, ethyl or isopropyl,
R² is selected from the group consisting of hydrogen, fluorine, chlorine and methyl,
R³ is selected from the group consisting of hydrogen, fluorine, chlorine and methyl,
R⁴ is selected from the group consisting of hydrogen, methyl, ethyl, isopropyl and cyano,
R⁵ and R⁶ are independently selected from the group consisting of hydrogen, methyl and ethyl,
R^{7c} is hydrogen or methyl, and
Q and Z¹ are as defined for the compounds of formula (I) according to the present invention.

In another embodiment of the invention, the compound of formula (II) may be a compound of formula (II-C) wherein A is A-4: 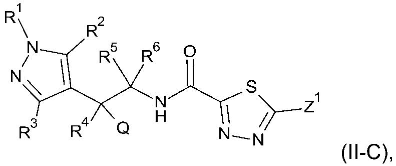 wherein R¹, R², R³, R⁴, R⁵, R⁶, Q and Z¹ are as defined for the compounds of formula (I) according to the present invention.

In a variant of this embodiment of the invention, the compound of formula (II) may be a compound of formula (II-C) wherein Q is Q¹.

Preferably, in the compound of formula (II-C) of the invention,
R¹ is C₁-C₄ alkyl, preferably methyl, ethyl or isopropyl,
R² is selected from the group consisting of hydrogen, fluorine, chlorine and methyl,
R³ is selected from the group consisting of hydrogen, fluorine, chlorine and methyl,
R⁴ is selected from the group consisting of hydrogen, methyl, ethyl, isopropyl and cyano,
R⁵ and R⁶ are independently selected from the group consisting of hydrogen, methyl and ethyl, and
Q and Z¹ are as defined for the compounds of formula (I) according to the present invention.

The presence of one or more possible asymmetric carbon atoms in any of the compounds of formula (I), (II), (II-A), (II-B) and (II-C) according to the invention means that the compounds may occur in chiral isomeric forms, i.e., enantiomeric or diastereomeric forms.

More preferably, the compound of formula (I) according to the invention is selected from compounds listed in any one of Tables C-1 to C-23 or in Table T1.

According to a fifth aspect of the invention, there is provided an intermediate compound of formula (III) or a salt thereof: 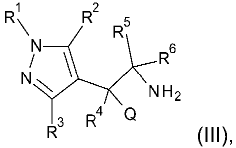 wherein R¹, R², R³, R⁴, R⁵, R⁶ and Q correspond to the same definitions as for the compounds of formula (I) according to the present invention.

The intermediate compounds of formula (III) possess the same definitions for R¹, R², R³, R⁴, R⁵, R⁶ and Q as for the compounds of formula (I) according to the invention and their corresponding preferences.

The presence of one or more possible asymmetric carbon atoms in a compound of formula (III) according to the invention means that the compounds may occur in chiral isomeric forms, i.e., enantiomeric or diastereomeric forms.

The compounds of formula (I) according to the present invention can be made as shown in the following Schemes 1 to 13 in which, unless otherwise stated, the definition of each variable is as defined above for a compound of formula (I).

In any of the Schemes 1 to 13 below, the presence of one or more possible asymmetric carbon atoms in a compound of formula (I) according to the invention means that the compounds may occur in chiral isomeric forms, i.e., enantiomeric or diastereomeric forms.

The compounds as defined in any of the embodiments of the present invention can be made as shown in the following Schemes 1 to 13, in which, unless otherwise stated, the definition of each variable is as defined above in any of the embodiments according to the invention.

Compounds of formula (I) may be prepared from compounds of formula (III) by reaction with a compound of formula (II) using dicyclohexylcarbodiimide (DCC), diisopropylcarbodiimide (DIC) or N-(3-Dimethylaminopropyl)-N'-ethylcarbodiimide (EDAC·HCl) together with an additive such as 1-hydroxybenzotriazole (HOBt), Hydroxy-3,4-dihydro-4-oxo-1,2,3-benzotriazine (HODhbt), N-hydroxysuccinimide (HOSu), 1-Hydroxy-7-aza-1H-benzotriazole (HOAt) or 4-(N,N-dimethylamino)pyridine (DMAP). This reaction is shown in Scheme 1.

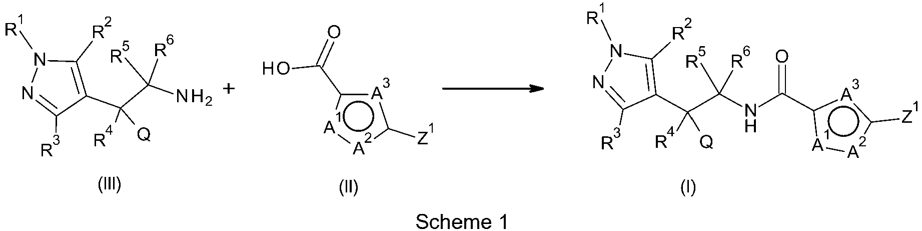

Alternatively, compounds of formula (I) may be prepared by reacting a compound of formula (IIa) with a compound of formula (III) in an inert solvent such as tetrahydrofuran (THF), ethyl acetate, methylene chloride, toluene and the like, optionally in the presence of an inorganic base, for example aqueous sodium hydroxide, or potassium carbonate, or in the presence of an organic base such as trimethylamine or diisopropyl amine. The latter reaction with organic bases can optionally be carried out in the presence of a catalyst such as DMAP. Compounds of formula (Ila), wherein X⁰ is halogen, preferably chlorine, can be prepared from compounds of formula (II) by treatment with a halogenating agent, such as thionyl chloride (SOCl₂) or oxalyl chloride (COCl₂), in an inert solvent as noted above, optionally mediated by the presence of catalytic quantities of N,N-dimethyl formamide. This reaction is shown in Scheme 2.

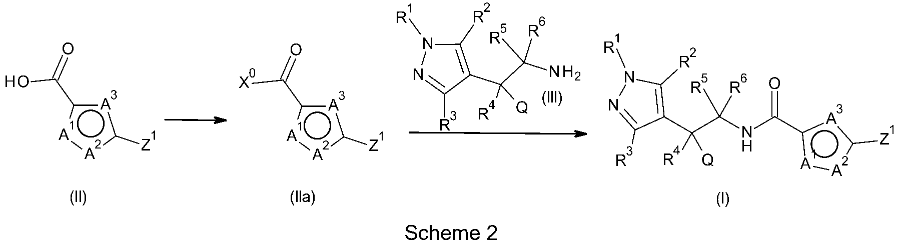

Acylation reactions of carboxylic acids such as compounds of formula (II) with amines such as compounds of formula (III) are well known to those skilled in the art and described for example in Eur. J. Org. Chem. 2020, 4641-4651, and references cited therein.

Compounds of formula (II) are commercially available or can be prepared according to, or analogous to, procedures described in the literature. For example, WO2018/019929, Org. Proc. Res. Dev. 2020, 24(2), 228-234, WO2018/019929, Lett. Org. Chem. 2010, 7(7), 502-507, and J. Het Chem. 2015, 52(6), 1823-1833.

Compounds of formula (III), or salts thereof, wherein R¹, R², R³, R⁴, R⁵, R⁶ and Q are as defined above for the compound of formula (I), may be prepared by a person skilled in the art by a reaction between nitriles of formula (IV), wherein R¹, R², R³, R⁴ and Q are as defined above for the compound of formula (I), and a suitable nucleophile such as (dimethyl sulfide)dihydroboron (BMS) in a suitable aprotic solvent such as THF, for example as described in J. Org. Chem. 1981 47, 3153. Alternatively, Grignard reagents R⁵MgBr or R⁶MgBr, wherein R⁵ and R⁶ are as defined above for the compound of formula (I), may be added as nucleophiles to compounds of formula (IV), sequentially or simultaneously, to allow more highly substituted amines of formula (III) to be prepared. Such Grignard additions to nitriles are carried out in an inert solvent such as diethyl ether, tert-butylmethyl ether, and cyclopentyl methyl ether in the presence of a Lewis acid such as Ti(O-ⁱPr)₄ (see Synlett 2007, (4), 652-654). This reaction is shown in Scheme 3.

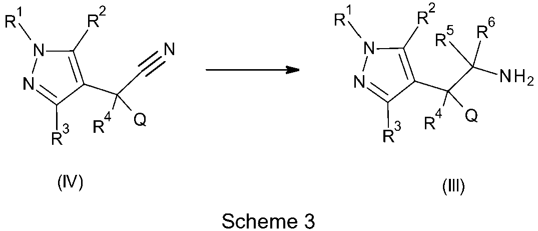

Compounds of formula (IV), wherein R¹, R², R³, R⁴ and Q are as defined above for the compound of formula (I), may be prepared by a person skilled in the art following known methods. More specifically, compounds of formula (IV), and intermediates thereof, may be prepared from compounds of formula (V) as shown in Scheme 4.

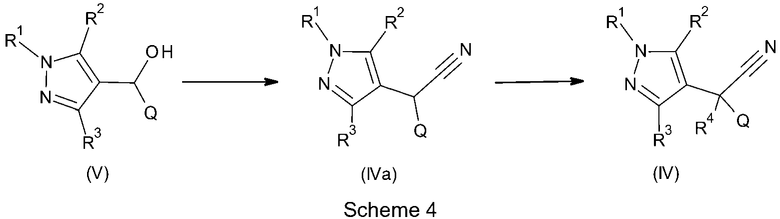

For example, compounds of formula (IV), wherein R¹, R², R³, R⁴ and Q are as defined above for the compound of formula (I) and R⁴ is different from hydrogen, may be prepared by a person skilled in the art by deprotonation of compound of formula (IVa), wherein R⁴ is hydrogen and R¹, R², R³ and Q are as defined above for the compound of formula (I), using a strong base such as n-butyl lithium or sodium hydride at cryogenic temperatures in an inert solvent such as THF, followed by addition of a suitable alkylating agent R⁴-X⁰, wherein X⁰ is halogen, for example iodomethane. Compounds of formula (IVa), wherein R⁴ is hydrogen and R¹, R², R³ and Q are as defined above for the compound of formula (I), may be prepared from alcohols of formula (V) by treatment with cyanotrimethylsilane (TMSCN) in the presence of a base such as lithium carbonate in a nonpolar solvent such as dichloromethane at temperatures between 0°C and the boiling point of the reaction mixture. Such transformations are well known in the literature under a variety of conditions, for example as described in Org. Lett. 2008 10, 4570 and references therein. This reaction is shown in Scheme 4.

Compounds of formula (V) may be prepared from compounds of formula (VI), respectively from any of compounds of formula (VIa), (VIb), (VIc), (VId) or (VIe), as shown in Scheme 5.

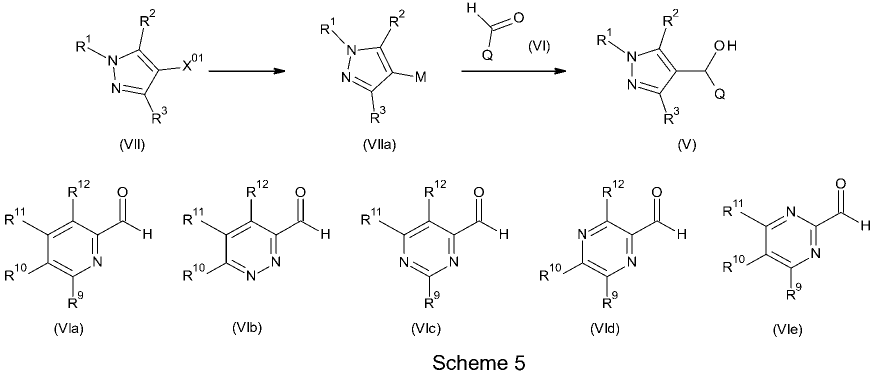

As shown in Scheme 5, compounds of formula (VII), wherein R¹, R² and R³ are as defined above for the compound of formula (I) and X⁰¹ is bromo or iodo, are metallated with an appropriate reagent such as turbo Grignard (isopropylmagnesium chloride-lithium chloride complex), or an alkyl lithium, such as n-butyl lithium to give an intermediate Grignard or alkyl lithium reagent (M is MgX⁰¹ or Lithium). Such metal insertions into C-X⁰¹ bonds are well known to those skilled in the art and are generally carried out at temperatures between -78°C to room temperature, in inert solvents such as ethers, e.g. tert-butyl methyl ether or THF and the like. Solutions of the metallated species (Vlla) are then treated with compounds of formula (VI), respectively (VIa), (VIb), (VIc), (VId), or (VIe) to give compounds of formula (V). Similar reactions of these type have been described in for example WO2012/102297 and Bio. Med.Chem. Lett. 2017, 27(17), 4044-4050 (X⁰¹ is Br, n-butyl lithium) and Ang. Chem. Int. Ed. 2016, 55(17), 5332-5336, US 2014/0349990, WO2002/004424, WO2021/009068 (X⁰¹ is I, Turbo Grignard).

Compounds of formula (VI) and (VII) are either commercially available or are readily prepared by methods known by those skilled in the art.

A further synthesis of compounds of formula (I) involves treatment of compounds of formula (VIII) with a base, such as sodium hydride or n-butyl lithium, in an inert solvent, such as THF, and subsequent alkylation with compounds of formula (IX), wherein R⁴ is as described under formula (I) and X⁰² is a leaving group such as halogen, mesylate or tosylate, to yield compounds of formula (X) (Scheme 6).

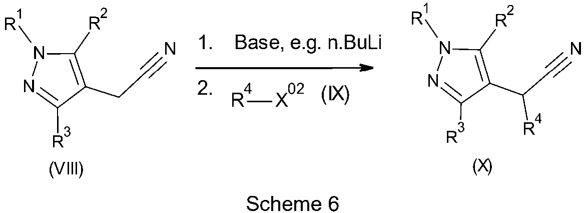

Compounds of formula (X), wherein R¹, R², R³, and R⁴ are as defined above for the compound of formula (I), are then treated with a strong base such as sodium hydride or an alkyl lithium base such as n-butyl lithium in an inert solvent, such as THF or tert-butyl methyl ether, at temperatures between -78°C to room temperature, followed by addition of a compound of formula (XI), respectively any of compounds of formula (XIa), (XIb), (XIc), (XId) or (Xle), wherein R⁹, R¹⁰, R¹¹, and R¹² are as defined above for the compound of formula (I), and X⁰³ is a leaving group such as halogen, preferably F, Cl or Br to give compounds of formula (IV). This reaction is shown in Scheme 7.

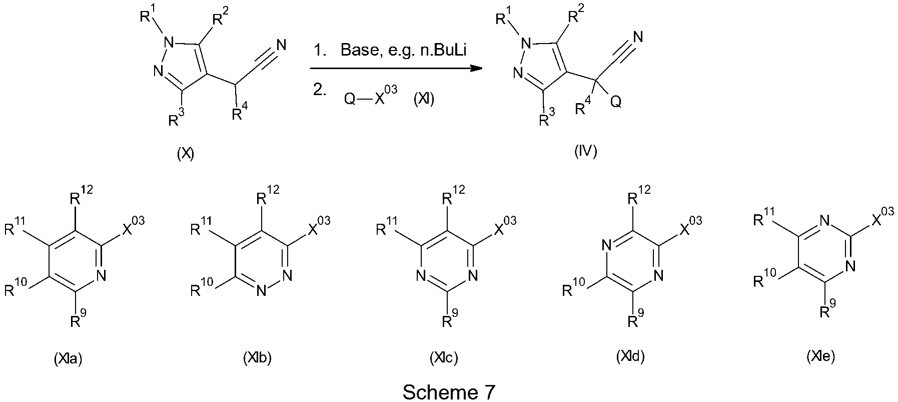

Compounds of formula (IV) are converted into compounds of formula (I) as previously described in Schemes 1, 2 and 3. Those skilled in the art will recognize that conversion of compounds (VIII) into compounds of formula (IV) can be carried out sequentially or in the same reaction vessel, enabling a streamlined conversion of compounds of formula (VIII) to compounds of formula (IV). This is described in more details in the preparative examples.

Compounds of formula (la), wherein R¹, R², R³, R⁵, Q, A¹, A², A³ and Z¹ are as described above for compounds of formula (I) and R⁴ and R⁶ are hydrogen, can also be prepared by treatment of compounds of formula (VI), respectively any of compounds of formula (Vla), (Vlb), (VIc), (Vld) or (VIe), with compounds of formula (XII), wherein R⁵ is as described above for compounds of formula (I) in the presence of a base, such as triethyl amine, optionally in an inert solvent, such as ethanol or methanol, to give compounds of formula (XIII). These compounds may be isolated and converted to compounds of formula (XIV) by treatment with an anhydride, such as trifluoroacetic acid anhydride, in an inert solvent, such as methylene chloride, in the presence of a base, for example triethyl amine. This reaction is shown in Scheme 8. 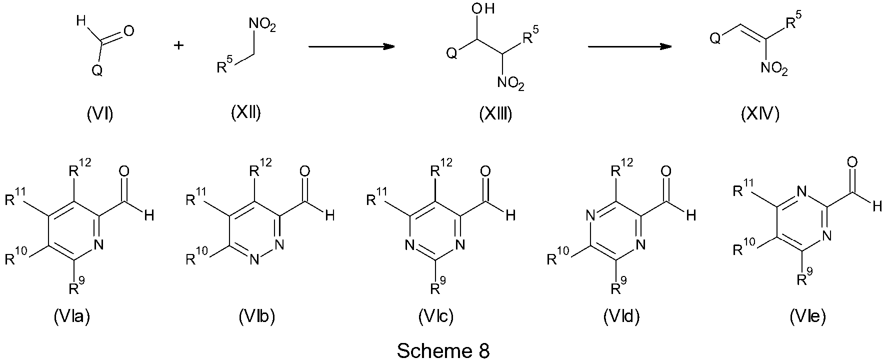 Those skilled in the art will appreciate that compounds of formula (VI) can be converted into compounds of formula (XIV) without isolation of the intermediates of formula (XIII). Such reactions, known as the Henry reactions, are well described in the literature, as evidenced in Tetrahedron. 2001, 57(6), 915-945, and references cited therein. Compounds of formula (XIV) can be converted into compounds of formula (XV) by treatment with compounds of formula (VIIa) (see Scheme 5) in an inert solvent such as THF. This reaction is shown in Scheme 9.

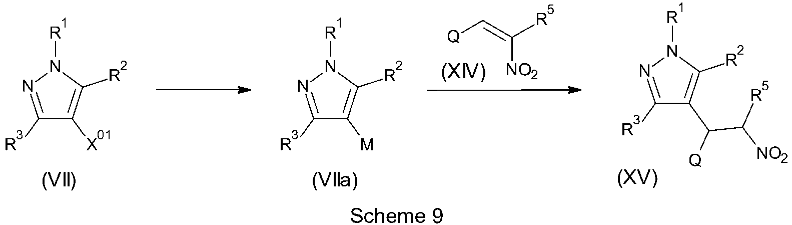

Similar Michael additions of organometallics to nitro alkenes have been reported for example in Org. Lett., 2007, 9, 85-87. Reduction of the nitro group in compounds of formula (XV) to the amine to give compounds of formula (Illa), wherein R¹, R², R³, R⁵ and Q are as defined above for the compound of formula (I) and R⁴ and R⁶ are hydrogen, can be achieved by a multitude of methods generally known to those skilled in the art, such as Bechamp reduction, or reduction with hydrogen in the presence of a metal catalyst. This reaction is shown in Scheme 10.

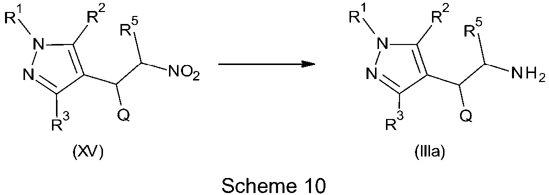

Compounds of formula (Illa) are converted into compounds of formula (la) by the methods described in Schemes 1 and 2.

Further compounds according to the invention can be prepared by derivatization at a later stage in the synthesis using a key central intermediate. For example, compounds of formula (I), wherein Q is Q¹, and R¹, R², R³, R⁴, R⁵, R⁶, R¹⁰, R¹¹, R¹², A¹, A², A³ and Z¹ are as defined above for the compounds of formula (I), and X⁰⁴ is halogen, preferably bromine or chlorine, i.e. compounds of formula (la): 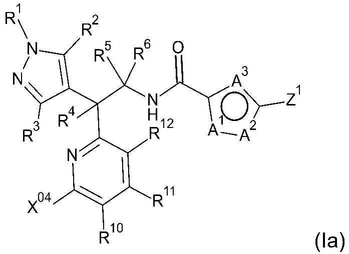 allow further chemistry to be carried out such as palladium catalysed carbonylations, Suzuki reactions, Stille couplings, copper catalysed introduction of sulphonyl groups, haloalkyl groups, and cyano moieties, as well as SnAr reactions with a variety of nucleophiles (Scheme 11).

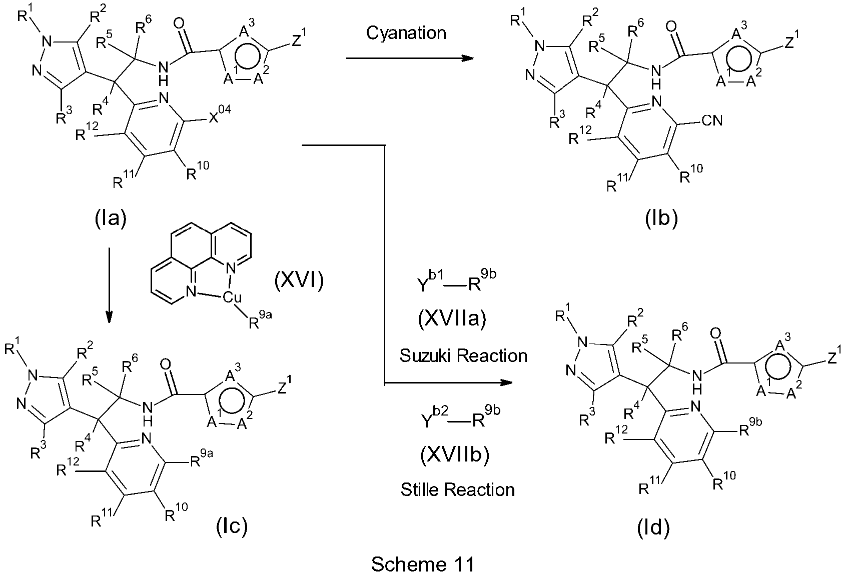

As shown in Scheme 11, compounds of formula (I), wherein Q is Q¹, and R¹, R², R³, R⁴, R⁵, R⁶, R¹⁰, R¹¹, R¹², A¹, A², A³ and Z¹ are as defined above for the compound of formula (I), and R⁹ is cyano, namely compounds of formula (Ib), can be obtained from compound of formula (la) by treatment with an inorganic cyanide source such as CuCN in inert solvent such as dimethylformamide (DMF) or N-methyl-2-pyrrolidone at temperatures between 0°C and 150°C. Such reactions are well known in the literature, for example, in J. Het. Chem., (1987), 24(2), 373-6, Liebigs Ann. Chem., (1994), (10), 1049-53, and Org. Prep. Proc. Int., (1985), 17(6), 391-9. Other methods for introduction of the cyano group by substitution of a halogen atom are known in the art. See, for example, Science of Synthesis (2004), 19, 173-195. Compounds of formula (I), wherein Q is Q¹, and R¹, R², R³, R⁴, R⁵, R⁶, R¹⁰, R¹¹, R¹², A¹, A², A³ and Z¹ are as defined above for the compound of formula (I), and R^{9a} is C₁-C₄ haloalkyl, namely compounds of formula (Ic), can be prepared by treating compounds of formula (la) wherein Q is Q¹, and R¹, R², R³, R⁴, R⁵, R⁶, R¹⁰, R¹¹, R¹², A¹, A², A³ and Z¹ are as defined above for the compound of formula (I), and X⁰⁴ is halogen, preferably bromine, with compounds of formula (XVI), wherein R^{9a} is C₁-C₄ haloalkyl, in an inert solvent such as DMF or N-methyl-2-pyrrolidone at temperatures between room temperature and 150°C. Such reactions are known in the literature (Org. Lett., (2014), 16(6), 1744-1747). Compounds of formula (I), wherein Q is Q¹, and R¹, R², R³, R⁴, R⁵, R⁶, R¹⁰, R¹¹, R¹², A¹, A², A³ and Z¹ are as defined above for the compound of formula (I), and R^{9b} is phenyl, 5- or 6-membered heteroaryl or C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl and C₁-C₄ alkoxy, namely compounds of formula (Id), can prepared (as shown in Scheme 11) by a Suzuki reaction, which involves, for example, reacting compounds of formula (la), wherein X⁰⁴ is a leaving group like, for example, chlorine, bromine or iodine, with compounds of formula (XVlla), wherein Y^{b1} can be a boron-derived functional group, as for example B(OH)₂ or B(OR_{b1})₂, wherein R_{b1} can be a C₁-C₄ alkyl group or the two groups OR_{b1} can form, together with the boron atom, a five membered ring, as for example a pinacol boronic ester. The reaction is catalyzed by a palladium based catalyst, for example *tetrakis*(triphenylphosphine)-palladium or (1,1'-bis(diphenylphosphino)-ferrocene)dichloropalladium-dichloromethane (1:1 complex), in presence of a base, like sodium carbonate or cesium fluoride, in a solvent or a solvent mixture, like, for example, a mixture of 1,2-dimethoxyethane and water, or dioxane and water, or methyl THF and water, preferably under inert atmosphere. The reaction temperature can preferentially range from room temperature to the boiling point of the reaction mixture. Such Suzuki reactions are well known to those skilled in the art and have been reviewed, for example, in J. Organomet. Chem., 576, 1999, 147-168.

Alternatively, compounds of formula (Ic) can be prepared by a Stille reaction of compounds of formula (XVIIb), wherein Y_{b2} is a trialkyl tin derivative, preferably tri-n-butyl tin, with compounds of formula (Ia). Such Stille reactions are carried out in the presence of a palladium catalyst, for example tetrakis(triphenylphosphine)palladium(0) or (1,1'-bis(diphenylphosphino)-ferrocene)dichloropalladium-dichloromethane (1:1 complex), in an inert solvent such as DMF, acetonitrile, or dioxane, optionally in the presence of an additive, such as cesium fluoride, or lithium chloride, and optionally in the presence of a further catalyst, for example copper(I) iodide. Such Stille couplings are also well known to those skilled in the art, and have been described, for example, in J. Org. Chem., 2005, 70, 8601-8604, J. Org. Chem., 2009, 74, 5599-5602, and Angew. Chem. Int. Ed., 2004, 43, 1132-1136. A large number of compounds of formula (XVlla) and (XVllb) are commercially available, or can be prepared by those skilled in the art. Further compounds available from compounds of formula (la) are shown in Scheme 12.

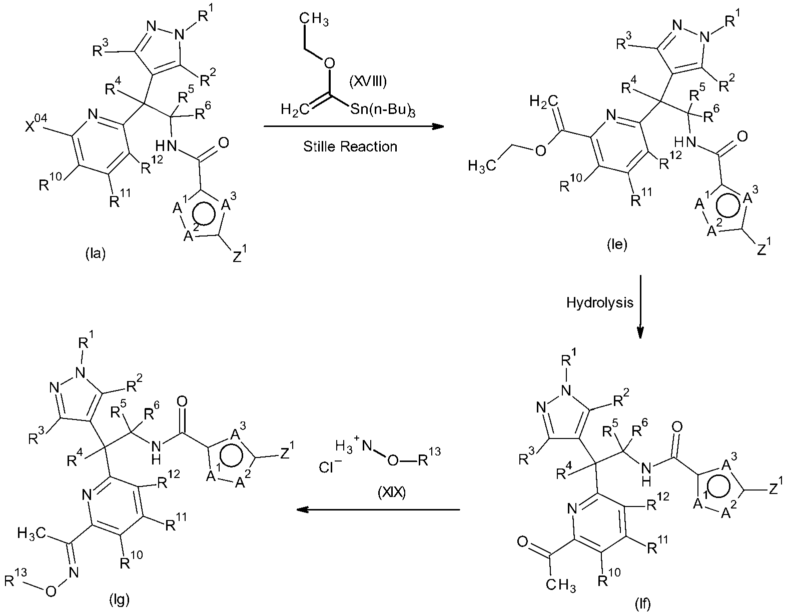

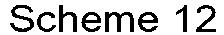

As shown in Scheme 12, compounds of formula (I), wherein Q is Q¹, and R¹, R², R³, R⁴, R⁵, R⁶, R¹⁰, R¹¹, R¹², A¹, A², A³ and Z¹ are as defined above for the compound of formula (I), and X⁰⁴ is a leaving group like, for example, chlorine, bromine or iodine, namely compounds of formula (la), can be treated with compounds of formula (XVIII) under Stille reactions conditions to give compounds of formula (le). Compounds of formula (le) can be isolated, or directly hydrolysed under aqueous acidic conditions to give compounds of formula (If). Such reactions are known in the literature and have been described, for example, in Synthesis (2001), (10), 1551-1555, and Tetrahedron (2001), 57(13), 2507-2514. Compounds of formula (If) can be converted to compound of formula (Ig), wherein Q is Q¹, and R¹, R², R³, R⁴, R⁵, R⁶, R¹⁰, R¹¹, R¹², A¹, A², A³ and Z¹ are as defined above for the compound of formula (I), and R¹³ is hydrogen or C₁-C₄ alkyl by treatment of compounds of formula (If) with compounds of formula (XIX) (or a salt thereof), wherein R¹³ is hydrogen or C₁-C₄ alkyl, in an inert solvent such as methanol, ethanol, THF, methyl, optionally in the presence of an inorganic base such as sodium or potassium carbonate, or organic bases such as triethylamine and the like. Many examples for the preparation of such oximes are known in the literature (see, for example, Molecules 2019, 24, 2470 and references cited therein) and are well known to those skilled in the art.

Further compounds that can be prepared from compounds of formula (I), wherein Q is Q¹, and R¹, R², R³, R⁴, R⁵, R⁶, R¹⁰, R¹¹, R¹², A¹, A², A³ and Z¹ are as defined above for the compound of formula (I), and X⁰⁴ is a leaving group like, for example, chlorine, bromine or iodine, namely compounds of formula (la), are shown in Scheme 13.

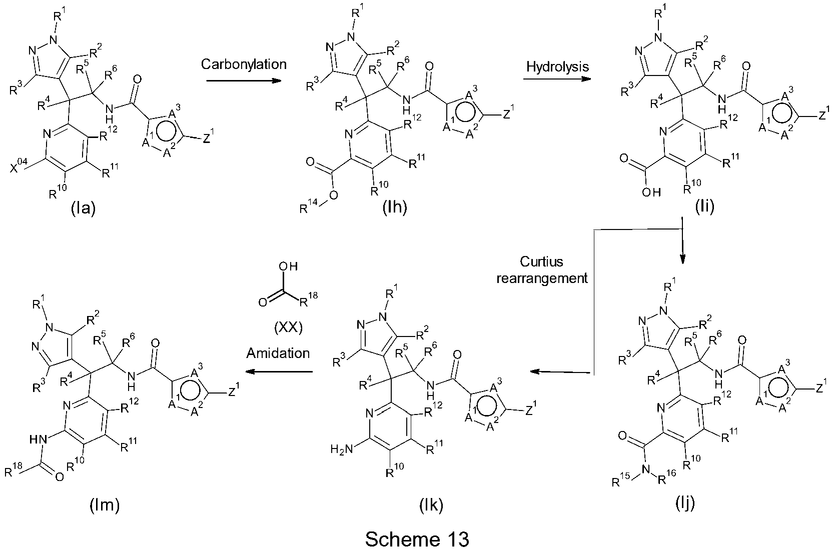

As shown in Scheme 13, compounds of formula (la) can be carbonylated to give compounds of formula (I), wherein Q is Q¹, and R¹, R², R³, R⁴, R⁵, R⁶, R¹⁹, R¹¹, R¹², A¹, A², A³ and Z¹ are as defined above for the compound of formula (I), and R¹⁴ is C₁-C₄ alkyl, namely compounds of formula (Ih). In such alkoxycarbonylations, compounds of formula (la) are reacted with carbon monoxide, usually under pressure, in the presence of metal catalyst such as a palladium catalyst (for example, palladium(II) acetate, [1,1'-bis(diphenylphosphino)ferrocene] palladium(II) dichloride Pd(dppf)Cl₂, bis(triphenylphosphine)-palladium(II) dichloride PdCl₂(PPh₃)₂ or bis(diphenylphosphino)propane]palladium(II) PdCl₂(dippp)), optionally in the presence of a phosphine ligand such as triphenylphosphine or 1,1'-bis(diphenylphosphino)ferrocene, in an alcohol R¹⁴OH solvent (typically methanol or ethanol), wherein R¹⁴ is C₁-C₄alkyl, optionally in the presence of a co-solvent (e.g. toluene, dioxane or N,N-dimethylformamide), and preferably in the presence of a base, such as for example trimethylamine, at temperatures between 20°C and 200°C, preferably between 50°C and 180°C. Such carbonylation reactions are well known to those skilled in the art and also in the literature (see J. Org. Chem. 2008, 73, 7102-7107, and references cited therein). Such compounds of formula (Ih) can be easily saponified to compounds of formula (li) under conditions known to those skilled in the art, for example conditions such as aqueous sodium, potassium or lithium hydroxide in methanol, ethanol, THF or dioxane at room temperature, or up to refluxing conditions. Alternatively, treating ester compounds of formula (Ih) with halide anions, preferably chloride anions, originating from, for example, lithium chloride (or alternatively, sodium or potassium chloride), in solvents such as N,N-dimethylformamide, N,N-dimethylacetamide or N-methyl-2-pyrrolidone, may also generate the carboxylic acid compounds of formula (li). The reaction temperatures for such an O-demethylation range preferably from 20°C to the boiling point of the reaction mixture, or the reaction may be performed under microwave irradiation. Compounds of formula (li) can be converted to amides of formula (Ij), wherein Q is Q¹, and R¹, R², R³, R⁴, R⁵, R⁶, R¹⁰, R¹¹, R¹², A¹, A², A³ and Z¹ are as defined above for the compound of formula (I), and R¹⁵ and R¹⁶ are independently hydrogen or C₁-C₄ alkyl, namely compounds of formula (Ij). Such reactions usually involve activating the carboxyl group, followed by treatment with a compound R¹⁵R¹⁶NH or using coupling agents to perform the direct conversion of the acids to the amides upon treatment with compounds of formula R¹⁵R¹⁶NH. These methods have been discussed *vide supra* in Schemes 1 and 2.

Compounds of formula (li) can be converted to compounds of formula (I), wherein Q is Q¹, and R¹, R², R³, R⁴, R⁵, R⁶, R¹⁰, R¹¹, R¹², A¹, A², A³ and Z¹ are as defined above for the compound of formula (I), and R⁹ is amino, namely compounds of formula (Ik), by a so called Curtius rearrangement. In the Curtius rearrangement compounds of formula (Ij) are treated with an organo-azide in the presence of a suitable base and optionally in the presence or absence of Lewis acids, in an inert solvent at temperatures between 50°C and 200°C. Examples of organo-azide include TMSN₃, sodium azide, diphenyl phosphoryl azide or tosyl azide and suitable solvent may be toluene, xylene, THF or acetonitrile. Example of suitable Lewis acids may include Zn(OTf)₂ amongst others. The isocyanates formed in the rearrangement react with water to form carbamates which decarboxylate under the reaction conditions to the corresponding amines of formula (Ik). Alternatively, the reactions can be carried out in alcohols, e.g. t-butyl alcohol, allowing the t-butyl carbamates to be isolated. These in turn can be cleaved in a separate step by methods known to those skilled in the art with acids (such as trifluoroacetic acid) to yield compounds of formula (Ik). Examples of such Curtius reactions have been reported, for example, in Org. Lett. 2005, 7, 4107-4110, J. Med. Chem. 2006, 49(12), 3614-3627, and Tetrahedron 1974, 30, 2151-2157. Compounds of formula (Ik) so obtained can be amidated to compounds of formula (Im), wherein Q is Q¹, and R¹, R², R³, R⁴, R⁵, R⁶, R¹⁰, R¹¹, R¹², A¹, A², A³ and Z¹ are as defined above for the compound of formula (I), and R¹⁸ is C₁-C₄ alkyl by treatment with compounds of formula (XX) according to the amidation methods described *vide supra.* Those skilled in the art will recognize that such chemistry can be applied to any of the compounds of formula (1), where Q is Q¹,Q²,Q³,Q⁴ or Q⁵at any positions of the heterocycles of formula Q (i.e. R⁹, R¹⁰, R¹¹ or R¹²) when the later groups are a leaving group such as a halogen atom.

A compound of formula (I) as defined in any of the embodiments of the present invention can be converted in a manner known *per se* into another compound as defined in any of the embodiments of the present invention by replacing one or more substituents of the starting compound in the customary manner by (an)other substituent(s) according to the invention. Those skilled in the art will also appreciate that compounds of formula (I) can be further transformed to further derivatives of formula (I) by, for example, alkylation, nucleophilic substitution, elimination, C-C-bond forming reactions in the presence of metal catalysts, heteroatom-carbon bond formation in the presence of metal catalysts, oxidation, and reduction.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula (I) may be prepared in a manner known *per se.* Thus, for example, acid addition salts of compounds of formula (I) are obtained by treatment with a suitable acid or a suitable ion exchanger reagent and salts with bases are obtained by treatment with a suitable base or with a suitable ion exchanger reagent.

Salts of compounds of formula (I) can be converted in the customary manner into the free compounds (I), acid addition salts, for example, by treatment with a suitable basic compound or with a suitable ion exchanger reagent and salts with bases, for example, by treatment with a suitable acid or with a suitable ion exchanger reagent.

Salts of compounds of formula (I) can be converted in a manner known *per se* into other salts of compounds of formula (I), acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula (I), which have salt-forming properties, can be obtained in free form or in the form of salts.

The compounds of formula (I) and, where appropriate, the tautomer's thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule, the invention relates to the pure isomers and also to all isomer mixtures which are possible and is to be understood in each case in this sense hereinabove and herein below, even when stereochemical details are not mentioned specifically in each case.

Diastereomeric mixtures or racemic mixtures of compounds of formula (I), in free form or in salt form, which can be obtained depending on which starting materials and procedures have been chosen can be separated in a known manner into the pure diastereomers or racemates on the basis of the physicochemical differences of the components, for example by fractional crystallization, distillation and/or chromatography.

Enantiomeric mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl cellulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

It is advantageous to isolate or synthesize in each case the biologically more effective isomer, for example enantiomer or diastereomer, or isomer mixture, for example enantiomer mixture or diastereomer mixture, if the individual components have a different biological activity.

As an example, compounds with more than one asymmetric carbon atoms may exist in diastereomeric forms which can be optionally separated using for example supercritical fluid chromatography (SFC) chromatography with chiral colums. Such diastereomers can show a different fungicidal activity profile, but all isomers and diastereomers form part of this invention.

The compounds of formula (I) of the present invention exhibit two asymmetric carbon atoms . The relationship between enantiomers and diastereomers of compounds of formula (I) is shown below.

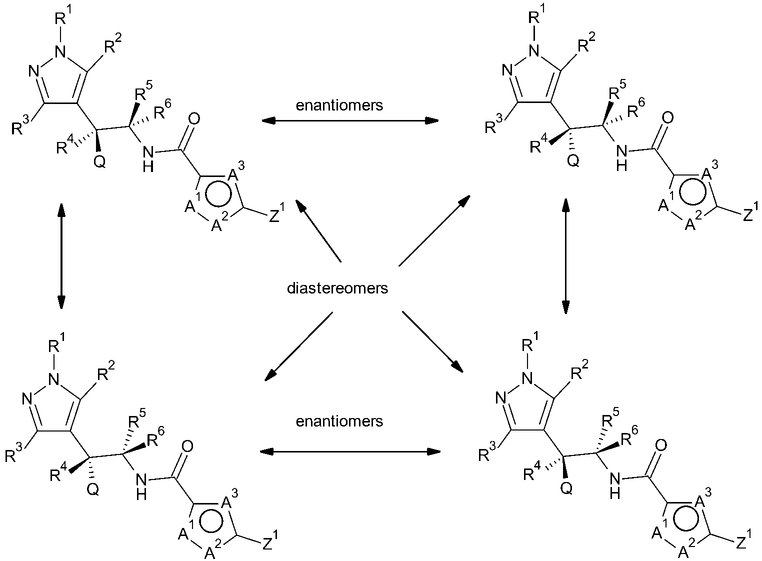

A person skilled in the art is well aware that above diastereomers and enantiomers of formula (I) wherein R¹, R², R³, R⁴, R⁵, R⁶, R⁷, R⁸, R⁹, R¹⁰, R¹¹, R¹², A¹, *A²,* A³, Q and Z¹ are as defined for formula (I) are within the scope of the invention.

The compounds of formula (I) and, where appropriate, the tautomers thereof, in each case in free form or in salt form, can, if appropriate, also be obtained in the form of hydrates and/or include other solvents, for example those which may have been used for the crystallization of compounds which are present in solid form.

As already indicated, surprisingly, it has now been found that the compounds of formula (I) of the present invention have, for practical purposes, a very advantageous level of biological activity for protecting plants against diseases that are caused by fungi.

The compounds of formula (I) according to the invention can be used in the agricultural sector and related fields of use, e.g., as active ingredients for controlling plant pests or on non-living materials for the control of spoilage microorganisms or organisms potentially harmful to man. The novel compounds are distinguished by excellent activity at low rates of application, by being well tolerated by plants and by being environmentally safe. They have very useful curative, preventive and systemic properties and can be used for protecting numerous cultivated plants. The compounds of formula (I) can be used to inhibit or destroy the pests that occur on plants or parts of plants (fruit, blossoms, leaves, stems, tubers, roots) of different crops of useful plants, while at the same time protecting also those parts of the plants that grow later, e.g., from phytopathogenic microorganisms.

The present invention further relates to a method for controlling or preventing infestation of plants or plant propagation material and/or harvested food crops susceptible to microbial attack by treating plants or plant propagation material and/or harvested food crops wherein an effective amount a compound of formula (I) according to the invention is applied to the plants, to parts thereof or the locus thereof.

It is also possible to use a compound of formula (I) according to the invention as a fungicide. The term "fungicide" as used herein means a compound that controls, modifies, or prevents the growth of fungi. The term "fungicidally effective amount" where used means the quantity of such a compound or combination of such compounds that is capable of producing an effect on the growth of fungi. Controlling or modifying effects include all deviation from natural development, such as killing, retardation and the like, and prevention includes barrier or other defensive formation in or on a plant to prevent fungal infection.

It may also be possible to use compounds of formula (I) according to the invention as dressing agents for the treatment of plant propagation material, e.g., seed, such as fruits, tubers or grains, or plant cuttings, for the protection against fungal infections as well as against phytopathogenic fungi occurring in the soil. The propagation material can be treated with a composition comprising a compound of formula (I) before planting: seed, for example, can be dressed before being sown. The active compounds of formula (I) can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore, the compounds of formula (I) according to the invention can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

The compounds of formula (I) according to the invention are for example, effective against fungi and fungal vectors of disease as well as phytopathogenic bacteria and viruses. These fungi and fungal vectors of disease as well as phytopathogenic bacteria and viruses are for example: Absidia corymbifera, Alternaria spp, Aphanomyces spp, Ascochyta spp, Aspergillus spp. including A. flavus, A. fumigatus, A. nidulans, A. niger, A. terrus, Aureobasidium spp. including A. pullulans, Blastomyces dermatitidis, Blumeria graminis, Bremia lactucae, Botryosphaeria spp. including B. dothidea, B. obtusa, Botrytis spp. including B. cinerea, Candida spp. including C. albicans, C. glabrata, C. krusei, C. lusitaniae, C. parapsilosis, C. tropicalis, Cephaloascus fragrans, Ceratocystis spp, Cercospora spp. including C. arachidicola, Cercosporidium personatum, Cladosporium spp, Claviceps purpurea, Coccidioides immitis, Cochliobolus spp, Colletotrichum spp. including C. musae, Cryptococcus neoformans, Diaporthe spp, Didymella spp, Drechslera spp, Elsinoe spp, Epidermophyton spp, Erwinia amylovora, Erysiphe spp. including E. cichoracearum, Eutypa lata, Fusarium spp. including F. culmorum, F. graminearum, F. langsethiae, F. moniliforme, F. oxysporum, F. proliferatum, F. subglutinans, F. solani, Gaeumannomyces graminis, Gibberella fujikuroi, Gloeodes pomigena, Gloeosporium musarum, Glomerella cingulate, Guignardia bidwellii, Gymnosporangium juniperi-virginianae, Helminthosporium spp, Hemileia spp, Histoplasma spp. including H. capsulatum, Laetisaria fuciformis, Leptographium lindbergi, Leveillula taurica, Lophodermium seditiosum, Microdochium nivale, Microsporum spp, Monilinia spp, Mucor spp, Mycosphaerella spp. including M. graminicola, M. pomi, Oncobasidium theobromaeon, Ophiostoma piceae, Paracoccidioides spp, Penicillium spp. including P. digitatum, P. italicum, Petriellidium spp, Peronosclerospora spp. Including P. maydis, P. philippinensis and P. sorghi, Peronospora spp, Phaeosphaeria nodorum, Phakopsora pachyrhizi, Phellinus igniarus, Phialophora spp, Phoma spp, Phomopsis viticola, Phytophthora spp. including P. infestans, Plasmopara spp. including P. halstedii, P. viticola, Pleospora spp., Podosphaera spp. including P. leucotricha, Polymyxa graminis, Polymyxa betae, Pseudocercosporella herpotrichoides, Pseudomonas spp, Pseudoperonospora spp. including P. cubensis, P. humuli, Pseudopeziza tracheiphila, Puccinia Spp. including P. hordei, P. recondita, P. striiformis, P. triticina, Pyrenopeziza spp, Pyrenophora spp, Pyricularia spp. including P. oryzae, Pythium spp. including P. ultimum, Ramularia spp, Rhizoctonia spp, Rhizomucor pusillus, Rhizopus arrhizus, Rhynchosporium spp, Scedosporium spp. including S. apiospermum and S. prolificans, Schizothyrium pomi, Sclerotinia spp, Sclerotium spp, Septoria spp, including S. nodorum, S. tritici, Sphaerotheca macularis, Sphaerotheca fusca (Sphaerotheca fuliginea), Sporothorix spp, Stagonospora nodorum, Stemphylium spp, Stereum hirsutum, Thanatephorus cucumeris, Thielaviopsis basicola, Tilletia spp, Trichoderma spp. including T. harzianum, T. pseudokoningii, T. viride, Trichophyton spp, Typhula spp, Uncinula necator, Urocystis spp, Ustilago spp, Venturia spp. including V. inaequalis, Verticillium spp, and Xanthomonas spp.

The compounds of formula (I) according to the invention may be used for example on turf, ornamentals, such as flowers, shrubs, broad-leaved trees or evergreens, for example conifers, as well as for tree injection, pest management and the like.

Within the scope of present invention, target crops and/or useful plants to be protected typically comprise perennial and annual crops, such as berry plants for example blackberries, blueberries, cranberries, raspberries and strawberries; cereals for example barley, maize (corn), millet, oats, rice, rye, sorghum triticale and wheat; fibre plants for example cotton, flax, hemp, jute and sisal; field crops for example sugar and fodder beet, coffee, hops, mustard, oilseed rape (canola), poppy, sugar cane, sunflower, tea and tobacco; fruit trees for example apple, apricot, avocado, banana, cherry, citrus, nectarine, peach, pear and plum; grasses for example Bermuda grass, bluegrass, bentgrass, centipede grass, fescue, ryegrass, St. Augustine grass and Zoysia grass; herbs such as basil, borage, chives, coriander, lavender, lovage, mint, oregano, parsley, rosemary, sage and thyme; legumes for example beans, lentils, peas and soya beans; nuts for example almond, cashew, ground nut, hazelnut, peanut, pecan, pistachio and walnut; palms for example oil palm; ornamentals for example flowers, shrubs and trees; other trees, for example cacao, coconut, olive and rubber; vegetables for example asparagus, aubergine, broccoli, cabbage, carrot, cucumber, garlic, lettuce, marrow, melon, okra, onion, pepper, potato, pumpkin, rhubarb, spinach and tomato; and vines for example grapes.

The term "useful plants" is to be understood as also including useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield^{®} summer rape (Canola). Examples of crops that have been rendered tolerant to herbicides or classes of herbicides by genetic engineering methods include glyphosate- and glufosinate-resistant maize varieties commercially available under the trade names RoundupReady^{®}, Herculex I* and LibertyLink^{®}.

The term "useful plants" is to be understood as also including useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard^{®} (maize variety that expresses a CryIA(b) toxin); YieldGard^{®} Rootworm (maize variety that expresses a CryllIB(b1) toxin); YieldGard^{®} Plus (maize variety that expresses a CryIA(b) and a CryllIB(b1) toxin); Starlink^{™} (maize variety that expresses a Cry9(c) toxin); Herculex^{®} I (maize variety that expresses a CrylF(a2) toxin and the enzyme phosphinothricine N-acetyltransferase (PAT) to achieve tolerance to the herbicide glufosinate ammonium); NuCOTN 33B^{®} (cotton variety that expresses a CryIA(c) toxin); Bollgard^{®} I (cotton variety that expresses a CryIA(c) toxin); Bollgard^{®} II (cotton variety that expresses a CrylA(c) and a CryllA(b) toxin); VIPCOT^{®} (cotton variety that expresses a VIP toxin); NewLeaf^{®} (potato variety that expresses a CryIIIA toxin); Agrisure^{®} GT Advantage (GA21 glyphosate-tolerant trait), Agrisure^{®} CB Advantage (Bt11 corn borer (CB) trait),Agrisure^{®} RW (corn rootworm trait), RoundupReady^{®} (corn variety with Glyphosate herbicide tolerance) and LibertyLink^{®} (glufosinate-resistant maize variety).

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins from Bacillus cereus or Bacillus popilliae; or insecticidal proteins from Bacillus thuringiensis, such as delta-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

Further, in the context of the present invention there are to be understood by delta-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO2002/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO2003/018810).

Examples of such toxins or transgenic plants capable of synthesising such toxins are disclosed, for example, in EP0374753, WO1993/07278, WO1995/34656, EP0427529, EP0451878 and WO2003/052073.

The processes for the preparation of such transgenic plants are generally known to a person skilled in the art and are described, for example, in the publications mentioned above. Cryl-type deoxyribonucleic acids and their preparation are known, for example, from WO1995/34656, EP0367474, EP0401979 and WO1990/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and butterflies (Lepidoptera).

Transgenic plants containing one or more genes that code for an insecticidal resistance and express one or more toxins are known and some of them are commercially available. Examples of such plants are: YieldGard^{®} (maize variety that expresses a CryIA(b) toxin); YieldGard^{®} Rootworm (maize variety that expresses a CryIIIB(b1) toxin); YieldGard^{®} Plus (maize variety that expresses a CryIA(b) and a CryIIIB(b1) toxin); Starlink^{™} (maize variety that expresses a Cry9(c) toxin); Herculex^{®} I (maize variety that expresses a CrylF(a2) toxin and the enzyme phosphinothricine N-acetyltransferase (PAT) to achieve tolerance to the herbicide glufosinate ammonium); NuCOTN 33B^{®} (cotton variety that expresses a CryIA(c) toxin); Bollgard^{®} I (cotton variety that expresses a CryIA(c) toxin); Bollgard^{®} II (cotton variety that expresses a CrylA(c) and a CryllA(b) toxin); VIPCOT^{®} (cotton variety that expresses a VIP toxin); NewLeaf^{®} (potato variety that expresses a CryIIIA toxin); Agrisure^{®} GT Advantage (GA21 glyphosate-tolerant trait), Agrisure^{®} CB Advantage (Bt11 corn borer (CB) trait),Agrisure^{®} RW (corn rootworm trait), RoundupReady^{®} (corn variety with Glyphosate herbicide tolerance) and LibertyLink^{®} (glufosinate-resistant maize variety).

Further examples of such transgenic crops are:
1. Bt11 Maize from Syngenta Seeds SAS, Chemin de l'Hobit 27, F-31 790 St. Sauveur, France, registration number C/FR/96/05/10. Genetically modified Zea mays which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. Bt176 Maize from Syngenta Seeds SAS, Chemin de l'Hobit 27, F-31 790 St. Sauveur, France, registration number C/FR/96/05/10. Genetically modified Zea mays which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. MIR604 Maize from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO2003/018810.
4. MON 863 Maize from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. IPC 531 Cotton from Monsanto Europe S.A. 270-272 Avenue de Tervuren, B-1150 Brussels, Belgium, registration number C/ES/96/02.
6. 1507 Maize from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. NK603 × MON 810 Maize from Monsanto Europe S.A. 270-272 Avenue de Tervuren, B 1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

The compounds of formula (I) according to the invention may be used in controlling or preventing phytopathogenic diseases, especially phytopathogenic fungi such as Alternaria species in fruits, vegetables and potatoes; Botrytis cinerea in strawberries, tomatoes, sunflower, pulse crops, vegetables and grapes; Rhizoctonia solani in potatoes and vegetables; Uncinula necator in grapes; Cladosporium cucumerinum, Didymella bryoniae, Sphaerotheca fuliginea and Glomerella lagenarium in cucurbits; Leveillula taurica in cucurbits and solanaceous crops; Fusarium spp. in cereals; Leptosphaeria spp. in cereals; and Zymoseptoria spp. in cereals.

The term "locus" as used herein means fields in or on which plants are growing, or where seeds of cultivated plants are sown, or where seed will be placed into the soil. It includes soil, seeds, and seedlings, as well as established vegetation.

The term "plants" refers to all physical parts of a plant, including seeds, seedlings, saplings, roots, tubers, stems, stalks, foliage, and fruits.

The term "plant propagation material" is understood to denote generative parts of the plant, such as seeds, which can be used for the multiplication of the latter, and vegetative material, such as cuttings or tubers, for example potatoes. There can be mentioned for example seeds (in the strict sense), roots, fruits, tubers, bulbs, rhizomes and parts of plants. Germinated plants and young plants which are to be transplanted after germination or after emergence from the soil, may also be mentioned. These young plants can be protected before transplantation by a total or partial treatment by immersion. Preferably "plant propagation material" is understood to denote seeds.

The compounds of formula (I) according to the invention may be used in unmodified form or, preferably, together with the adjuvants conventionally employed in the art of formulation. To this end they may be conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants, e.g. for agricultural use, can be solid or liquid and are substances useful in formulation technology, e.g. natural or regenerated mineral substances, solvents, dispersants, wetting agents, tackifiers, thickeners, binders or fertilizers. Such carriers are for example described in WO1997/33890.

Suspension concentrates are aqueous formulations in which finely divided solid particles of the active compound are suspended. Such formulations include anti-settling agents and dispersing agents and may further include a wetting agent to enhance activity as well an anti-foam and a crystal growth inhibitor. In use, these concentrates are diluted in water and normally applied as a spray to the area to be treated. The amount of active ingredient may range from 0.5% to 95% of the concentrate.

Wettable powders are in the form of finely divided particles which disperse readily in water or other liquid carriers. The particles contain the active ingredient retained in a solid matrix. Typical solid matrices include fuller's earth, kaolin clays, silicas and other readily wet organic or inorganic solids. Wettable powders normally contain from 5% to 95% of the active ingredient plus a small amount of wetting, dispersing or emulsifying agent.

Emulsifiable concentrates are homogeneous liquid compositions dispersible in water or other liquid and may consist entirely of the active compound with a liquid or solid emulsifying agent, or may also contain a liquid carrier, such as xylene, heavy aromatic naphthas, isophorone and other non-volatile organic solvents. In use, these concentrates are dispersed in water or other liquid and normally applied as a spray to the area to be treated. The amount of active ingredient may range from 0.5% to 95% of the concentrate.

Granular formulations include both extrudates and relatively coarse particles and are usually applied without dilution to the area in which treatment is required. Typical carriers for granular formulations include sand, fuller's earth, attapulgite clay, bentonite clays, montmorillonite clay, vermiculite, perlite, calcium carbonate, brick, pumice, pyrophyllite, kaolin, dolomite, plaster, wood flour, ground corn cobs, ground peanut hulls, sugars, sodium chloride, sodium sulphate, sodium silicate, sodium borate, magnesia, mica, iron oxide, zinc oxide, titanium oxide, antimony oxide, cryolite, gypsum, diatomaceous earth, calcium sulphate and other organic or inorganic materials which absorb or which can be coated with the active compound. Granular formulations normally contain 5% to 25% of active ingredients which may include surface-active agents such as heavy aromatic naphthas, kerosene and other petroleum fractions, or vegetable oils; and/or stickers such as dextrins, glue or synthetic resins.

Dusts are free-flowing admixtures of the active ingredient with finely divided solids such as talc, clays, flours and other organic and inorganic solids which act as dispersants and carriers.

Microcapsules are typically droplets or granules of the active ingredient enclosed in an inert porous shell which allows escape of the enclosed material to the surroundings at controlled rates. Encapsulated droplets are typically 1 to 50 microns in diameter. The enclosed liquid typically constitutes 50 to 95% of the weight of the capsule and may include solvent in addition to the active compound. Encapsulated granules are generally porous granules with porous membranes sealing the granule pore openings, retaining the active species in liquid form inside the granule pores. Granules typically range from 1 millimetre to 1 centimetre and preferably 1 to 2 millimetres in diameter. Granules are formed by extrusion, agglomeration or prilling, or are naturally occurring. Examples of such materials are vermiculite, sintered clay, kaolin, attapulgite clay, sawdust and granular carbon. Shell or membrane materials include natural and synthetic rubbers, cellulosic materials, styrene-butadiene copolymers, polyacrylonitriles, polyacrylates, polyesters, polyamides, polyureas, polyurethanes and starch xanthates.

Other useful formulations for agrochemical applications include simple solutions of the active ingredient in a solvent in which it is completely soluble at the desired concentration, such as acetone, alkylated naphthalenes, xylene and other organic solvents. Pressurised sprayers, wherein the active ingredient is dispersed in finely-divided form as a result of vaporisation of a low boiling dispersant solvent carrier, may also be used.

Suitable agricultural adjuvants and carriers that are useful in formulating the compositions of the invention in the formulation types described above are well known to a person skilled in the art.

Liquid carriers that can be employed include, for example, water, toluene, xylene, petroleum naphtha, crop oil, acetone, methyl ethyl ketone, cyclohexanone, acetic anhydride, acetonitrile, acetophenone, amyl acetate, 2-butanone, chlorobenzene, cyclohexane, cyclohexanol, alkyl acetates, diacetonalcohol, 1,2-dichloropropane, diethanolamine, p diethylbenzene, diethylene glycol, diethylene glycol abietate, diethylene glycol butyl ether, diethylene glycol ethyl ether, diethylene glycol methyl ether, N,N-dimethyl formamide, dimethyl sulfoxide, 1,4-dioxane, dipropylene glycol, dipropylene glycol methyl ether, dipropylene glycol dibenzoate, diproxitol, alkyl pyrrolidinone, ethyl acetate, 2-ethyl hexanol, ethylene carbonate, 1,1,1-trichloroethane, 2-heptanone, alpha pinene, d-limonene, ethylene glycol, ethylene glycol butyl ether, ethylene glycol methyl ether, gamma-butyrolactone, glycerol, glycerol diacetate, glycerol monoacetate, glycerol triacetate, hexadecane, hexylene glycol, isoamyl acetate, isobornyl acetate, isooctane, isophorone, isopropyl benzene, isopropyl myristate, lactic acid, laurylamine, mesityl oxide, methoxy-propanol, methyl isoamyl ketone, methyl isobutyl ketone, methyl laurate, methyl octanoate, methyl oleate, methylene chloride, m-xylene, n-hexane, n-octylamine, octadecanoic acid, octyl amine acetate, oleic acid, oleylamine, o-xylene, phenol, polyethylene glycol (PEG400), propionic acid, propylene glycol, propylene glycol monomethyl ether, p-xylene, toluene, triethyl phosphate, triethylene glycol, xylene sulfonic acid, paraffin, mineral oil, trichloroethylene, perchloroethylene, ethyl acetate, amyl acetate, butyl acetate, methanol, ethanol, isopropanol, and higher molecular weight alcohols such as amyl alcohol, tetrahydrofurfuryl alcohol, hexanol, octanol, etc., ethylene glycol, propylene glycol, glycerine and N-methyl-2-pyrrolidinone. Water is generally the carrier of choice for the dilution of concentrates.

Suitable solid carriers include, for example, talc, titanium dioxide, pyrophyllite clay, silica, attapulgite clay, kieselguhr, chalk, diatomaxeous earth, lime, calcium carbonate, bentonite clay, fuller's earth, cotton seed hulls, wheat flour, soybean flour, pumice, wood flour, walnut shell flour and lignin.

A broad range of surface-active agents are advantageously employed in both said liquid and solid compositions, especially those designed to be diluted with carrier before application. These agents, when used, normally comprise from 0.1% to 15% by weight of the formulation. They can be anionic, cationic, non-ionic or polymeric in character and can be employed as emulsifying agents, wetting agents, suspending agents or for other purposes. Typical surface active agents include salts of alkyl sulfates, such as diethanolammonium lauryl sulphate; alkylarylsulfonate salts, such as calcium dodecylbenzenesulfonate; alkylphenol-alkylene oxide addition products, such as nonylphenol-C.sub. 18 ethoxylate; alcohol-alkylene oxide addition products, such as tridecyl alcohol-C.sub. 16 ethoxylate; soaps, such as sodium stearate; alkylnaphthalenesulfonate salts, such as sodium dibutylnaphthalenesulfonate; dialkyl esters of sulfosuccinate salts, such as sodium di(2 ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; and salts of mono and dialkyl phosphate esters.

Other adjuvants commonly utilized in agricultural compositions include crystallisation inhibitors, viscosity modifiers, suspending agents, spray droplet modifiers, pigments, antioxidants, foaming agents, antifoaming agents, light-blocking agents, compatibilizing agents, antifoam agents, sequestering agents, neutralising agents and buffers, corrosion inhibitors, dyes, odorants, spreading agents, penetration aids, micronutrients, emollients, lubricants and sticking agents.

In addition, further, other biocidally active ingredients or compositions may be combined with the compositions of the invention and used in the methods of the invention and applied simultaneously or sequentially with the compositions of the invention. When applied simultaneously, these further active ingredients may be formulated together with the compositions of the invention or mixed in, for example, the spray tank. These further biocidally active ingredients may be fungicides, herbicides, insecticides, bactericides, acaricides, nematicides and/or plant growth regulators.

Pesticidal agents are referred to herein using their common name are known, for example, from "The Pesticide Manual", 15th Ed., British Crop Protection Council 2009.

In addition, the compositions of the invention may also be applied with one or more systemically acquired resistance inducers ("SAR" inducer). SAR inducers are known and described in, for example, United States Patent No. US 6,919,298 and include, for example, salicylates and the commercial SAR inducer acibenzolar-S-methyl.

The compounds of formula (I) according to the invention are normally used in the form of agrochemical compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula (I) according to the invention may be used in the form of (fungicidal) compositions for controlling or protecting against phytopathogenic microorganisms, comprising as active ingredient at least one compound of formula (I) or of at least one preferred individual compound as defined herein, in free form or in agrochemically usable salt form, and at least one of the above-mentioned adjuvants.

The invention therefore provides a composition, preferably a fungicidal composition, comprising at least one compound of formula (I) according to the invention, an agriculturally acceptable carrier and optionally an adjuvant. An agricultural acceptable carrier is for example a carrier that is suitable for agricultural use. Agricultural carriers are well known in the art. Preferably, said composition may comprise at least one or more pesticidally-active compounds, for example an additional fungicidal active ingredient in addition to the compound of formula (1).

The compound of formula (I) according to the invention may be the sole active ingredient of a composition or it may be admixed with one or more additional active ingredients such as a pesticide, fungicide, synergist, herbicide or plant growth regulator where appropriate. An additional active ingredient may, in some cases, result in unexpected synergistic activities.

Examples of suitable additional active ingredients include the following: acycloamino acid fungicides, aliphatic nitrogen fungicides, amide fungicides, anilide fungicides, antibiotic fungicides, aromatic fungicides, arsenical fungicides, aryl phenyl ketone fungicides, benzamide fungicides, benzanilide fungicides, benzimidazole fungicides, benzothiazole fungicides, botanical fungicides, bridged diphenyl fungicides, carbamate fungicides, carbanilate fungicides, conazole fungicides, copper fungicides, dicarboximide fungicides, dinitrophenol fungicides, dithiocarbamate fungicides, dithiolane fungicides, furamide fungicides, furanilide fungicides, hydrazide fungicides, imidazole fungicides, mercury fungicides, morpholine fungicides, organophosphorous fungicides, organotin fungicides, oxathiin fungicides, oxazole fungicides, phenylsulfamide fungicides, polysulfide fungicides, pyrazole fungicides, pyridine fungicides, pyrimidine fungicides, pyrrole fungicides, quaternary ammonium fungicides, quinoline fungicides, quinone fungicides, quinoxaline fungicides, strobilurin fungicides, sulfonanilide fungicides, thiadiazole fungicides, thiazole fungicides, thiazolidine fungicides, thiocarbamate fungicides, thiophene fungicides, triazine fungicides, triazole fungicides, triazolopyrimidine fungicides, urea fungicides, valinamide fungicides, and zinc fungicides.

Examples of suitable additional active ingredients include the following: petroleum oils, 1,1-bis(4-chlorophenyl)-2-ethoxyethanol, 2,4-dichlorophenyl benzenesulfonate, 2-fluoro-N-methyl-N-1-naphthylacetamide, 4-chlorophenyl phenylsulfone, acetoprole, aldoxycarb, amidithion, amidothioate, amiton, amiton hydrogen oxalate, amitraz, aramite, arsenous oxide, azobenzene, azothoate, benomyl, benoxa-fos, benzyl benzoate, bixafen, brofenvalerate, bromocyclen, bromophos, bromopropylate, buprofezin, butocarboxim, butoxycarboxim, butylpyridaben, calcium polysulfide, camphechlor, carbanolate, carbophenothion, cymiazole, chinomethionat, chlorbenside, chlordimeform, chlordimeform hydrochloride, chlorfenethol, chlorfenson, chlorfensulfide, chlorobenzilate, chloromebuform, chloromethiuron, chloropropylate, chlorthiophos, cinerin I, cinerin II, cinerins, closantel, coumaphos, crotamiton, crotoxyphos, cufraneb, cyanthoate, DCPM, DDT, demephion, demephion-O, demephion-S, demeton-methyl, demeton-O, demeton-O-methyl, demeton-S, demeton-S-methyl, demeton-S-methylsulfon, dichlofluanid, dichlorvos, dicliphos, dienochlor, dimefox, dinex, dinex-diclexine, dinocap-4, dinocap-6, dinocton, dinopenton, dinosulfon, dinoterbon, dioxathion, diphenyl sulfone, disulfiram, DNOC, dofenapyn, doramectin, endothion, eprinomectin, ethoate-methyl, etrimfos, fenazaflor, fenbutatin oxide, fenothiocarb, fenpyrad, fenpyroximate, fenpyrazamine, fenson, fentrifanil, flubenzimine, flucycloxuron, fluenetil, fluorbenside, FMC 1137, formetanate, formetanate hydrochloride, formparanate, gamma-HCH, glyodin, halfenprox, hexadecyl cyclopropanecarboxylate, isocarbophos, jasmolin I, jasmolin II, jodfenphos, lindane, malonoben, mecarbam, mephosfolan, mesulfen, methacrifos, methyl bromide, metolcarb, mexacarbate, milbemycin oxime, mipafox, monocrotophos, morphothion, moxidectin, naled, 4-chloro-2-(2-chloro-2-methyl-propyl)-5-[(6-iodo-3-pyridyl)methoxy]pyridazin-3-one, nifluridide, nikkomycins, nitrilacarb, nitrilacarb 1:1 zinc chloride complex, omethoate, oxydeprofos, oxydisulfoton, pp'-DDT, parathion, permethrin, phenkapton, phosalone, phosfolan, phosphamidon, polychloroterpenes, polynactins, proclonol, promacyl, propoxur, prothidathion, prothoate, pyrethrin I, pyrethrin II, pyrethrins, pyridaphenthion, pyrimitate, quinalphos, quintiofos, R-1492, phosglycin, rotenone, schradan, sebufos, selamectin, sophamide, SSI-121, sulfiram, sulfluramid, sulfotep, sulfur, diflovidazin, tau-fluvalinate, TEPP, terbam, tetradifon, tetrasul, thiafenox, thiocarboxime, thiofanox, thiometon, thioquinox, thuringiensin, triamiphos, triarathene, triazophos, triazuron, trifenofos, trinactin, vamidothion, vaniliprole, bethoxazin, copper dioctanoate, copper sulfate, cybutryne, dichlone, dichlorophen, endothal, fentin, hydrated lime, nabam, quinoclamine, quinonamid, simazine, triphenyltin acetate, triphenyltin hydroxide, crufomate, piperazine, thiophanate, chloralose, fenthion, pyridin-4-amine, strychnine, 1-hydroxy-1H-pyridine-2-thione, 4-(quinoxalin-2-ylamino)benzenesulfonamide, 8-hydroxyquinoline sulfate, bronopol, copper hydroxide, cresol, dipyrithione, dodicin, fenaminosulf, formaldehyde, hydrargaphen, kasugamycin, kasugamycin hydrochloride hydrate, nickel bis(dimethyldithiocarbamate), nitrapyrin, octhilinone, oxolinic acid, oxytetracycline, potassium hydroxyquinoline sulfate, probenazole, streptomycin, streptomycin sesquisulfate, tecloftalam, thiomersal, Adoxophyes orana GV, Agrobacterium radiobacter, Amblyseius spp., Anagrapha falcifera NPV, Anagrus atomus, Aphelinus abdominalis, Aphidius colemani, Aphidoletes aphidimyza, Autographa californica NPV, Bacillus sphaericus Neide, Beauveria brongniartii, Chrysoperla carnea, Cryptolaemus montrouzieri, Cydia pomonella GV, Dacnusa sibirica, Diglyphus isaea, Encarsia formosa, Eretmocerus eremicus, Heterorhabditis bacteriophora and H. megidis, Hippodamia convergens, Leptomastix dactylopii, Macrolophus caliginosus, Mamestra brassicae NPV, Metaphycus helvolus, Metarhizium anisopliae var. acridum, Metarhizium anisopliae var. anisopliae, Neodiprion sertifer NPV and N. lecontei NPV, Orius spp., Paecilomyces fumosoroseus, Phytoseiulus persimilis, Steinernema bibionis, Steinernema carpocapsae, Steinernema feltiae, Steinernema glaseri, Steinernema riobrave, Steinernema riobravis, Steinernema scapterisci, Steinernema spp., Trichogramma spp., Typhlodromus occidentalis, Verticillium lecanii, apholate, bisazir, busulfan, dimatif, hemel, hempa, metepa, methiotepa, methyl apholate, morzid, penfluron, tepa, thiohempa, thiotepa, tretamine, uredepa, (E)-dec-5-en-1-yl acetate with (E)-dec-5-en-1-ol, (E)-tridec-4-en-1-yl acetate, (E)-6-methylhept-2-en-4-ol, (E,Z)-tetradeca-4,10-dien-1-yl acetate, (Z)-dodec-7-en-1-yl acetate, (Z)-hexadec-11-enal, (Z)-hexadec-11-en-1-yl acetate, (Z)-hexadec-13-en-11-yn-1-yl acetate, (Z)-icos-13-en-10-one, (Z)-tetradec-7-en-1-al, (Z)-tetradec-9-en-1-ol, (Z)-tetradec-9-en-1-yl acetate, (7E,9Z)-dodeca-7,9-dien-1-yl acetate, (9Z,11E)-tetradeca-9,11-dien-1-yl acetate, (9Z,12E)-tetradeca-9,12-dien-1-yl acetate, 14-methyloctadec-1-ene, 4-methylnonan-5-ol with 4-methylnonan-5-one, alpha-multistriatin, brevicomin, codlelure, codlemone, cuelure, disparlure, dodec-8-en-1-yl acetate, dodec-9-en-1-yl acetate, dodeca-8, 10-dien-1-yl acetate, dominicalure, ethyl 4-methyloctanoate, eugenol, frontalin, grandlure, grandlure I, grandlure II, grandlure III, grandlure IV, hexalure, ipsdienol, ipsenol, japonilure, lineatin, litlure, looplure, medlure, megatomoic acid, methyl eugenol, muscalure, octadeca-2,13-dien-1-yl acetate, octadeca-3,13-dien-1-yl acetate, orfralure, oryctalure, ostramone, siglure, sordidin, sulcatol, tetradec-11-en-1-yl acetate, trimedlure, trimedlure A, trimedlure B1, trimedlure B2, trimedlure C, trunc-call, 2-(octylthio)ethanol, butopyronoxyl, butoxy(polypropylene glycol), dibutyl adipate, dibutyl phthalate, dibutyl succinate, diethyltoluamide, dimethyl carbate, dimethyl phthalate, ethyl hexanediol, hexamide, methoquin-butyl, methylneodecanamide, oxamate, picaridin, 1-dichloro-1-nitroethane, 1,1-dichloro-2,2-bis(4-ethylphenyl)-ethane, 1,2-dichloropropane with 1,3-dichloropropene, 1-bromo-2-chloroethane, 2,2,2-trichloro-1-(3,4-dichlorophenyl)ethyl acetate, 2,2-dichlorovinyl 2-ethylsulfinylethyl methyl phosphate, 2-(1,3-dithiolan-2-yl)phenyl dimethylcarbamate, 2-(2-butoxyethoxy)ethyl thiocyanate, 2-(4,5-dimethyl-1,3-dioxolan-2-yl)phenyl methylcarbamate, 2-(4-chloro-3,5-xylyloxy)ethanol, 2-chlorovinyl diethyl phosphate, 2-imidazolidone, 2-isovalerylindan-1,3-dione, 2-methyl(prop-2-ynyl)aminophenyl methylcarbamate, 2-thiocyanatoethyl laurate, 3-bromo-1-chloroprop-1-ene, 3-methyl-1-phenylpyrazol-5-yl dimethylcarbamate, 4-methyl(prop-2-ynyl)amino-3,5-xylyl methylcarbamate, 5,5-dimethyl-3-oxocyclohex-1-enyl dimethylcarbamate, acethion, acrylonitrile, aldrin, allosamidin, allyxycarb, alpha-ecdysone, aluminium phosphide, aminocarb, anabasine, athidathion, azamethiphos, Bacillus thuringiensis delta endotoxins, barium hexafluorosilicate, barium polysulfide, barthrin, Bayer 22/190, Bayer 22408, beta-cyfluthrin, beta-cypermethrin, bioethanomethrin, biopermethrin, bis(2-chloroethyl) ether, borax, bromfenvinfos, bromo-DDT, bufencarb, butacarb, butathiofos, butonate, calcium arsenate, calcium cyanide, carbon disulfide, carbon tetrachloride, cartap hydrochloride, cevadine, chlorbicyclen, chlordane, chlordecone, chloroform, chloropicrin, chlorphoxim, chlorprazophos, cis-resmethrin, cismethrin, clocythrin, copper acetoarsenite, copper arsenate, copper oleate, coumithoate, cryolite, CS 708, cyanofenphos, cyanophos, cyclethrin, cythioate, d-tetramethrin, DAEP, dazomet, decarbofuran, diamidafos, dicapthon, dichlofenthion, dicresyl, dicyclanil, dieldrin, diethyl 5-methylpyrazol-3-yl phosphate, dilor, dimefluthrin, dimetan, dimethrin, dimethylvinphos, dimetilan, dinoprop, dinosam, dinoseb, diofenolan, dioxabenzofos, dithicrofos, DSP, ecdysterone, El 1642, EMPC, EPBP, etaphos, ethiofencarb, ethyl formate, ethylene dibromide, ethylene dichloride, ethylene oxide, EXD, fenchlorphos, fenethacarb, fenitrothion, fenoxacrim, fenpirithrin, fensulfothion, fenthion-ethyl, flucofuron, fosmethilan, fospirate, fosthietan, furathiocarb, furethrin, guazatine, guazatine acetates, sodium tetrathiocarbonate, halfenprox, HCH, HEOD, heptachlor, heterophos, HHDN, hydrogen cyanide, hyquincarb, IPSP, isazofos, isobenzan, isodrin, isofenphos, isolane, isoprothiolane, isoxathion, juvenile hormone I, juvenile hormone II, juvenile hormone III, kelevan, kinoprene, lead arsenate, leptophos, lirimfos, lythidathion, m-cumenyl methylcarbamate, magnesium phosphide, mazidox, mecarphon, menazon, mercurous chloride, mesulfenfos, metam, metam-potassium, metam-sodium, methanesulfonyl fluoride, methocrotophos, methoprene, methothrin, methoxychlor, methyl isothiocyanate, methylchloroform, methylene chloride, metoxadiazone, mirex, naftalofos, naphthalene, NC-170, nicotine, nicotine sulfate, nithiazine, nornicotine, O-5-dichloro-4-iodophenyl O-ethyl ethylphosphonothioate, O,O-diethyl O-4-methyl-2-oxo-2H-chromen-7-yl phosphorothioate, O,O-diethyl O-6-methyl-2-propylpyrimidin-4-yl phosphorothioate, O,O,O',O'-tetrapropyl dithiopyrophosphate, oleic acid, para-dichlorobenzene, parathion-methyl, pentachlorophenol, pentachlorophenyl laurate, PH 60-38, phenkapton, phosnichlor, phosphine, phoxim-methyl, pirimetaphos, polychlorodicyclopentadiene isomers, potassium arsenite, potassium thiocyanate, precocene I, precocene II, precocene III, primidophos, profluthrin, promecarb, prothiofos, pyrazophos, pyresmethrin, quassia, quinalphos-methyl, quinothion, rafoxanide, resmethrin, rotenone, kadethrin, ryania, ryanodine, sabadilla, schradan, sebufos, SI-0009, thiapronil, sodium arsenite, sodium cyanide, sodium fluoride, sodium hexafluorosilicate, sodium pentachlorophenoxide, sodium selenate, sodium thiocyanate, sulcofuron, sulcofuron-sodium, sulfuryl fluoride, sulprofos, tar oils, tazimcarb, TDE, tebupirimfos, temephos, terallethrin, tetrachloroethane, thicrofos, thiocyclam, thiocyclam hydrogen oxalate, thionazin, thiosultap, thiosultap-sodium, tralomethrin, transpermethrin, triazamate, trichlormetaphos-3, trichloronat, trimethacarb, tolprocarb, triclopyricarb, triprene, veratridine, veratrine, XMC, zetamethrin, zinc phosphide, zolaprofos, meperfluthrin, tetramethylfluthrin, bis(tributyltin) oxide, bromoacetamide, ferric phosphate, niclosamide-olamine, tributyltin oxide, pyrimorph, trifenmorph, 1,2-dibromo-3-chloropropane, 1,3-dichloropropene, 3,4-dichlorotetrahydrothiophene 1,1-dioxide, 3-(4-chlorophenyl)-5-methylrhodanine, 5-methyl-6-thioxo-1,3,5-thiadiazinan-3-ylacetic acid, 6-isopentenylaminopurine, anisiflupurin, benclothiaz, cytokinins, DCIP, furfural, isamidofos, kinetin, Myrothecium verrucaria composition, tetrachlorothiophene, xylenols, zeatin, potassium ethylxanthate, acibenzolar, acibenzolar-S-methyl, Reynoutria sachalinensis extract, alpha-chlorohydrin, antu, barium carbonate, bisthiosemi, brodifacoum, bromadiolone, bromethalin, chlorophacinone, cholecalciferol, coumachlor, coumafuryl, coumatetralyl, crimidine, difenacoum, difethialone, diphacinone, ergocalciferol, flocoumafen, fluoroacetamide, flupropadine, flupropadine hydrochloride, norbormide, phosacetim, phosphorus, pindone, pyrinuron, scilliroside, sodium fluoroacetate, thallium sulfate, warfarin, 2-(2-butoxyethoxy)ethyl piperonylate, 5-(1,3-benzodioxol-5-yl)-3-hexylcyclohex-2-enone, farnesol with nerolidol, verbutin, MGK 264, piperonyl butoxide, piprotal, propyl isomer, S421, sesamex, sesasmolin, sulfoxide, anthraquinone, copper naphthenate, copper oxychloride, dicyclopentadiene, thiram, zinc naphthenate, ziram, imanin, ribavirin, chloroinconazide, mercuric oxide, thiophanate-methyl, azaconazole, bitertanol, bromuconazole, cyproconazole, difenoconazole, diniconazole, epoxiconazole, fenbuconazole, fluquinconazole, flusilazole, flutriafol, furametpyr, hexaconazole, imazalil, imibenconazole, ipconazole, metconazole, myclobutanil, paclobutrazole, pefurazoate, penconazole, prothioconazole, pyrifenox, prochloraz, propiconazole, pyrisoxazole, simeconazole, tebuconazole, tetraconazole, triadimefon, triadimenol, triflumizole, triticonazole, ancymidol, fenarimol, nuarimol, bupirimate, dimethirimol, ethirimol, dodemorph, fenpropidine, fenpropimorph, spiroxamine, tridemorph, cyprodinil, mepanipyrim, pyrimethanil, fenpiclonil, fludioxonil, benalaxyl, furalaxyl, metalaxyl, R-metalaxyl, ofurace, oxadixyl, carbendazim, debacarb, fuberidazole, thiabendazole, chlozolinate, dichlozoline, myclozoline, procymidone, vinclozoline, boscalid, carboxin, fenfuram, flutolanil, mepronil, oxycarboxin, penthiopyrad, thifluzamide, dodine, iminoctadine, azoxystrobin, dimoxystrobin, enestroburin, fenaminstrobin, flufenoxystrobin, fluoxastrobin, kresoxim-methyl, metominostrobin, trifloxystrobin, orysastrobin, picoxystrobin, pyraclostrobin, pyrametostrobin, pyraoxystrobin, ferbam, mancozeb, maneb, metiram, propineb, zineb, captafol, captan, fluoroimide, folpet, tolylfluanid, bordeaux mixture, copper oxide, mancopper, oxine-copper, nitrothal-isopropyl, edifenphos, iprobenphos, phosdiphen, tolclofos-methyl, anilazine, benthiavalicarb, blasticidin-S, chloroneb, chlorothalonil, cyflufenamid, cymoxanil, cyclobutrifluram, diclocymet, diclomezine, dicloran, diethofencarb, dimethomorph, flumorph, dithianon, ethaboxam, etridiazole, famoxadone, fenamidone, fenoxanil, ferimzone, fluazinam, flumetylsulforim,fluopicolide, fluoxytioconazole, flusulfamide, fluxapyroxad, fenhexamid, fosetylaluminium, hymexazol, iprovalicarb, cyazofamid, methasulfocarb, metrafenone, pencycuron, phthalide, polyoxins, propamocarb, pyribencarb, proquinazid, pyroquilon, pyriofenone, quinoxyfen, quintozene, tiadinil, triazoxide, tricyclazole, triforine, validamycin, valifenalate, zoxamide, mandipropamid, flubeneteram, isopyrazam, sedaxane, benzovindiflupyr, pydiflumetofen, 3-difluoromethyl-1-methyl-1H-pyrazole-4-carboxylic acid (3',4',5'-trifluoro-biphenyl-2-yl)-amide, isoflucypram, isotianil, dipymetitrone, 6-ethyl-5,7-dioxo-pyrrolo[4,5][1,4]dithiino[1,2-c]isothiazole-3-carbonitrile, 2-(difluoromethyl)-N-[3-ethyl-1,1-dimethyl-indan-4-yl]pyridine-3-carboxamide, 4-(2,6-difluorophenyl)-6-methyl-5-phenyl-pyridazine-3-carbonitrile, (R)-3-(difluoromethyl)-1-methyl-N-[1,1,3-trimethylindan-4-yl]pyrazole-4-carboxamide, 4-(2-bromo-4-fluoro-phenyl)-N-(2-chloro-6-fluoro-phenyl)-2,5-dimethyl-pyrazol-3-amine, 4-(2-bromo-4-fluorophenyl)-N-(2-chloro-6-fluorophenyl)-1,3-dimethyl-1H-pyrazol-5-amine, fluindapyr, coumethoxystrobin (jiaxiangjunzhi), Ivbenmixianan, dichlobentiazox, mandestrobin, 3-(4,4-difluoro-3,4-dihydro-3,3-dimethylisoquinolin-1-yl)quinolone, 2-[2-fluoro-6-[(8-fluoro-2-methyl-3-quinolyl)oxy]phenyl]-propan-2-ol, oxathiapiprolin, tert-butyl N-[6-[[[(1-methyltetrazol-5-yl)-phenyl-methylene]amino]oxymethyl]-2-pyridyl]carbamate, pyraziflumid, inpyrfluxam, trolprocarb, mefentrifluconazole, ipfentrifluconazole, 2-(difluoromethyl)-N-[(3R)-3-ethyl-1,1-dimethyl-indan-4-yl]pyridine-3-carboxamide, N'-(2,5-dimethyl-4-phenoxy-phenyl)-N-ethyl-N-methyl-formamidine, N'-[4-(4,5-dichlorothiazol-2-yl)oxy-2,5-dimethyl-phenyl]-N-ethyl-N-methyl-formamidine, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate, but-3-ynyl N-[6-[[(Z)-[(1-methyltetrazol-5-yl)-phenyl-methylene]amino]oxymethyl]-2-pyridyl]carbamate, methyl-N-[[5-[4-(2,4-dimethyl-phenyl)-triazol-2-yl]-2-methyl-phenyl]methyl]carbamate, 3-chloro-6-methyl-5-phenyl-4-(2,4,6-trifluoro-phenyl)-pyridazine, pyridachlometyl, 3-(difluoromethyl)-1-methyl-N-[1,1,3-trimethylindan-4-yl]pyrazole-4-carboxamide, 1-[2-[[1-(4-chlorophenyl)pyrazol-3-yl]oxymethyl]-3-methyl-phenyl]-4-methyl-tetrazol-5-one, 1-methyl-4-[3-methyl-2-[[2-methyl-4-(3,4,5-trimethylpyrazol-1-yl)phenoxy]methyl]-phenyl]-tetrazol-5-one, aminopyrifen, ametoctradin, amisulbrom, penflufen, (Z,2E)-5-[1-(4-chlorophenyl)pyrazol-3-yl]oxy-2-methoxyimino-N,3-dimethyl-pent-3-enamide, florylpicoxamid, fenpicoxamid, metarylpicoxamid, tebufloquin, ipflufenoquin, quinofumelin, isofetamid, ethyl 1-[[4-[[2-(trifluoromethyl)-1,3-dioxolan-2-yl]methoxy]phenyl]methyl]pyrazole-3-carboxylate (may be prepared from the methods described in WO2020/056090), ethyl 1-[[4-[(Z)-2-ethoxy-3,3,3-trifluoro-prop-1-enoxy]phenyl]methyl]pyrazole-3-carboxylate (may be prepared from the methods described in WO2020/056090), methyl N-[[4-[1-(4-cyclopropyl-2,6-difluoro-phenyl)pyrazol-4-yl]-2-methyl-phenyl]-methyl]carbamate (may be prepared from the methods described in WO2020/097012), methyl N-[[4-[1-(2,6-difluoro-4-isopropyl-phenyl)pyrazol-4-yl]-2-methyl-phenyl]methyl]carbamate (may be prepared from the methods described in WO2020/097012), 6-chloro-3-(3-cyclopropyl-2-fluoro-phenoxy)-N-[2-(2,4-dimethylphenyl)-2,2-difluoro-ethyl]-5-methylpyridazine-4-carboxamide (may be prepared from the methods described in WO2020/109391), 6-chloro-N-[2-(2-chloro-4-methyl-phenyl)-2,2-difluoro-ethyl]-3-(3-cyclopropyl-2-fluoro-phenoxy)-5-methylpyridazine-4-carboxamide (may be prepared from the methods described in WO2020/109391), 6-chloro-3-(3-cyclopropyl-2-fluoro-phenoxy)-N-[2-(3,4-dimethylphenyl)-2,2-difluoro-ethyl]-5-methyl-pyridazine-4-carboxamide (may be prepared from the methods described in WO2020/109391),N-[2-[2,4-dichloro-phenoxy]phenyl]-3-(difluoromethyl)-1-methyl-pyrazole-4-carboxamide, N-[2-[2-chloro-4-(trifluoromethyl)-phenoxy]phenyl]-3-(difluoromethyl)-1-methyl-pyrazole-4-carboxamide, benzothiostrobin, phenamacril, 5-amino-1,3,4-thiadiazole-2-thiol zinc salt (2:1), fluopyram, flufenoxadiazam, flutianil, fluopimomide, pyrapropoyne, picarbutrazox, 2-(difluoromethyl)-N-(3-ethyl-1,1-dimethyl-indan-4-yl)pyridine-3-carboxamide, 2-(difluoromethyl)-N-((3R)-1,1,3- trimethylindan-4-yl) pyridine-3-carboxamide, 4-[[6-[2-(2,4-difluorophenyl)-1,1-difluoro-2-hydroxy-3-(1,2,4-triazol-1-yl)propyl]-3-pyridyl]oxy]benzonitrile, metyltetraprole, 2-(difluoromethyl)-N-((3R)-1,1,3-trimethylindan-4-yl)pyridine-3-carboxamide, α-(1,1-dimethylethyl)-α-[4'-(trifluoromethoxy)[1,1'-biphenyl]-4-yl]-5-pyrimidinemethanol, fluoxapiprolin, enoxastrobin, methyl (Z)-3-methoxy-2-[2-methyl-5-[4-(trifluoromethyl)triazol-2-yl]phenoxy]prop-2-enoate, methyl (Z)-3-methoxy-2-[2-methyl-5-(4-propyltriazol-2-yl)phenoxy]prop-2-enoate, methyl (Z)-2-[5-(3-isopropylpyrazol-1-yl)-2-methyl-phenoxy]-3-methoxy-prop-2-enoate, methyl (Z)-3-methoxy-2-[2-methyl-5-(3-propylpyrazol-1-yl)phenoxy]prop-2-enoate, methyl(Z)-3-methoxy-2-[2-methyl-5-[3-(trifluoromethyl)-pyrazol-1-yl]phenoxy]prop-2-enoate (these compounds may be prepared from the methods described in WO2020/079111), methyl (Z)-2-(5-cyclohexyl-2-methyl-phenoxy)-3-methoxy-prop-2-enoate, methyl (Z)-2-(5-cyclopentyl-2-methyl-phenoxy)-3-methoxy-prop-2-enoate (these compounds may be prepared from the methods described in WO2020/193387), 4-[[6-[2-(2,4-difluorophenyl)-1,1-difluoro-2-hydroxy-3-(1,2,4-triazol-1-yl)propyl]-3-pyridyl]oxy] benzonitrile, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile, trinexapac, coumoxystrobin, zhongshengmycin, thiodiazole copper, zinc thiazole, amectotractin, iprodione, seboctylamine, N'-[5-bromo-2-methyl-6-[(1S)-1-methyl-2-propoxy-ethoxy]-3-pyridyl]-N-ethyl-N-methyl-formamidine, N'-[5-bromo-2-methyl-6-[(1R)-1-methyl-2-propoxy-ethoxy]-3-pyridyl]-N-ethyl-N-methyl-formamidine, N'-[5-bromo-2-methyl-6-(1-methyl-2-propoxy-ethoxy)-3-pyridyl]-N-ethyl-N-methyl-formamidine, N'-[5-chloro-2-methyl-6-(1-methyl-2-propoxy-ethoxy)-3-pyridyl]-N-ethyl-N-methyl-formamidine, N'-[5-bromo-2-methyl-6-(1-methyl-2-propoxy-ethoxy)-3-pyridyl]-N-isopropyl-N-methyl-formamidine (these compounds may be prepared from the methods described in WO2015/155075); N'-[5-bromo-2-methyl-6-(2-propoxypropoxy)-3-pyridyl]-N-ethyl-N-methyl-formamidine (this compound may be prepared from the methods described in IPCOM000249876D); N-isopropyl-N'-[5-methoxy-2-methyl-4-(2,2,2-trifluoro-1-hydroxy-1-phenyl-ethyl)-phenyl]-N-methyl-formamidine, N'-[4-(1-cyclopropyl-2,2,2-trifluoro-1-hydroxy-ethyl)-5-methoxy-2-methylphenyl]-N-isopropyl-N-methyl-formamidine (these compounds may be prepared from the methods described in WO2018/228896); N-ethyl-N'-[5-methoxy-2-methyl-4-[(2-trifluoromethyl)oxetan-2-yl]phenyl]-N-methyl-formamidine, N-ethyl-N'-[5-methoxy-2-methyl-4-[(2-trifluoromethyl)tetrahydrofuran-2-yl]phenyl]-N-methyl-formamidine (these compounds may be prepared from the methods described in WO2019/110427); N-[(1R)-1-benzyl-3-chloro-1-methyl-but-3-enyl]-8-fluoro-quinoline-3-carboxamide, N-[(1S)-1-benzyl-3-chloro-1-methyl-but-3-enyl]-8-fluoro-quinoline-3-carboxamide, N-[(1R)-1-benzyl-3,3,3-trifluoro-1-methyl-propyl]-8-fluoro-quinoline-3-carboxamide, N-[(1S)-1-benzyl-3,3,3-trifluoro-1-methylpropyl]-8-fluoro-quinoline-3-carboxamide, N-[(1R)-1-benzyl-1,3-dimethyl-butyl]-7,8-difluoro-quinoline-3-carboxamide, N-[(1S)-1-benzyl-1,3-dimethyl-butyl]-7,8-difluoro-quinoline-3-carboxamide, 8-fluoro-N-[(1R)-1-[(3-fluorophenyl)methyl]-1,3-dimethyl-butyl]quinoline-3-carboxamide, 8-fluoro-N-[(1S)-1-[(3-fluorophenyl)methyl]-1,3-dimethyl-butyl]quinoline-3-carboxamide, N-[(1R)-1-benzyl-1,3-dimethyl-butyl]-8-fluoro-quinoline-3-carboxamide, N-[(1S)-1-benzyl-1,3-dimethyl-butyl]-8-fluoro-quinoline-3-carboxamide, N-((1R)-1-benzyl-3-chloro-1-methyl-but-3-enyl)-8-fluoro-quinoline-3-carboxamide, N-((1S)-1-benzyl-3-chloro-1-methyl-but-3-enyl)-8-fluoro-quinoline-3-carboxamide (these compounds may be prepared from the methods described in WO2017/153380); 1-(6,7-dimethylpyrazolo[1,5-a]pyridin-3-yl)-4,4,5-trifluoro-3,3-dimethyl-isoquinoline, 1-(6,7-dimethylpyrazolo[1,5-a]pyridin-3-yl)-4,4,6-trifluoro-3,3-dimethyl-isoquinoline, 4,4-difluoro-3,3-dimethyl-1-(6-methylpyrazolo[1,5-a]pyridin-3-yl)isoquinoline, 4,4-difluoro-3,3-dimethyl-1-(7-methylpyrazolo[1,5-a]pyridin-3-yl)isoquinoline, 1-(6-chloro-7-methyl-pyrazolo[1,5-a]pyridin-3-yl)-4,4-difluoro-3,3-dimethyl-isoquinoline (these compounds may be prepared from the methods described in WO02017/025510); 1-(4,5-dimethylbenzimidazol-1-yl)-4,4,5-trifluoro-3,3-dimethyl-isoquinoline, 1-(4,5-dimethylbenzimidazol-1-yl)-4,4-difluoro-3,3-dimethyl-isoquinoline, 6-chloro-4,4-difluoro-3,3-dimethyl-1-(4-methylbenzimidazol-1-yl)isoquinoline, 4,4-difluoro-1-(5-fluoro-4-methyl-benzimidazol-1-yl)-3,3-dimethyl-isoquinoline, 3-(4,4-difluoro-3,3-dimethyl-1-isoquinolyl)-7,8-dihydro-6H-cyclopenta[e]benzimidazole (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide, N,2-dimethoxy-N-[[4-[5-(trifluoromethyl)-1,2,4-oxadiazol-3-yl]phenyl]methyl]propanamide, N-ethyl-2-methyl-N-[[4-[5-(trifluoromethyl)-1,2,4-oxadiazol-3-yl]phenyl]-methyl]propanamide, 1-methoxy-3-methyl-1-[[4-[5-(trifluoromethyl)-1,2,4-oxadiazol-3-yl]phenyl]-methyl]-urea, 1,3-dimethoxy-1-[[4-[5-(trifluoromethyl)-1,2,4-oxadiazol-3-yl]-phenyl]methyl]urea, 3-ethyl-1-methoxy-1-[[4-[5-(trifluoromethyl)-1,2,4-oxadiazol-3-yl]phenyl]methyl]urea, N-[[4-[5-(trifluoromethyl)-1,2,4-oxadiazol-3-yl]phenyl]methyl]propanamide, 4,4-dimethyl-2-[[4-[5-(trifluoromethyl)-1,2,4-oxadiazol-3-yl]-phenyl]methyl]isoxazolidin-3-one, 5,5-dimethyl-2-[[4-[5-(trifluoromethyl)-1,2,4-oxadiazol-3-yl]phenyl]-methyl]isoxazolidin-3-one, ethyl 1-[[4-[5-(trifluoromethyl)-1,2,4-oxadiazol-3-yl]phenyl]methyl]pyrazole-4-carboxylate, N,N-dimethyl-1-[[4-[5-(trifluoromethyl)-1,2,4-oxadiazol-3-yl]-phenyl]methyl]-1,2,4-triazol-3-amine (these compounds may be prepared from the methods described in WO2017/055473, WO2017/055469, WO2017/093348 and WO2017/118689); 2-[6-(4-chlorophenoxy)-2-(trifluoromethyl)-3-pyridyl]-1-(1,2,4-triazol-1-yl)propan-2-ol (this compound may be prepared from the methods described in WO2017/029179); 2-[6-(4-bromophenoxy)-2-(trifluoromethyl)-3-pyridyl]-1-(1,2,4-triazol-1-yl)propan-2-ol (this compound may be prepared from the methods described in WO2017/029179); 3-[2-(1-chlorocyclopropyl)-3-(2-fluorophenyl)-2-hydroxy-propyl]imidazole-4-carbonitrile (this compound may be prepared from the methods described in WO2016/156290); 3-[2-(1-chlorocyclopropyl)-3-(3-chloro-2-fluoro-phenyl)-2-hydroxy-propyl]imidazole-4-carbonitrile (this compound may be prepared from the methods described in WO2016/156290); (4-phenoxyphenyl)methyl 2-amino-6-methyl-pyridine-3-carboxylate (this compound may be prepared from the methods described in WO2014/006945); 2,6-Dimethyl-1H,5H-[1,4]dithiino[2,3-c:5,6-c']dipyrrole-1,3,5,7(2H,6H)-tetrone (this compound may be prepared from the methods described in WO2011/138281); N-methyl-4-[5-(trifluoromethyl)-1,2,4-oxadiazol-3-yl]benzenecarbothioamide; N-methyl-4-[5-(trifluoromethyl)-1,2,4-oxadiazol-3-yl]benzamide; (Z,2E)-5-[1-(2,4-dichlorophenyl)pyrazol-3-yl]oxy-2-methoxyimino-N,3-dimethyl-pent-3-enamide (this compound may be prepared from the methods described in WO2018/153707); N'-(2-chloro-5-methyl-4-phenoxy-phenyl)-N-ethyl-N-methyl-formamidine; N'-[2-chloro-4-(2-fluorophenoxy)-5-methyl-phenyl]-N-ethyl-N-methyl-formamidine (this compound may be prepared from the methods described in WO2016/202742); 2-(difluoromethyl)-N-[(3S)-3-ethyl-1,1-dimethyl-indan-4-yl]pyridine-3-carboxamide (this compound may be prepared from the methods described in WO2014/095675); (5-methyl-2-pyridyl)-[4-[5-(trifluoromethyl)-1,2,4-oxadiazol-3-yl]phenyl]methanone, (3-methylisoxazol-5-yl)-[4-[5-(trifluoromethyl)-1,2,4-oxadiazol-3-yl]phenyl]methanone (these compounds may be prepared from the methods described in WO2017/220485); 2-oxo-N-propyl-2-[4-[5-(trifluoromethyl)-1,2,4-oxadiazol-3-yl]phenyl]acetamide (this compound may be prepared from the methods described in WO2018/065414); ethyl 1-[[5-[5-(trifluoromethyl)-1,2,4-oxadiazol-3-yl]-2-thienyl]methyl]pyrazole-4-carboxylate (this compound may be prepared from the methods described in WO2018/158365); 2,2-difluoro-N-methyl-2-[4-[5-(trifluoromethyl)-1,2,4-oxadiazol-3-yl]phenyl]acetamide, N-[(E)-methoxyiminomethyl]-4-[5-(trifluoromethyl)-1,2,4-oxadiazol-3-yl]benzamide, N-[(Z)-methoxyiminomethyl]-4-[5-(trifluoromethyl)-1,2,4-oxadiazol-3-yl]benzamide, N-[N-methoxy-methyl-carbonimidoyl]-4-[5-(trifluoromethyl)-1,2,4-oxadiazol-3-yl]benzamide (these compounds may be prepared from the methods described in WO2018/202428).

The compounds of the invention may also be used in combination with anthelmintic agents. Such anthelmintic agents include, compounds selected from the macrocyclic lactone class of compounds such as ivermectin, avermectin, abamectin, emamectin, eprinomectin, doramectin, selamectin, moxidectin, nemadectin and milbemycin derivatives as described in EP0357460, EP0444964 and EP0594291. Additional anthelmintic agents include semisynthetic and biosynthetic avermectin/milbemycin derivatives such as those described in US5015630, WO9415944 and WO9522552. Additional anthelmintic agents include the benzimidazoles such as albendazole, cambendazole, fenbendazole, flubendazole, mebendazole, oxfendazole, oxibendazole, parbendazole, and other members of the class. Additional anthelmintic agents include imidazothiazoles and tetrahydropyrimidines such as tetramisole, levamisole, pyrantel pamoate, oxantel or morantel. Additional anthelmintic agents include flukicides, such as triclabendazole and clorsulon and the cestocides, such as praziquantel and epsiprantel.

The compounds of the invention may be used in combination with derivatives and analogues of the paraherquamide/marcfortine class of anthelmintic agents, as well as the antiparasitic oxazolines such as those disclosed in US5478855, US4639771 and DE19520936.

The compounds of the invention may be used in combination with derivatives and analogues of the general class of dioxomorpholine antiparasitic agents as described in WO9615121 and also with anthelmintic active cyclic depsipeptides such as those described in WO9611945, WO9319053, WO 9325543, EP0626375, EP0382173, WO9419334, EP0382173, and EP0503538.

The compounds of the invention may be used in combination with other ectoparasiticides; for example, fipronil; pyrethroids; organophosphates; insect growth regulators such as lufenuron; ecdysone agonists such as tebufenozide and the like; neonicotinoids such as imidacloprid and the like.

The compounds of the invention may be used in combination with terpene alkaloids, for example those described in WO95/19363 or WO04/72086, particularly the compounds disclosed therein.

Other examples of such biologically active compounds that the compounds of the invention may be used in combination with include but are not restricted to the following:
Organophosphates: acephate, azamethiphos, azinphos-ethyl, azinphos- methyl, bromophos, bromophos-ethyl, cadusafos, chlorethoxyphos, chlorpyrifos, chlorfenvinphos, chlormephos, demeton, demeton-S-methyl, demeton-S-methyl sulphone, dialifos, diazinon, dichlorvos, dicrotophos, dimethoate, disulfoton, ethion, ethoprophos, etrimfos, famphur, fenamiphos, fenitrothion, fensulfothion, fenthion, flupyrazofos, fonofos, formothion, fosthiazate, heptenophos, isazophos, isothioate, isoxathion, malathion, methacriphos, methamidophos, methidathion, methyl- parathion, mevinphos, monocrotophos, naled, omethoate, oxydemeton-methyl, paraoxon, parathion, parathion-methyl, phenthoate, phosalone, phosfolan, phosphocarb, phosmet, phosphamidon, phorate, phoxim, pirimiphos, pirimiphos- methyl, profenofos, propaphos, propetamphos, prothiofos, pyraclofos, pyridaphenthion, quinalphos, sulprophos, temephos, terbufos, tebupirimfos, tetrachlorvinphos, thimeton, triazophos, trichlorfon, vamidothion.

Carbamates: alanycarb, aldicarb, 2-sec-butylphenyl methylcarbamate, benfuracarb, carbaryl, carbofuran, carbosulfan, cloethocarb, ethiofencarb, fenoxycarb, fenthiocarb, furathiocarb, HCN-801, isoprocarb, indoxacarb, methiocarb, methomyl, 5-methyl-m-cumenylbutyryl(methyl)carbamate, oxamyl, pirimicarb, propoxur, thiodicarb, thiofanox, triazamate, UC-51717.

Pyrethroids: acrinathin, allethrin, alphametrin, 5-benzyl-3-furylmethyl (E)-(1R)-cis-2,2-dimethyl-3-(2-oxothiolan-3-ylidenemethyl)cyclopropanecarboxylate, bifenthrin, beta-cyfluthrin, cyfluthrin, a-cypermethrin, beta-cypermethrin, bioallethrin, bioallethrin((S)-cyclopentylisomer), bioresmethrin, bifenthrin, NCI-85193, cycloprothrin, cyhalothrin, cythithrin, cyphenothrin, deltamethrin, empenthrin, esfenvalerate, etofenprox, fenfluthrin, fenpropathrin, fenvalerate, flucythrinate, flumethrin, fluvalinate (D-isomer), imiprothrin, cyhalothrin, lambda-cyhalothrin, permethrin, phenothrin, prallethrin, pyrethrins (natural products), resmethrin, tetramethrin, transfluthrin, theta-cypermethrin, silafluofen, t-fluvalinate, tefluthrin, tralomethrin, zeta-cypermethrin.

Arthropod growth regulators: a) chitin synthesis inhibitors: benzoylureas: chlorfluazuron, diflubenzuron, fluazuron, flucycloxuron, flufenoxuron, hexaflumuron, lufenuron, novaluron, teflubenzuron, triflumuron, buprofezin, diofenolan, hexythiazox, etoxazole, chlorfentazine; b) ecdysone antagonists: halofenozide, methoxyfenozide, tebufenozide; c) juvenoids: pyriproxyfen, methoprene (including S-methoprene), fenoxycarb; d) lipid biosynthesis inhibitors: spirodiclofen.

Other antiparasitics: acequinocyl, amitraz, AKD-1022, ANS-118, azadirachtin, Bacillus thuringiensis, bensultap, bifenazate, binapacryl, bromopropylate, BTG-504, BTG-505, camphechlor, cartap, chlorobenzilate, chlordimeform, chlorfenapyr, chromafenozide, clothianidine, cyromazine, diacloden, diafenthiuron, DBI-3204, dinactin, dihydroxymethyldihydroxypyrrolidine, dinobuton, dinocap, endosulfan, ethiprole, etofenprox, fenazaquin, flumite, MTI- 800, fenpyroximate, fluacrypyrim, flubenzimine, flubrocythrinate, flufenzine, flufenprox, fluproxyfen, halofenprox, hydramethylnon, IKI-220, kanemite, NC-196, neem guard, nidinorterfuran, nitenpyram, SD-35651, WL-108477, pirydaryl, propargite, protrifenbute, pymethrozine, pyridaben, pyrimidifen, NC-1111, R-195,RH-0345, RH-2485, RYI-210, S-1283, S-1833, SI-8601, silafluofen, silomadine, spinosad, tebufenpyrad, tetradifon, tetranactin, thiacloprid, thiocyclam, thiamethoxam, tolfenpyrad, triazamate, triethoxyspinosyn, trinactin, verbutin, vertalec, YI-5301.

Biological agents: Bacillus thuringiensis ssp aizawai, kurstaki, Bacillus thuringiensis delta endotoxin, baculovirus, entomopathogenic bacteria, virus and fungi.

Bactericides: chlortetracycline, oxytetracycline, streptomycin.

Other biological agents: enrofloxacin, febantel, penethamate, moloxicam, cefalexin, kanamycin, pimobendan, clenbuterol, omeprazole, tiamulin, benazepril, pyriprole, cefquinome, florfenicol, buserelin, cefovecin, tulathromycin, ceftiour, carprofen, metaflumizone, praziquarantel, triclabendazole.

The following mixtures of the compounds of Formula (I) with active ingredients are preferred. The abbreviation "TX" means one compound selected from the group consisting of the compounds as represented in Tables C1-C23 or Table T1 (below): a compound selected from the group of substances consisting of petroleum oils + TX, 1,1-bis(4-chlorophenyl)-2-ethoxyethanol + TX, 2,4-dichlorophenyl benzenesulfonate + TX, 2-fluoro-N-methyl-N-1-naphthylacetamide + TX, 4-chlorophenyl phenyl sulfone + TX, acetoprole + TX, aldoxycarb + TX, amidithion + TX, amidothioate + TX, amiton + TX, amiton hydrogen oxalate + TX, amitraz + TX, aramite + TX, arsenous oxide + TX, azobenzene + TX, azothoate + TX, benomyl + TX, benoxafos + TX, benzyl benzoate + TX, bixafen + TX, brofenvalerate + TX, bromocyclen + TX, bromophos + TX, bromopropylate + TX, buprofezin + TX, butocarboxim + TX, butoxycarboxim + TX, butylpyridaben + TX, calcium polysulfide + TX, camphechlor + TX, carbanolate + TX, carbophenothion + TX, cymiazole + TX, chinomethionat + TX, chlorbenside + TX, chlordimeform + TX, chlordimeform hydrochloride + TX, chlorfenethol + TX, chlorfenson + TX, chlorfensulfide + TX, chlorobenzilate + TX, chloromebuform + TX, chloromethiuron + TX, chloropropylate + TX, chlorthiophos + TX, cinerin I + TX, cinerin II + TX, cinerins + TX, closantel + TX, coumaphos + TX, crotamiton + TX, crotoxyphos + TX, cufraneb + TX, cyanthoate + TX, DCPM + TX, DDT + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, dichlofluanid + TX, dichlorvos + TX, dicliphos + TX, dienochlor + TX, dimefox + TX, dinex + TX, dinex-diclexine + TX, dinocap-4 + TX, dinocap-6 + TX, dinocton + TX, dinopenton + TX, dinosulfon + TX, dinoterbon + TX, dioxathion + TX, diphenyl sulfone + TX, disulfiram + TX, DNOC + TX, dofenapyn + TX, doramectin + TX, endothion + TX, eprinomectin + TX, ethoate-methyl + TX, etrimfos + TX, fenazaflor + TX, fenbutatin oxide + TX, fenothiocarb + TX, fenpyrad + TX, fenpyroximate + TX, fenpyrazamine + TX, fenson + TX, fentrifanil + TX, flubenzimine + TX, flucycloxuron + TX, fluenetil + TX, fluorbenside + TX, FMC 1137 + TX, formetanate + TX, formetanate hydrochloride + TX, formparanate + TX, gamma-HCH + TX, glyodin + TX, halfenprox + TX, hexadecyl cyclopropanecarboxylate + TX, isocarbophos + TX, jasmolin I + TX, jasmolin II + TX, jodfenphos + TX, lindane + TX, malonoben + TX, mecarbam + TX, mephosfolan + TX, mesulfen + TX, methacrifos + TX, methyl bromide + TX, metolcarb + TX, mexacarbate + TX, milbemycin oxime + TX, mipafox + TX, monocrotophos + TX, morphothion + TX, moxidectin + TX, naled + TX, 4-chloro-2-(2-chloro-2-methylpropyl)-5-[(6-iodo-3-pyridyl)methoxy]pyridazin-3-one + TX, nifluridide + TX, nikkomycins + TX, nitrilacarb + TX, nitrilacarb 1:1 zinc chloride complex + TX, omethoate + TX, oxydeprofos + TX, oxydisulfoton + TX, pp'-DDT + TX, parathion + TX, permethrin + TX, phenkapton + TX, phosalone + TX, phosfolan + TX, phosphamidon + TX, polychloroterpenes + TX, polynactins + TX, proclonol + TX, promacyl + TX, propoxur + TX, prothidathion + TX, prothoate + TX, pyrethrin I + TX, pyrethrin II + TX, pyrethrins + TX, pyridaphenthion + TX, pyrimitate + TX, quinalphos + TX, quintiofos + TX, R-1492 + TX, phosglycin + TX, rotenone + TX, schradan + TX, sebufos + TX, selamectin + TX, sophamide + TX, SSI-121 + TX, sulfiram + TX, sulfluramid + TX, sulfotep + TX, sulfur + TX, diflovidazin + TX, tau-fluvalinate + TX, TEPP + TX, terbam + TX, tetradifon + TX, tetrasul + TX, thiafenox + TX, thiocarboxime + TX, thiofanox + TX, thiometon + TX, thioquinox + TX, thuringiensin + TX, triamiphos + TX, triarathene + TX, triazophos + TX, triazuron + TX, trifenofos + TX, trinactin + TX, vamidothion + TX, vaniliprole + TX, bethoxazin + TX, copper dioctanoate + TX, copper sulfate + TX, cybutryne + TX, dichlone + TX, dichlorophen + TX, endothal + TX, fentin + TX, hydrated lime + TX, nabam + TX, quinoclamine + TX, quinonamid + TX, simazine + TX, triphenyltin acetate + TX, triphenyltin hydroxide + TX, crufomate + TX, piperazine + TX, thiophanate + TX, chloralose + TX, fenthion + TX, pyridin-4-amine + TX, strychnine + TX, 1-hydroxy-1H-pyridine-2-thione + TX, 4-(quinoxalin-2-ylamino)benzenesulfonamide + TX, 8-hydroxyquinoline sulfate + TX, bronopol + TX, copper hydroxide + TX, cresol + TX, dipyrithione + TX, dodicin + TX, fenaminosulf + TX, formaldehyde + TX, hydrargaphen + TX, kasugamycin + TX, kasugamycin hydrochloride hydrate + TX, nickel bis(dimethyldithiocarbamate) + TX, nitrapyrin + TX, octhilinone + TX, oxolinic acid + TX, oxytetracycline + TX, potassium hydroxyquinoline sulfate + TX, probenazole + TX, streptomycin + TX, streptomycin sesquisulfate + TX, tecloftalam + TX, thiomersal + TX, Adoxophyes orana GV + TX, Agrobacterium radiobacter + TX, Amblyseius spp. + TX, Anagrapha falcifera NPV + TX, Anagrus atomus + TX, Aphelinus abdominalis + TX, Aphidius colemani + TX, Aphidoletes aphidimyza + TX, Autographa californica NPV + TX, Bacillus sphaericus Neide + TX, Beauveria brongniartii + TX, Chrysoperla carnea + TX, Cryptolaemus montrouzieri + TX, Cydia pomonella GV + TX, Dacnusa sibirica + TX, Diglyphus isaea + TX, Encarsia formosa + TX, Eretmocerus eremicus + TX, Heterorhabditis bacteriophora and H. megidis + TX, Hippodamia convergens + TX, Leptomastix dactylopii + TX, Macrolophus caliginosus + TX, Mamestra brassicae NPV + TX, Metaphycus helvolus + TX, Metarhizium anisopliae var. acridum + TX, Metarhizium anisopliae var. anisopliae + TX, Neodiprion sertifer NPV and N. lecontei NPV + TX, Orius spp. + TX, Paecilomyces fumosoroseus + TX, Phytoseiulus persimilis + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Trichogramma spp. + TX, Typhlodromus occidentalis + TX , Verticillium lecanii + TX, apholate + TX, bisazir + TX, busulfan + TX, dimatif + TX, hemel + TX, hempa + TX, metepa + TX, methiotepa + TX, methyl apholate + TX, morzid + TX, penfluron + TX, tepa + TX, thiohempa + TX, thiotepa + TX, tretamine + TX, uredepa + TX, (E)-dec-5-en-1-yl acetate with (E)-dec-5-en-1-ol + TX, (E)-tridec-4-en-1-yl acetate + TX, (E)-6-methylhept-2-en-4-ol + TX, (E,Z)-tetradeca-4,10-dien-1-yl acetate + TX, (Z)-dodec-7-en-1-yl acetate + TX, (Z)-hexadec-11-enal + TX, (Z)-hexadec-11-en-1-yl acetate + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, 14-methyloctadec-1-ene + TX, 4-methylnonan-5-ol with 4-methylnonan-5-one + TX, alpha-multistriatin + TX, brevicomin + TX, codlelure + TX, codlemone + TX, cuelure + TX, disparlure + TX, dodec-8-en-1-yl acetate + TX, dodec-9-en-1-yl acetate + TX, dodeca-8,10-dien-1-yl acetate + TX, dominicalure + TX, ethyl 4-methyloctanoate + TX, eugenol + TX, frontalin + TX, grandlure + TX, grandlure I + TX, grandlure II + TX, grandlure III + TX, grandlure IV + TX, hexalure + TX, ipsdienol + TX, ipsenol + TX, japonilure + TX, lineatin + TX, litlure + TX, looplure + TX, medlure + TX, megatomoic acid + TX, methyl eugenol + TX, muscalure + TX, octadeca-2,13-dien-1-yl acetate + TX, octadeca-3,13-dien-1-yl acetate + TX, orfralure + TX, oryctalure + TX, ostramone + TX, siglure + TX, sordidin + TX, sulcatol + TX, tetradec-11-en-1-yl acetate + TX, trimedlure + TX, trimedlure A + TX, trimedlure B₁ + TX, trimedlure B₂ + TX, trimedlure C + TX, trunc-call + TX, 2-(octylthio)ethanol + TX, butopyronoxyl + TX, butoxy(polypropylene glycol) + TX, dibutyl adipate + TX, dibutyl phthalate + TX, dibutyl succinate + TX, diethyltoluamide + TX, dimethyl carbate + TX, dimethyl phthalate + TX, ethyl hexanediol + TX, hexamide + TX, methoquin-butyl + TX, methylneodecanamide + TX, oxamate + TX, picaridin + TX, 1-dichloro-1-nitroethane + TX, 1,1-dichloro-2,2-bis(4-ethylphenyl)ethane + TX, 1,2-dichloropropane with 1,3-dichloropropene + TX, 1-bromo-2-chloroethane + TX, 2,2,2-trichloro-1-(3,4-dichlorophenyl)ethyl acetate + TX, 2,2-dichlorovinyl 2-ethylsulfinylethyl methyl phosphate + TX, 2-(1,3-dithiolan-2-yl)phenyl dimethylcarbamate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-chlorovinyl diethyl phosphate + TX, 2-imidazolidone + TX, 2-isovalerylindan-1,3-dione + TX, 2-methyl(prop-2-ynyl)aminophenyl methylcarbamate + TX, 2-thiocyanatoethyl laurate + TX, 3-bromo-1-chloroprop-1-ene + TX, 3-methyl-1-phenylpyrazol-5-yl dimethylcarbamate + TX, 4-methyl(prop-2-ynyl)amino-3,5-xylyl methylcarbamate + TX, 5,5-dimethyl-3-oxocyclohex-1-enyl dimethylcarbamate + TX, acethion + TX, acrylonitrile + TX, aldrin + TX, allosamidin + TX, allyxycarb + TX, alpha-ecdysone + TX, aluminium phosphide + TX, aminocarb + TX, anabasine + TX, athidathion + TX, azamethiphos + TX, Bacillus thuringiensis delta endotoxins + TX, barium hexafluorosilicate + TX, barium polysulfide + TX, barthrin + TX, Bayer 22/190 + TX, Bayer 22408 + TX, beta-cyfluthrin + TX, beta-cypermethrin + TX, bioethanomethrin + TX, biopermethrin + TX, bis(2-chloroethyl) ether + TX, borax + TX, bromfenvinfos + TX, bromo-DDT + TX, bufencarb + TX, butacarb + TX, butathiofos + TX, butonate + TX, calcium arsenate + TX, calcium cyanide + TX, carbon disulfide + TX, carbon tetrachloride + TX, cartap hydrochloride + TX, cevadine + TX, chlorbicyclen + TX, chlordane + TX, chlordecone + TX, chloroform + TX, chloropicrin + TX, chlorphoxim + TX, chlorprazophos + TX, cis-resmethrin + TX, cismethrin + TX, clocythrin + TX, copper acetoarsenite + TX, copper arsenate + TX, copper oleate + TX, coumithoate + TX, cryolite + TX, CS 708 + TX, cyanofenphos + TX, cyanophos + TX, cyclethrin + TX, cythioate + TX, d-tetramethrin + TX, DAEP + TX, dazomet + TX, decarbofuran + TX, diamidafos + TX, dicapthon + TX, dichlofenthion + TX, dicresyl + TX, dicyclanil + TX, dieldrin + TX, diethyl 5-methylpyrazol-3-yl phosphate + TX, dilor + TX, dimefluthrin + TX, dimetan + TX, dimethrin + TX, dimethylvinphos + TX, dimetilan + TX, dinoprop + TX, dinosam + TX, dinoseb + TX, diofenolan + TX, dioxabenzofos + TX, dithicrofos + TX, DSP + TX, ecdysterone + TX, El 1642 + TX, EMPC + TX, EPBP + TX, etaphos + TX, ethiofencarb + TX, ethyl formate + TX, ethylene dibromide + TX, ethylene dichloride + TX, ethylene oxide + TX, EXD + TX, fenchlorphos + TX, fenethacarb + TX, fenitrothion + TX, fenoxacrim + TX, fenpirithrin + TX, fensulfothion + TX, fenthion-ethyl + TX, flucofuron + TX, fosmethilan + TX, fospirate + TX, fosthietan + TX, furathiocarb + TX, furethrin + TX, guazatine + TX, guazatine acetates + TX, sodium tetrathiocarbonate + TX, halfenprox + TX, HCH + TX, HEOD + TX, heptachlor + TX, heterophos + TX, HHDN + TX, hydrogen cyanide + TX, hyquincarb + TX, IPSP + TX, isazofos + TX, isobenzan + TX, isodrin + TX, isofenphos + TX, isolane + TX, isoprothiolane + TX, isoxathion + TX, juvenile hormone I + TX, juvenile hormone II + TX, juvenile hormone III + TX, kelevan + TX, kinoprene + TX, lead arsenate + TX, leptophos + TX, lirimfos + TX, lythidathion + TX, m-cumenyl methylcarbamate + TX, magnesium phosphide + TX, mazidox + TX, mecarphon + TX, menazon + TX, mercurous chloride + TX, mesulfenfos + TX, metam + TX, metam-potassium + TX, metam-sodium + TX, methanesulfonyl fluoride + TX, methocrotophos + TX, methoprene + TX, methothrin + TX, methoxychlor + TX, methyl isothiocyanate + TX, methylchloroform + TX, methylene chloride + TX, metoxadiazone + TX, mirex + TX, naftalofos + TX, naphthalene + TX, NC-170 + TX, nicotine + TX, nicotine sulfate + TX, nithiazine + TX, nornicotine + TX, O-5-dichloro-4-iodophenyl O-ethyl ethylphosphonothioate + TX, O,O-diethyl O-4-methyl-2-oxo-2H-chromen-7-yl phosphorothioate + TX, O,O-diethyl 0-6-methyl-2-propylpyrimidin-4-yl phosphorothioate + TX, O,O,O',O'-tetrapropyl dithiopyrophosphate + TX, oleic acid + TX, para-dichlorobenzene + TX, parathion-methyl + TX, pentachlorophenol + TX, pentachlorophenyl laurate + TX, PH 60-38 + TX, phenkapton + TX, phosnichlor + TX, phosphine + TX, phoxim-methyl + TX, pirimetaphos + TX, polychlorodicyclopentadiene isomers + TX, potassium arsenite + TX, potassium thiocyanate + TX, precocene I + TX, precocene II + TX, precocene III + TX, primidophos + TX, profluthrin + TX, promecarb + TX, prothiofos + TX, pyrazophos + TX, pyresmethrin + TX, quassia + TX, quinalphos-methyl + TX, quinothion + TX, rafoxanide + TX, resmethrin + TX, rotenone + TX, kadethrin + TX, ryania + TX, ryanodine + TX, sabadilla + TX, schradan + TX, sebufos + TX, SI-0009 + TX, thiapronil + TX, sodium arsenite + TX, sodium cyanide + TX, sodium fluoride + TX, sodium hexafluorosilicate + TX, sodium pentachlorophenoxide + TX, sodium selenate + TX, sodium thiocyanate + TX, sulcofuron + TX, sulcofuron-sodium + TX, sulfuryl fluoride + TX, sulprofos + TX, tar oils + TX, tazimcarb + TX, TDE + TX, tebupirimfos + TX, temephos + TX, terallethrin + TX, tetrachloroethane + TX, thicrofos + TX, thiocyclam + TX, thiocyclam hydrogen oxalate + TX, thionazin + TX, thiosultap + TX, thiosultap-sodium + TX, tralomethrin + TX, transpermethrin + TX, triazamate + TX, trichlormetaphos-3 + TX, trichloronat + TX, trimethacarb + TX, tolprocarb + TX, triclopyricarb + TX, triprene + TX, veratridine + TX, veratrine + TX, XMC + TX, zetamethrin + TX, zinc phosphide + TX, zolaprofos + TX, meperfluthrin + TX, tetramethylfluthrin + TX, bis(tributyltin) oxide + TX, bromoacetamide + TX, ferric phosphate + TX, niclosamide-olamine + TX, tributyltin oxide + TX, pyrimorph + TX, trifenmorph + TX, 1,2-dibromo-3-chloropropane + TX, 1,3-dichloropropene + TX, 3,4-dichlorotetrahydrothiophene 1,1-dioxide + TX, 3-(4-chlorophenyl)-5-methylrhodanine + TX, 5-methyl-6-thioxo-1,3,5-thiadiazinan-3-ylacetic acid + TX, 6-isopentenylaminopurine + TX, 2-fluoro-N-(3-methoxyphenyl)-9H-purin-6-amine + TX, benclothiaz + TX, cytokinins + TX, DCIP + TX, furfural + TX, isamidofos + TX, kinetin + TX, Myrothecium verrucaria composition + TX, tetrachlorothiophene + TX, xylenols + TX, zeatin + TX, potassium ethylxanthate + TX ,acibenzolar + TX, acibenzolar-S-methyl + TX, Reynoutria sachalinensis extract + TX, alpha-chlorohydrin + TX, antu + TX, barium carbonate + TX, bisthiosemi + TX, brodifacoum + TX, bromadiolone + TX, bromethalin + TX, chlorophacinone + TX, cholecalciferol + TX, coumachlor + TX, coumafuryl + TX, coumatetralyl + TX, crimidine + TX, difenacoum + TX, difethialone + TX, diphacinone + TX, ergocalciferol + TX, flocoumafen + TX, fluoroacetamide + TX, flupropadine + TX, flupropadine hydrochloride + TX, norbormide + TX, phosacetim + TX, phosphorus + TX, pindone + TX, pyrinuron + TX, scilliroside + TX, sodium fluoroacetate + TX, thallium sulfate + TX, warfarin + TX, 2-(2-butoxyethoxy)ethyl piperonylate + TX, 5-(1,3-benzodioxol-5-yl)-3-hexylcyclohex-2-enone + TX, farnesol with nerolidol + TX, verbutin + TX, MGK 264 + TX, piperonyl butoxide + TX, piprotal + TX, propyl isomer + TX, S421 + TX, sesamex + TX, sesasmolin + TX, sulfoxide + TX, anthraquinone + TX, copper naphthenate + TX, copper oxychloride + TX, dicyclopentadiene + TX, thiram + TX, zinc naphthenate + TX, ziram + TX, imanin + TX, ribavirin + TX, mercuric oxide + TX, thiophanate-methyl + TX, azaconazole + TX, bitertanol + TX, bromuconazole + TX, cyproconazole + TX, difenoconazole + TX, diniconazole + TX, epoxiconazole + TX, fenbuconazole + TX, fluquinconazole + TX, flusilazole + TX, flutriafol + TX, furametpyr + TX, hexaconazole + TX, imazalil + TX, imibenconazole + TX, ipconazole + TX, metconazole + TX, myclobutanil + TX, paclobutrazole + TX, pefurazoate + TX, penconazole + TX, prothioconazole + TX, pyrifenox + TX, prochloraz + TX, propiconazole + TX, pyrisoxazole + TX, simeconazole + TX, tebuconazole + TX, tetraconazole + TX, triadimefon + TX, triadimenol + TX, triflumizole + TX, triticonazole + TX, ancymidol + TX, fenarimol + TX, nuarimol + TX, bupirimate + TX, dimethirimol + TX, ethirimol + TX, dodemorph + TX, fenpropidine + TX, fenpropimorph + TX, spiroxamine + TX, tridemorph + TX, cyprodinil + TX, mepanipyrim + TX, pyrimethanil + TX, fenpiclonil + TX, fludioxonil + TX, benalaxyl + TX, furalaxyl + TX, metalaxyl + TX, R-metalaxyl + TX, ofurace + TX, oxadixyl + TX, carbendazim + TX, debacarb + TX, fuberidazole + TX, thiabendazole + TX, chlozolinate + TX, dichlozoline + TX, myclozoline + TX, procymidone + TX, vinclozoline + TX, boscalid + TX, carboxin + TX, fenfuram + TX, flutolanil + TX, mepronil + TX, oxycarboxin + TX, penthiopyrad + TX, thifluzamide + TX, dodine + TX, iminoctadine + TX, azoxystrobin + TX, dimoxystrobin + TX, enestroburin + TX, fenaminstrobin + TX, flufenoxystrobin + TX, fluoxastrobin + TX, kresoxim-methyl + TX, metominostrobin + TX, trifloxystrobin + TX, orysastrobin + TX, picoxystrobin + TX, pyraclostrobin + TX, pyrametostrobin + TX, pyraoxystrobin + TX, ferbam + TX, mancozeb + TX, maneb + TX, metiram + TX, propineb + TX, zineb + TX, captafol + TX, captan + TX, fluoroimide + TX, folpet + TX, tolylfluanid + TX, bordeaux mixture + TX, copper oxide + TX, mancopper + TX, oxine-copper + TX, nitrothal-isopropyl + TX, edifenphos + TX, iprobenphos + TX, phosdiphen + TX, tolclofos-methyl + TX, anilazine + TX, benthiavalicarb + TX, blasticidin-S + TX, chloroneb + TX, chlorothalonil + TX, cyflufenamid + TX, cymoxanil + TX, cyclobutrifluram + TX, diclocymet + TX, diclomezine + TX, dicloran + TX, diethofencarb + TX, dimethomorph + TX, flumorph + TX, dithianon + TX, ethaboxam + TX, etridiazole + TX, famoxadone + TX, fenamidone + TX, fenoxanil + TX, ferimzone + TX, fluazinam + TX, fluopicolide + TX, flusulfamide + TX, fluxapyroxad + TX, fenhexamid + TX, fosetyl-aluminium + TX, hymexazol + TX, iprovalicarb + TX, cyazofamid + TX, methasulfocarb + TX, metrafenone + TX, pencycuron + TX, phthalide + TX, polyoxins + TX, propamocarb + TX, pyribencarb + TX, proquinazid + TX, pyroquilon + TX, pyriofenone + TX, quinoxyfen + TX, quintozene + TX, tiadinil + TX, triazoxide + TX, tricyclazole + TX, triforine + TX, validamycin + TX, valifenalate + TX, zoxamide + TX, mandipropamid + TX, flubeneteram + TX, isopyrazam + TX, sedaxane + TX, benzovindiflupyr + TX, pydiflumetofen + TX, 3-difluoromethyl-1-methyl-1H-pyrazole-4-carboxylic acid (3',4',5'-trifluoro-biphenyl-2-yl)-amide + TX, isoflucypram + TX, isotianil + TX, dipymetitrone + TX, 6-ethyl-5,7-dioxo-pyrrolo[4,5][1,4]dithiino[1,2-c]isothiazole-3-carbonitrile + TX, 2-(difluoromethyl)-N-[3-ethyl-1,1-dimethyl-indan-4-yl]pyridine-3-carboxamide + TX, 4-(2,6-difluorophenyl)-6-methyl-5-phenyl-pyridazine-3-carbonitrile + TX, (R)-3-(difluoromethyl)-1-methyl-N-[1,1,3-trimethylindan-4-yl]pyrazole-4-carboxamide + TX, 4-(2-bromo-4-fluoro-phenyl)-N-(2-chloro-6-fluorophenyl)-2,5-dimethyl-pyrazol-3-amine + TX, 4- (2- bromo- 4- fluorophenyl) - N- (2- chloro- 6- fluorophenyl) - 1, 3- dimethyl- 1H- pyrazol- 5- amine + TX, fluindapyr + TX, coumethoxystrobin (jiaxiangjunzhi) + TX, Ivbenmixianan + TX, dichlobentiazox + TX, mandestrobin + TX, 3-(4,4-difluoro-3,4-dihydro-3,3-dimethylisoquinolin-1-yl)quinolone + TX, 2-[2-fluoro-6-[(8-fluoro-2-methyl-3-quinolyl)oxy]phenyl]propan-2-ol + TX, oxathiapiprolin + TX, tert-butyl N-[6-[[[(1-methyltetrazol-5-yl)-phenyl-methylene]amino]oxymethyl]-2-pyridyl]carbamate + TX, pyraziflumid + TX, inpyrfluxam + TX, trolprocarb + TX, mefentrifluconazole + TX, ipfentrifluconazole+ TX, 2-(difluoromethyl)-N-[(3R)-3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine + TX, [2-[3-[2-[1-[2-[3,5-bis(difluoromethyl)-pyrazol-1-yl]acetyl]-4-piperidyl]thiazol-4-yl]-4,5-dihydroisoxazol-5-yl]-3-chloro-phenyl] methanesulfonate + TX, but-3-ynyl N-[6-[[(Z)-[(1-methyltetrazol-5-yl)-phenyl-methylene]amino]oxymethyl]-2-pyridyl]carbamate + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine + TX, pyridachlometyl + TX, 3-(difluoromethyl)-1-methyl-N-[1,1,3-trimethylindan-4-yl]pyrazole-4-carboxamide + TX, 1-[2-[[1-(4-chlorophenyl)pyrazol-3-yl]oxymethyl]-3-methyl-phenyl]-4-methyl-tetrazol-5-one + TX, 1-methyl-4-[3-methyl-2-[[2-methyl-4-(3,4,5-trimethylpyrazol-1-yl)phenoxy]methyl]phenyl]tetrazol-5-one + TX, aminopyrifen + TX, ametoctradin + TX, amisulbrom + TX, penflufen + TX, (Z,2E)-5-[1-(4-chlorophenyl)pyrazol-3-yl]oxy-2-methoxyimino-N,3-dimethyl-pent-3-enamide + TX, florylpicoxamid + TX, fenpicoxamid + TX, tebufloquin + TX, ipflufenoquin + TX, quinofumelin + TX, isofetamid + TX, N-[2-[2,4-dichloro-phenoxy]phenyl]-3-(difluoromethyl)-1-methylpyrazole-4-carboxamide + TX, N-[2-[2-chloro-4-(trifluoromethyl)phenoxy]phenyl]-3-(difluoromethyl)-1-methyl-pyrazole-4-carboxamide + TX, benzothiostrobin + TX, phenamacril + TX, 5-amino-1,3,4-thiadiazole-2-thiol zinc salt (2:1) + TX, fluopyram + TX, flutianil + TX, fluopimomide + TX, pyrapropoyne + TX, picarbutrazox + TX, 2-(difluoromethyl)-N-(3-ethyl-1,1-dimethyl-indan-4-yl)pyridine-3-carboxamide + TX, 2- (difluoromethyl) -N-((3R) - 1,1,3- trimethylindan- 4- yl) pyridine- 3- carboxamide + TX, 4-[[6-[2-(2,4-difluorophenyl)-1,1-difluoro-2-hydroxy-3-(1,2,4-triazol-1-yl)propyl]-3-pyridyl]oxy]benzonitrile + TX, metyltetraprole + TX, 2-(difluoromethyl) -N-((3R)-1,1,3-trimethylindan- 4- yl) pyridine-3-carboxamide + TX, α- (1,1-dimethylethyl) - α- [4'-(trifluoromethoxy) [1,1'-biphenyl]-4-yl] -5-pyrimidinemethanol + TX, fluoxapiprolin + TX, enoxastrobin + TX, 4-[[6-[2-(2,4-difluorophenyl)-1,1-difluoro-2-hydroxy-3-(1,2,4-triazol-1-yl)propyl]-3-pyridylloxy] benzonitrile + TX, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile + TX, trinexapac + TX, coumoxystrobin + TX, zhongshengmycin + TX, thiodiazole copper + TX, zinc thiazole + TX, amectotractin + TX, iprodione + TX, N-octyl-N'-[2-(octylamino)ethyl]ethane-1,2-diamine + TX; N'-[5-bromo-2-methyl-6-[(1S)-1-methyl-2-propoxy-ethoxy]-3-pyridyl]-N-ethyl-N-methyl-formamidine + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine + TX (these compounds may be prepared from the methods described in WO2015/155075); N'-[5-bromo-2-methyl-6-(2-propoxypropoxy)-3-pyridyl]-N-ethyl-N-methyl-formamidine + TX (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine+ TX, N'-[4-(1-cyclopropyl-2,2,2-trifluoro-1-hydroxyethyl)-5-methoxy-2-methyl-phenyl]-N-isopropyl-N-methyl-formamidine + TX (these compounds may be prepared from the methods described in WO2018/228896); N-ethyl-N'-[5-methoxy-2-methyl-4-[2-trifluoromethyl)oxetan-2-yl]phenyl]-N-methyl-formamidine + TX, N-ethyl-N'-[5-methoxy-2-methyl-4-[2-trifluoromethyl)tetrahydrofuran-2-yl]phenyl]-N-methyl-formamidine + TX (these compounds may be prepared from the methods described in WO2019/110427); N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-3,3,3-trifluoro-1-methyl-propyl]-8-fluoro-quinoline-3-carboxamide + TX, N-[(1R)-1-benzyl-1,3-dimethyl-butyl]-7,8-difluoro-quinoline-3-carboxamide + TX, N-[(1S)-1-benzyl-1,3-dimethyl-butyl]-7,8-difluoro-quinoline-3-carboxamide + TX, 8-fluoro-N-[(1R)-1-[(3-fluorophenyl)methyl]-1,3-dimethyl-butyl]quinoline-3-carboxamide + TX, 8-fluoro-N-[(1S)-1-[(3-fluorophenyl)methyl]-1,3-dimethyl-butyl]quinoline-3-carboxamide + TX, N-[(1R)-1-benzyl-1,3-dimethyl-butyl]-8-fluoro-quinoline-3-carboxamide + TX, N-[(1S)-1-benzyl-1,3-dimethyl-butyl]-8-fluoro-quinoline-3-carboxamide + TX, N-((1R)-1-benzyl-3-chloro-1-methyl-but-3-enyl)-8-fluoro-quinoline-3-carboxamide + TX, N-((1S)-1-benzyl-3-chloro-1-methyl-but-3-enyl)-8-fluoro-quinoline-3-carboxamide + TX (these compounds may be prepared from the methods described in WO2017/153380); 1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 1-(6-chloro-7-methyl-pyrazolo[1,5-a]pyridin-3-yl)-4,4-difluoro-3,3-dimethyl-isoquinoline + TX (these compounds may be prepared from the methods described in WO2017/025510); 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 6-chloro-4,4-difluoro-3,3-dimethyl-1-(4-methylbenzimidazol-1-yl)isoquinoline + TX, 4,4-difluoro-1-(5-fluoro-4-methyl-benzimidazol-1-yl)-3,3-dimethyl-isoquinoline + TX, 3-(4,4-difluoro-3,3-dimethyl-1-isoquinolyl)-7,8-dihydro-6H-cyclopenta[e]benzimidazole + TX (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide + TX, N,2-dimethoxy-N-[[4-[5-(trifluoromethyl)-1,2,4-oxadiazol-3-yl]phenyl]methyl]propanamide + TX, N-ethyl-2-methyl-N-[[4-[5-(trifluoromethyl)-1,2,4-oxadiazol-3-yl]phenyl]methyl]propanamide + TX, 1-methoxy-3-methyl-1-[[4-[5-(trifluoromethyl)-1,2,4-oxadiazol-3-yl]phenyl]methyl]urea + TX, 1,3-dimethoxy-1-[[4-[5-(trifluoromethyl)-1,2,4-oxadiazol-3-yl]phenyl]methyl]urea + TX, 3-ethyl-1-methoxy-1-[[4-[5-(trifluoromethyl)-1,2,4-oxadiazol-3-yl]phenyl]-methyl]urea + TX, N-[[4-[5-(trifluoromethyl)-1,2,4-oxadiazol-3-yl]phenyl]methyl]propanamide + TX, 4,4-dimethyl-2-[[4-[5-(trifluoromethyl)-1,2,4-oxadiazol-3-yl]phenyl]methyl]isoxazolidin-3-one + TX, 5,5-dimethyl-2-[[4-[5-(trifluoromethyl)-1,2,4-oxadiazol-3-yl]phenyl]methyl]isoxazolidin-3-one + TX, ethyl 1-[[4-[5-(trifluoromethyl)-1,2,4-oxadiazol-3-yl]phenyl]methyl]pyrazole-4-carboxylate + TX, N,N-dimethyl-1-[[4-[5-(trifluoromethyl)-1,2,4-oxadiazol-3-yl]phenyl]methyl]-1,2,4-triazol-3-amine + TX (The compounds may be prepared from the methods described in WO2017/055473, WO2017/055469, WO2017/093348 and WO2017/118689); 2-[6-(4-chlorophenoxy)-2-(trifluoromethyl)-3-pyridyl]-1-(1,2,4-triazol-1-yl)propan-2-ol + TX (this compound may be prepared from the methods described in WO2017/029179); 2-[6-(4-bromophenoxy)-2-(trifluoromethyl)-3-pyridyl]-1-(1,2,4-triazol-1-yl)propan-2-ol + TX (this compound may be prepared from the methods described in WO2017/029179); 3-[2-(1-chlorocyclopropyl)-3-(2-fluorophenyl)-2-hydroxy-propyl]imidazole-4-carbonitrile + TX (this compound may be prepared from the methods described in WO2016/156290); 3-[2-(1-chlorocyclopropyl)-3-(3-chloro-2-fluoro-phenyl)-2-hydroxypropyl]imidazole-4-carbonitrile + TX (this compound may be prepared from the methods described in WO2016/156290); (4-phenoxyphenyl)methyl 2-amino-6-methyl-pyridine-3-carboxylate + TX (this compound may be prepared from the methods described in WO2014/006945); 2,6-Dimethyl-1H,5H-[1,4]dithiino[2,3-c:5,6-c']dipyrrole-1,3,5,7(2H,6H)-tetrone + TX (this compound may be prepared from the methods described in WO2011/138281); N-methyl-4-[5-(trifluoromethyl)-1,2,4-oxadiazol-3-yl]benzene-carbothioamide + TX; N-methyl-4-[5-(trifluoromethyl)-1,2,4-oxadiazol-3-yl]benzamide + TX; (Z,2E)-5-[1-(2,4-dichlorophenyl)pyrazol-3-yl]oxy-2-methoxyimino-N,3-dimethyl-pent-3-enamide + TX (this compound may be prepared from the methods described in WO2018/153707); N'-(2-chloro-5-methyl-4-phenoxyphenyl)-N-ethyl-N-methyl-formamidine + TX; N'-[2-chloro-4-(2-fluorophenoxy)-5-methyl-phenyl]-N-ethyl-N-methyl-formamidine + TX (this compound may be prepared from the methods described in WO2016/202742); 2-(difluoromethyl)-N-[(3S)-3-ethyl-1,1-dimethyl-indan-4-yl]pyridine-3-carboxamide + TX (this compound may be prepared from the methods described in WO2014/095675); (5-methyl-2-pyridyl)-[4-[5-(trifluoromethyl)-1,2,4-oxadiazol-3-yl]phenyl]methanone + TX, (3-methylisoxazol-5-yl)-[4-[5-(trifluoromethyl)-1,2,4-oxadiazol-3-yl]phenyl]methanone + TX (these compounds may be prepared from the methods described in WO2017/220485); 2-oxo-N-propyl-2-[4-[5-(trifluoromethyl)-1,2,4-oxadiazol-3-yl]phenyl]acetamide + TX (this compound may be prepared from the methods described in WO2018/065414); ethyl 1-[[5-[5-(trifluoromethyl)-1,2,4-oxadiazol-3-yl]-2-thienyl]methyl]pyrazole-4-carboxylate + TX (this compound may be prepared from the methods described in WO2018/158365) ; 2,2-difluoro-N-methyl-2-[4-[5-(trifluoromethyl)-1,2,4-oxadiazol-3-yl]phenyl]acetamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[N-methoxy-C-methyl-carbonimidoyl]-4-[5-(trifluoromethyl)-1,2,4-oxadiazol-3-yl]benzamide + TX (these compounds may be prepared from the methods described in WO2018/202428), chloroinconazide + TX, flumetylsulforim + TX, fluoxytioconazole + TX, flufenoxadiazam +TX, metarylpicoxamid +TX.

The references in brackets behind the active ingredients, e.g. *[3878-19-1]* refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright ^{©} 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula (I) selected from one compound as represented in Table C1 to C-23 or Table T1 (below) is preferably in a mixing ratio of from 100:1 to 1:100, especially from 50:1 to 1:50, more especially in a ratio of from 20:1 to 1:20, even more especially from 10:1 to 1:10, still more preferably from 2:1 to 1:2. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound as represented in Tables C-1 to C-23 or Table T1 (below), and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying a compound as represented in Tables C-1 to C-23 or Table T1 (below) and the active ingredient(s) as described above, is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known *per se,* in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds (I) for the preparation of these compositions are also a subject of the invention.

Another aspect of the invention is related to the use of a compound of formula (I) according to the invention or of a preferred individual compound as defined herein, of a composition comprising at least one compound of formula (I) or at least one preferred individual compound as defined herein, or of a fungicidal or insecticidal mixture comprising at least one compound of formula (I) or at least one preferred individual compound as defined herein, in admixture with other fungicides or insecticides as described above, for controlling or preventing infestation of plants, e.g. useful plants such as crop plants, propagation material thereof, e.g. seeds, harvested crops, e.g. harvested food crops, or non-living materials by insects or by phytopathogenic microorganisms, preferably fungal organisms.

A further aspect of invention is related to a method of controlling or preventing an infestation of plants, e.g. useful plants such as crop plants, propagation material thereof, e.g. seeds, harvested crops, e.g. harvested food crops, or of non-living materials by phytopathogenic or spoilage microorganisms or organisms potentially harmful to man, especially fungal organisms, which comprises the application of a compound of formula (I) according to the invention or of a preferred individual compound as defined herein as active ingredient to the plants, to parts of the plants or to the locus thereof, to the propagation material thereof, or to any part of the non-living materials.

Controlling or preventing means reducing infestation by insects or by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, or insects which comprises the application of a compound of formula (I) according to the invention, or an agrochemical composition which contains at least one compound of formula (I), is foliar application. The frequency of application and the rate of application will depend on the risk of infestation by the corresponding pathogen or insect. However, the compounds of formula (I) according to the invention can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula (I) may also be applied to seeds (coating) by impregn¬ting the seeds or tubers either with a liquid formulation of the fungicide or coating them with a solid formulation.

A formulation, e.g. a composition containing the compound of formula (I) according to the invention and, if desired, a solid or liquid adjuvant or monomers for encapsulating the compound of formula (I), may be prepared in a known manner, typically by intimately mixing and/or grinding the compound with extenders, for example solvents, solid carriers and, optionally, surface active compounds (surfactants).

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient dosages are from 10mg to 1g of active substance per kg of seeds.

When the combinations of the present invention are used for treating seed, rates of 0.001 to 50 g of a compound of formula (I) per kg of seed, preferably from 0.01 to 10g per kg of seed are generally sufficient.

Suitably, a composition comprising a compound of formula (I) according to the present invention is applied either preventative, meaning prior to disease development or curative, meaning after disease development.

The compositions of the invention may be employed in any conventional form, for example in the form of a twin pack, a powder for dry seed treatment (DS), an emulsion for seed treatment (ES), a flowable concentrate for seed treatment (FS), a solution for seed treatment (LS), a water dispersible powder for seed treatment (WS), a capsule suspension for seed treatment (CF), a gel for seed treatment (GF), an emulsion concen¬trate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP) or any technically feasible formulation in combination with agriculturally acceptable adjuvants.

Such compositions may be produced in conventional manner, e.g. by mixing the active ingre¬dients with appropriate formulation inerts (diluents, solvents, fillers and optionally other formulating ingredients such as surfactants, biocides, anti-freeze, stickers, thickeners and compounds that provide adjuvancy effects). Also conventional slow release formulations may be employed where long lasting efficacy is intended. Particularly formulations to be applied in spraying forms, such as water dispersible concentrates (e.g. EC, SC, DC, OD, SE, EW, EO and the like), wettable powders and granules, may contain surfactants such as wetting and dispersing agents and other compounds that provide adjuvancy effects, e.g. the condensation product of formaldehyde with naphthalene sulphonate, an alkylarylsulphonate, a lignin sulphonate, a fatty alkyl sulphate, and ethoxylated alkylphenol and an ethoxylated fatty alcohol.

A seed dressing formulation is applied in a manner known *per se* to the seeds employing the combination of the invention and a diluent in suitable seed dressing formulation form, e.g. as an aqueous suspension or in a dry powder form having good adherence to the seeds. Such seed dressing formulations are known in the art. Seed dressing formulations may contain the single active ingredients or the combination of active ingredients in encapsulated form, e.g. as slow release capsules or microcapsules.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of formula (I) according to the invention optionally together with other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

Whereas it is preferred to formulate commercial products as concentrates, the end user will normally use dilute formulations.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The disclosure in the present application makes available each and every combination of embodiments disclosed herein.

The compounds according to the following Tables C-1 to C-23 may be prepared according to the methods described above. The examples which follow are intended to illustrate the invention and show preferred compounds of formula (I). In any of Tables C-1 to C-23 below, the presence of one or more possible asymmetric carbon atoms in a compound of formula (I) according to the invention means that the compounds may occur in chiral isomeric forms, i.e., enantiomeric or diastereomeric forms.

**Table A**

| This table discloses 12 compounds of formula (A): | | | |
|---|---|---|---|
| 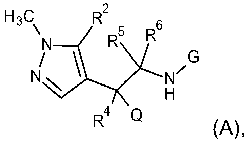 | | | |
| wherein R², R⁴, R⁵, R⁶ and Q are as defined above for the compounds of formula (I) and G is of formula | | | |
| 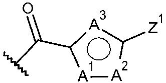 as defined below: | | | |
| Index | G | Index | G |
| G1 | [5-(2,4-difluorophenyl)isoxazole-3-carbonyl] | G7 | [5-(4-fluorophenyl)isoxazole-3-carbonyl] |
| G2 | [5-(2,4-dichlorophenyl)isoxazole-3-carbonyl] | G8 | [5-(4-chlorophenyl)isoxazole-3-carbonyl] |
| G3 | [3-(2,4-difluorophenyl)isoxazole-5-carbonyl] | G9 | [3-(4-fluorophenyl)isoxazole-5-carbonyl] |
| G4 | [3-(2,4-dichlorophenyl)isoxazole-5-carbonyl] | G10 | [3-(4-chlorophenyl)isoxazole-5-carbonyl] |
| G5 | [5-(2,4-difluorophenyl)-1,3,4-thiadiazole-2-carbonyl] | G11 | [5-(4-fluorophenyl)-1,3,4-thiadiazole-2-carbonyl] |
| G6 | [5-(2,4-dichlorophenyl)-1,3,4-thiadiazole-2-carbonyl] | G12 | [5-(4-chlorophenyl)-1,3,4-thiadiazole-2-carbonyl] |

**Table B**

| This table discloses 33 compounds of formula (A): | | | |
|---|---|---|---|
| 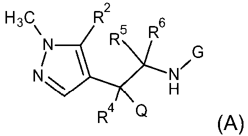 | | | |
| wherein R², R⁴, R⁵, R⁶ and G are as defined above for the compounds of formula (I) and Q is as defined below: | | | |
| Index | Q | Index | Q |
| Q1 | 2-pyridyl | Q18 | [4-(4-chloropyrazol-1-yl)-2-pyridyl] |
| Q2 | (6-chloro-2-pyridyl) | Q19 | [4-(4-chlorophenyl)-2-pyridyl] |
| Q3 | (6-methoxy-2-pyridyl) | Q20 | (6-bromo-2-pyridyl) |
| Q4 | (6-methylsulfonyl-2-pyridyl) | Q21 | (6-fluoro-2-pyridyl) |
| Q5 | [6-(trifluoromethyl)-2-pyridyl] | Q22 | (5-chloro-2-pyridyl) |
| Q6 | [6-(1,1,2,2,2-pentafluoroethyl)-2-pyridyl] | Q23 | (3-chloro-2-pyridyl) |
| Q7 | [6-(2,2,2-trifluoroethoxy)-2-pyridyl] | Q24 | pyridazin-3-yl |
| Q8 | [6-(2,2-difluoroethoxy)-2-pyridyl] | Q25 | (6-chloropyridazin-3-yl) |
| Q9 | [6-(4-chloropyrazol-1-yl)-2-pyridyl] | Q26 | pyrimidin-4-yl |
| Q10 | [6-(4-chlorophenyl)-2-pyridyl] | Q27 | (6-chloropyrimidin-4-yl) |
| Q11 | (4-chloro-2-pyridyl) | Q28 | (2-chloropyrimidin-4-yl) |
| Q12 | (4-methoxy-2-pyridyl) | Q29 | (5-chloropyrazin-2-yl) |
| Q13 | (4-methylsulfonyl-2-pyridyl) | Q30 | (6-chloropyrazin-2-yl) |
| Q14 | [4-(trifluoromethyl)-2-pyridyl] | Q31 | pyrazin-2-yl |
| Q15 | [4-(1,1,2,2,2-pentafluoroethyl)-2-pyridyl] | Q32 | (4-chloropyrimidin-2-yl) |
| Q16 | [4-(2,2,2-trifluoroethoxy)-2-pyridyl] | Q33 | pyrimidin-2-yl |
| Q17 | [4-(2,2-difluoroethoxy)-2-pyridyl] | | |

Tables C-1 to C-23 disclose specific compounds of formula (A): 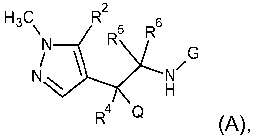 wherein R², R⁴, R⁵ and R⁶ are as defined in any of Tables C-1 to C-23, and G and Q substituents are as defined in Tables A and B, respectively.

**Table C-1:** This table provides 12 compounds C-1.01 to C-1.012 of formula (A) wherein R², R⁴, R⁵ and R⁶ are H, Q is Q1, and G is as defined in Table A. For example, compound C-1.01 has the following structure:

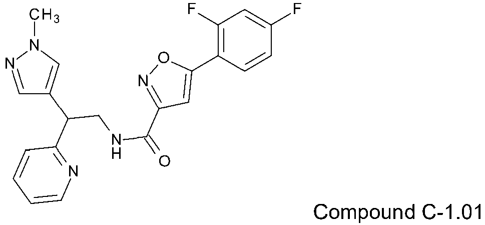

**Table C-2:** This table provides 12 compounds C-2.01 to C-2.012 of formula (A) wherein R², R⁵ and R⁶ are H, R⁴ is CH₃, Q is Q1 and G is as defined in Table A. For example, compound C-2.02 has the following structure:

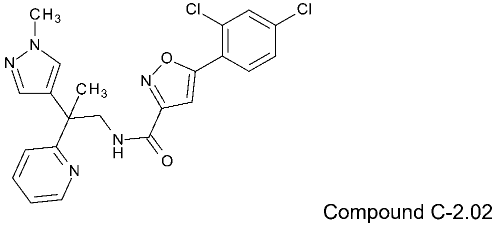

**Table C-3:** This table provides 12 compounds C-3.01 to C-3.012 of formula (A) wherein R², R⁴, R⁵ and R⁶ are H, Q is Q2, and G is as defined in Table A.

**Table C-4:** This table provides 12 compounds C-4.01 to C-4.012 of formula (A) wherein R², R⁵ and R⁶ are H, R⁴ is CH₃, Q is Q2, and G is as defined in Table A.

**Table C-5:** This table provides 12 compounds C-5.01 to C-5.012 of formula (A) wherein R⁵ and R⁶ are H, R² and R⁴ are CH₃, Q is Q2, and G is as defined in Table A.

**Table C-6:** This table provides 12 compounds C-6.01 to C-6.012 of formula (A) wherein R⁵ and R⁶ are H, R² is fluorine, R⁴ is CH₃, Q is Q2, and G is as defined in Table A.

**Table C-7:** This table provides 12 compounds C-7.01 to C-7.012 of formula (A) wherein R², R⁵ and R⁶ are H, R⁴ is CH₂CH₃, Q is Q2, and G is as defined in Table A. For example, compound C-7.05 has the following structure:

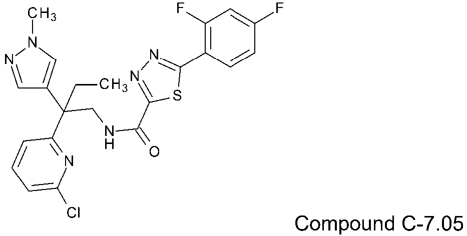

**Table C-8:** This table provides 12 compounds C-8.01 to C-8.012 of formula (A) wherein R², R⁵ and R⁶ are H, R⁴ is CH(CH₃)₂, Q is Q2, and G is as defined in Table A.

**Table C-9:** This table provides 12 compounds C-9.01 to C-9.012 of formula (A) wherein R², R⁵ and R⁶ are H, R⁴ is CN, Q is Q2, and G is as defined in Table A.

**Table C-10:** This table provides 12 compounds C-10.01 to C-10.012 of formula (A) wherein R², R⁵ and R⁶ are H, R⁴ is CH₂OCH₃, Q is Q2, and G is as defined in Table A.

**Table C-11:** This table provides 6 compounds C-11.01 to C-11.06 of formula (A) wherein R², R⁵ and R⁶ are H, R⁴ is CH₃, Q is Q24, and G is as defined in Table A. For example, compound C-11.03 has the following structure:

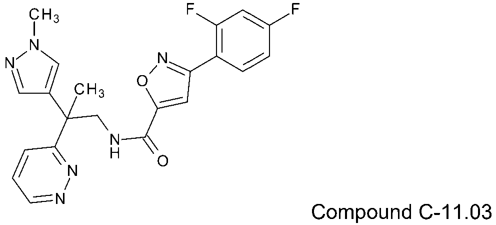

**Table C-12:** This table provides 6 compounds C-12.01 to C-12.06 of formula (A) wherein R², R⁵ and R⁶ are H, R⁴ is CH₃, Q is Q25, and G is as defined in Table A.

**Table C-13:** This table provides 6 compounds C-13.01 to C-13.06 of formula (A) wherein R², R⁵ and R⁶ are H, R⁴ is CH₃, Q is Q26, and G is as defined in Table A.

**Table C-14:** This table provides 6 compounds C-14.01 to C-14.06 of formula (A) wherein R², R⁵ and R⁶ are H, R⁴ is CH₃, Q is Q27, and G is as defined in Table A.

**Table C-15:** This table provides 6 compounds C-15.01 to C-15.06 of formula (A) wherein R², R⁵ and R⁶ are H, R⁴ is CH₃, Q is Q28, and G is as defined in Table A.

**Table C-16:** This table provides 6 compounds C-16.01 to C-16.06 of formula (A) wherein R², R⁵ and R⁶ are H, R⁴ is CH₃, Q is Q29, and G is as defined in Table A.

**Table C-17:** This table provides 6 compounds C-17.01 to C-17.06 of formula (A) wherein R², R⁵ and R⁶ are H, R⁴ is CH₃, Q is Q30, and G is as defined in Table A.

**Table C-18:** This table provides 6 compounds C-18.01 to C-18.06 of formula (A) wherein R², R⁵ and R⁶ are H, R⁴ is CH₃, Q is Q31, and G is as defined in Table A.

**Table C-19:** This table provides 6 compounds C-19.01 to C-19.06 of formula (A) wherein R², R⁵ and R⁶ are H, R⁴ is CH₃, Q is Q32, and G is as defined in Table A.

**Table C-20:** This table provides 6 compounds C-20.01 to C-20.06 of formula (A) wherein R², R⁵ and R⁶ are H, R⁴ is CH₃, Q is Q33, and G is as defined in Table A. For example, compound C-20.06 has the following structure:

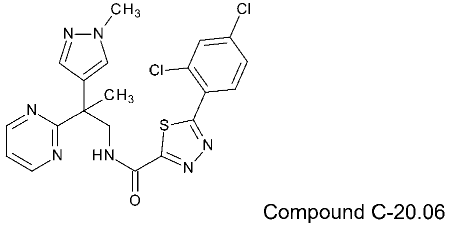

**Table C-21:** This table provides 31 compounds C-21.03 to C-21.033 of formula (A) wherein R², R⁵ and R⁶ are H, R⁴ is CH₃, G is G1, and Q is as defined in Table B.

For example, compound C-21.013 has the following structure:

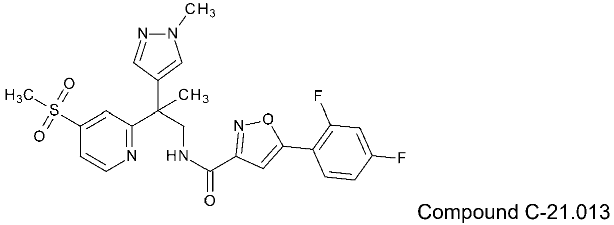

**Table C-22:** This table provides 6 compounds C-22.01 to C-22.06 of formula (A) wherein R² and R⁴ are H, R⁵and R⁶ are methyl, Q is Q2, and G is as defined in Table A. For example, compound C-22.04 has the following structure:

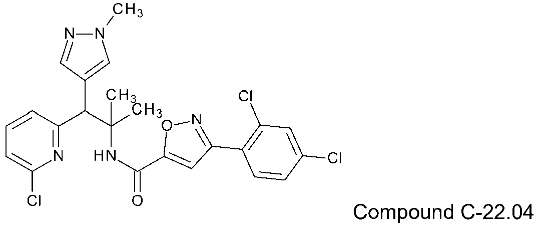

**Table C-23:** This table provides 6 compounds C-23.01 to C-23.06 of formula (A) wherein R², R⁴ and R⁵ are H, R⁶ is methyl, Q is Q2, and G is as defined in Table A.

### EXAMPLES

The Examples which follow serve to illustrate the invention and are not meant in any way to limit the invention.

The compounds of the invention can be distinguished from known compounds by virtue of greater efficacy at low application rates, which can be verified by a person skilled in the art using the experimental procedures outlined in the Examples, using lower application rates if necessary, for example 60 ppm, 20 ppm or 2 ppm.

Compounds of formula (I) may possess any number of benefits including, *inter alia,* advantageous levels of biological activity for protecting plants against diseases that are caused by fungi or superior properties for use as agrochemical active ingredients (for example, greater biological activity, an advantageous spectrum of activity, an increased safety profile (including improved crop tolerance), improved physicochemical properties, or increased biodegradability).

Throughout this description, temperatures are given in degrees Celsius and "m.p." means melting point. LC/MS means Liquid Chromatography Mass Spectroscopy and the description of the apparatus and the methods is as follows.

¹H NMR and ¹⁹F NMR measurements were recorded on a Bruker 400MHz spectrometer, chemical shifts are given in ppm relevant to a TMS (¹H) and CFCI3 (¹⁹F) standard. Spectra measured in deuterated solvents as indicated. Either one of the LCMS methods below was used to characterize the compounds. The characteristic LCMS values obtained for each compound were the retention time ("Rt", recorded in minutes) and the measured molecular ion (M+H)⁺ or (M-H)⁻.

**Method A:** Spectra were recorded on a Mass Spectrometer from Waters Corporation (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 I/h, Desolvation Gas Flow: 650-1000 I/h, Mass range: 50 to 900 Da and an Acquity UPLC from Waters Corporation: Binary pump, heated column compartment, diode-array detector and ELSD. Column: Waters UPLC HSS T3, 1.8 µm, 30 x 2.1 mm, Temp: 60 °C, DAD Wavelength range (nm): 210 to 400, Runtime: 1.5 min; Solvents: A = water + 5% MeOH + 0.05 % HCOOH, B= Acetonitrile + 0.05 % HCOOH; Flow (ml/min) 0.85, Gradient: 10% B isocratic for 0.2 min, then 10-100% B in 1.0 min, 100% B isocratic for 0.2min, 100-10% B in 0.05min, 10% B isocratic for 0.05 min.

**Method B:** Spectra were recorded on a Mass Spectrometer from Waters Corporation (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 I/h, Desolvation Gas Flow: 650-1000 I/h, Mass range: 50 to 900 Da and an Acquity UPLC from Waters Corporation: Binary pump, heated column compartment, diode-array detector and ELSD. Column: Waters UPLC HSS T3, 1.8 µm, 30 x 2.1 mm, Temp: 60 °C, DAD Wavelength range (nm): 210 to 400, Runtime: 3.0 min; Solvents: A = water + 5% MeOH + 0.05 % HCOOH, B= Acetonitrile + 0.05 % HCOOH; Flow (ml/min) 0.85, Gradient: 10% B isocratic for 0.2 min, then 10-100% B in 2.5 min, 100% B isocratic for 0.3min

**Method C:** Spectra were recorded on a Mass Spectrometer from Waters Corporation (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 I/h, Desolvation Gas Flow: 650-1000 I/h, Mass range: 50 to 900 Da and an Acquity UPLC from Waters Corporation: Binary pump, heated column compartment, diode-array detector and ELSD. Column: Waters UPLC HSS T3, 1.8 µm, 30 x 2.1 mm, Temp: 60 °C, DAD Wavelength range (nm): 210 to 400, Runtime: 3.0 min; Solvents: A = water + 5% MeOH + 0.05 % HCOOH, B= Acetonitrile + 0.05 % HCOOH; Flow: 0-2.5 min: 0.85, then 1 ml/min, Gradient: 0% B isocratic for 0.2 min, then 0-10% B in 2.3 min, 10-100%B in 0.05min Flow 1 ml/min, 100% B isocratic for 0.2 min, 100-0% B in 0.05min, 0% B isocratic for 0.2 min.

**Method D:** Spectra were recorded on a Mass Spectrometer from Waters Corporation (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 I/h, Desolvation Gas Flow: 650-1000 I/h, Mass range: 50 to 900 Da and an Acquity UPLC from Waters Corporation: Binary pump, heated column compartment, diode-array detector and ELSD. Column: Waters UPLC HSS T3, 1.8 µm, 30 x 2.1 mm, Temp: 60 °C, DAD Wavelength range (nm): 210 to 400, Runtime: 1.5 min; Solvents: A = water + 5% MeOH + 0.05 % HCOOH, B= Acetonitrile + 0.05 % HCOOH; Flow (ml/min) 0.85, Gradient: 10-40% B in 0.1min, 40% B isocratic for 0.1 min, then 40-100% B in 1.0 min, 100% B isocratic for 0.2min, 100-10% B in 0.05min, 10% B isocratic for 0.1 min.

**Method Agilent:** Spectra were recorded on a Mass Spectrometer from Agilent Technologies (6410 Triple Quadruple MS, Agilent 1200 Series HPLC) equipped with an electrospray source (ESI, Positive and Negative Polarity Switch, scan type: MS2), Capillary: 7.00kV, Fragmentor: 120.00 V, Gas Temperature 350°C, Gas Flow (L/min): 11, Nebulizer Gas (psi): 40, Mass range: 110 to 650 Da, VWD Wavelength: 254 nm. Optimized Chromatographic Parameter are as follows: Column: KINETEX EVO C18, Column length: 50 mm, Internal diameter of column: 4.6 mm, Particle Size: 2.6 µ, Column oven temperature: 40°C. Gradient conditions: Solvent A: Water with 0.1% formic acid : Acetonitrile = 95 : 5 v/v, Solvent B: Acetonitrile with 0.1% formic acid

| Time (minutes) | A (%) | B (%) | Flow rate (ml/min) |
|---|---|---|---|
| 0 | 90 | 10 | 1.8 |
| 0.9 | 0 | 100 | 1.8 |
| 1.8 | 0 | 100 | 1.8 |
| 2.2 | 90 | 10 | 1.8 |
| 2.5 | 90 | 10 | 1.8 |

**Method SQD:** Spectra were recorded on a Acquity SQD Mass Spectrometer from Waters (HPLC: UPLC 'H' class) equipped with an electrospray source (ESI, Positive and Negative Polarity Switch, scan type: full scan), Capillary (kV): 3.00, Cone Voltage (V): 41.00, Source Temperature (°C): 150, Desolvation Gas Flow (L/Hr): 1000, Desolvation Temperature (°C): 500, Gas Flow @ Cone (L/Hr): 50, Mass range: 110 to 800 Da, PDA Wavelength range: 210 to 400 nm. Column: Acquity UPLC HSS T3 C18, Column length: 30 mm, Internal diameter of column: 2.1 mm, Particle Size: 1.8 µ, Column oven temperature: 40°C. Optimized Chromatographic Parameter are as follows: Gradient conditions: Solvent A: Water with 0.1% formic acid : Acetonitrile = 95 : 5 v/v, Solvent B: Acetonitrile with 0.05% formic acid

| Time (minutes) | A (%) | B (%) | Flow rate (ml/min) |
|---|---|---|---|
| 0.0 | 90 | 10 | 0.6 |
| 0.2 | 90 | 10 | 0.6 |
| 0.3 | 50 | 50 | 0.6 |
| 0.6 | 0 | 100 | 0.6 |
| 1.3 | 0 | 100 | 0.6 |
| 1.4 | 90 | 10 | 0.6 |
| 1.6 | 90 | 10 | 0.6 |

**Method QDA:** Spectra were recorded on a Acquity QDA Mass Spectrometer from Waters (HPLC: UPLC 'H' class) equipped with an electrospray source (ESI, Positive and Negative Polarity Switch, scan type: full scan), Capillary (kV): 0.8, Cone Voltage (V): 25.00, Source Temperature (°C): 120, Desolvation Gas Flow (L/Hr): 1000, Desolvation Temperature (°C): 600, Gas Flow @ Cone (L/Hr): 50, Mass range: 110 to 850 Da, PDA Wavelength range: 230 to 400 nm. Optimized Chromatographic parameter are as follows: Column: Acquity UPLC HSS T3 C18, Column length: 30 mm, Internal diameter of column: 2.1 mm, Particle Size: 1.8 µ, Column oven temperature: 40°C, Gradient conditions: Solvent A: Water with 0.1% formic acid: Acetonitrile = 95: 5 v/v, Solvent B: Acetonitrile with 0.05% formic acid

| Time (minutes) | A (%) | B (%) | Flow rate (ml/min) |
|---|---|---|---|
| 0 | 90 | 10 | 0.6 |
| 0.2 | 90 | 10 | 0.6 |
| 0.3 | 50 | 50 | 0.6 |
| 0.6 | 0 | 100 | 0.6 |
| 1.3 | 0 | 100 | 0.6 |
| 1.4 | 90 | 10 | 0.6 |
| 1.6 | 90 | 10 | 0.6 |

**Method HSS:** Spectra were recorded on a ACQUITY Mass Spectrometer from Waters Corporations (SQD or SQDII Single quadrupole mass spectrometer) equipped with an electrospray source (Polarity: positive or negative ions, Capillary: 3.0 kV, Cone: 30V, Extractor: 3.00 V, Source Temperature: 150°C, Desolvation Temperature: 400°C, Cone Gas Flow: 60 L/hr, Desolvation Gas Flow: 700 L/hr, Mass range: 140 to 800 Da) and an ACQUITY UPLC from Waters Corporations with solvent degasser, binary pump, heated column compartment and diode-array detector. Column: Waters UPLC HSS T3, 1.8 µm, 30 x 2.1 mm, Temp: 60 °C, DAD Wavelength range (nm): 210 to 400, Solvent Gradient: A = Water/Methanol 9:1 + 0.1% formic acid, B= Acetonitrile + 0.1% formic acid, gradient: 0-100% B in 2.5 min; Flow (ml/min) 0.75.

### PREPARATION EXAMPLES

The compounds of formula (I) according to the invention may be prepared using the synthetic techniques described both above and below.

### Example P1: Preparation of N-[2-(6-chloro-2-pyridyl)-2-(1-methylpyrazol-4-yl)propyl]-5-(2,4-difluorophenyl)isoxazole-3-carboxamide (Compound P-10, Table T1)

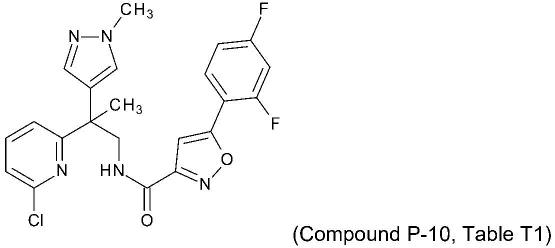

### Step 1: Preparation of 2-(1-methylpyrazol-4-yl)propane nitrile

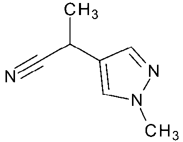

A sample of 2-(1-methyl-1h-pyrazol-4-yl)acetonitrile (10.00 g, 78.42 mmol) was dissolved in tetrahydrofuran (314 mL) under argon and the pale yellow solution was cooled to -78°C. To this solution was added dropwise n-butyl lithium (31 mL, 78.42 mmol) and the resulting pale brown suspension was stirred at this temperature for 25 minutes. After this time, iodomethane (11.24 g, 4.93 mL, 78.42 mmol) was added dropwise. The resulting brown solution was stirred at -78°C for 5 minutes, allowed to warm to room temperature and stirred for 30 minutes under argon. The reaction mixture was poured into water, and extracted twice with ethyl acetate. The combined organic layers were washed once with brine, dried over anhydrous sodium sulfate, filtered and concentrated under vacuum to give 2-(1-methylpyrazol-4-yl)propanenitrile as a light brown liquid. LC/MS (Method A); 136 [M+H]⁺; retention time: 0.43 min, 0.44 min, 0.55 min. ¹H NMR (400 MHz, CDCl₃) δ ppm 1.64 (d, J=6.90 Hz, 3 H) 3.86 - 3.93 (m, 4 H) 7.40 (s, 1 H) 7.46 (s, 1 H).

### Step 2: Preparation of 2-(6-chloro-2-pyridyl)-2-(1-methylpyrazol-4-yl)propanenitrile

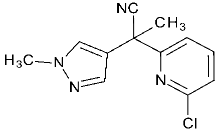

A sample of 2-(1-methylpyrazol-4-yl)propanenitrile (2.00 g, 14.80 mmol) was dissolved in tetrahydrofuran (59.18 mL) under argon to give a pale yellow solution. The solution was cooled to -78°C and then treated dropwise with n-butyl lithium (5.90 mL, 14.80 mmol). The resulting pale brown suspension was stirred at this temperature for 10 minutes before adding 2,6-dichloropyridine (2.23 g, 14.80 mmol) portion wise. The resulting brown suspension was stirred at -78°C for 5 minutes, let to reach room temperature and stirred for 30 minutes under argon to give a light brown solution. The reaction mixture was poured into water and extracted twice with ethyl acetate. The combined organic layers were washed once with brine, dried over anhydrous sodium sulfate, filtered and concentrated under vacuum at 60°C to give 3.68 g of a dark brown liquid. The crude was purified by flash chromatography with an eluent of ethyl acetate in cyclohexane to yield 2-(6-chloro-2-pyridyl)-2-(1-methylpyrazol-4-yl)propanenitrile (2.71 g, 74.2 % yield) as a pale yellow oil. LC/MS (method A); 247 [M+H]⁺; retention time: 0.82 min. ¹H NMR (400 MHz, CDCl₃) δ ppm 2.12 (s, 3 H) 3.90 (s, 3 H) 7.27 - 7.32 (m, 1 H) 7.45 (s, 1 H) 7.46 - 7.54 (m, 2 H) 7.62 - 7.74 (m, 1 H).

### Step 3: Preparation of 2-(6-chloro-2-pyridyl)-2-(1-methylpyrazol-4-yl)propan-1-amine

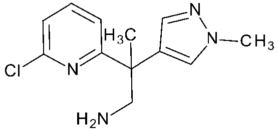

A solution of 2-(6-chloro-2-pyridyl)-2-(1-methylpyrazol-4-yl)propanenitrile (2.71 g, 11.0 mmol) in tetrahydrofuran (33.0 mL) was treated dropwise with BMS (2.50 g, 3.13 mL, 33.0 mmol) at room temperature under argon and the resulting pale-yellow solution was stirred for 4 hours at 65°C. The reaction mixture was cooled to 0°C before adding dropwise concentrated hydrochloric acid (7.36 mL, 44.2 mmol). The reaction mixture was then was stirred for 1 hour at 50°C. The reaction mixture was cooled and treated with water. The reaction mixture was neutralised to pH 12 with NaOH 6N and extracted three times with ethyl acetate. The combined organic layers were washed once with brine, dried over anhydrous sodium sulfate and concentrated under vacuum to give 2-(6-chloro-2-pyridyl)-2-(1-methylpyrazol-4-yl)propan-1-amine as a pale-yellow oil, that could be used in the next step without further purification. ¹H NMR (400 MHz, DMSO d6) δ ppm 1.57 (s, 3 H) 2.98 (d, *J*=12.72 Hz, 1 H) 3.16 (d, *J*=12.72 Hz, 1 H) 3.77 (s, 3 H) 7.20 - 7.35 (m, 3 H) 7.50 (s, 1 H) 7.75 (t, *J*=7.81 Hz, 1 H).

### Step 4: Preparation of N-[2-(6-chloro-2-pyridyl)-2-(1-methylpyrazol-4-yl)propyl]-5-(2,4-difluorophenyl)isoxazole-3-carboxamide (Compound P-10, Table T1)

A sample of (1-cyano-2-ethoxy-2-oxoethylidenaminooxy)dimethylamino-morpholino-carbenium-hexafluorophosphate (COMU) (0.10 g, 0.23 mmol) was added to a white suspension of 2-(6-chloro-2-pyridyl)-2-(1-methylpyrazol-4-yl)propan-1-amine (0.05 g, 0.20 mmol), 5-(2,4-difluorophenyl)isoxazole-3-carboxylic acid (0.05 g, 0.22 mmol, prepared as described in WO2018/019929) and N,N-diisopropylethylamine (0.05 g, 0.07 mL, 0.40 mmol) in 2-MeTHF (2.0 mL) under argon. After reaction completion, the reaction mixture was diluted with ethyl acetate, and the separated ethyl acetate phase washed once with 10 ml of 0.05 M aqueous HCI, once with saturated aqueous sodium bicarbonate, and then dried over anhydrous Na₂SO₄, filtered and concentrated under vacuum to give a pale brown oily powder. The crude mixture was first purified by flash chromatography eluting with ethyl acetate in cyclohexane and then by flash chromatography eluting with acetonitrile in water to give N-[2-(6-chloro-2-pyridyl)-2-(1-methylpyrazol-4-yl)propyl]-5-(2,4-difluorophenyl)isoxazole-3-carboxamide as a white powder. LC/MS (Method A); 458 [M+H]⁺; retention time: 1.08 min. ¹H NMR (400 MHz, CDCl₃) δ ppm 1.74 (s, 3 H) 3.89 (s, 3 H) 4.01 - 4.15 (m, 2 H) 6.96 - 7.16 (m, 4 H) 7.23 - 7.31 (m, 3 H) 7.60 (t, *J*=7.81 Hz, 1 H) 7.94 (qd, *J*=8.90, 6.36 Hz, 2 H).

### Example P2: Preparation of N-[2-(6-chloro-2-pyridyl)-2-(1-methylpyrazol-4-yl)propyl]-5-(2,4-difluorophenyl)-1,3,4-thiadiazole-2-carboxamide (Compound P-9, Table T1)

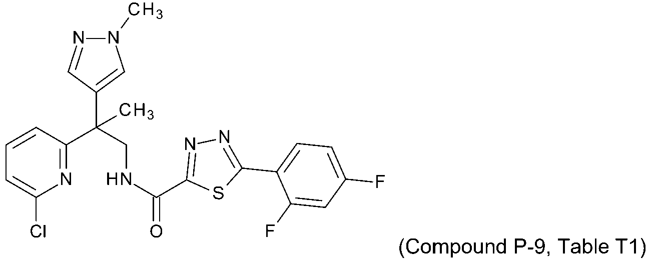

Step 1-3 are the same as for Example 1.

### Step 4: Preparation of N-[2-(6-chloro-2-pyridyl)-2-(1-methylpyrazol-4-yl)propyl]-5-(2,4-difluorophenyl)-1,3,4-thiadiazole-2-carboxamide (Compound P-9, Table T1)

Trimethylaluminum (0.20 mL, 0.40 mmol) was added dropwise to a pale beige suspension of ethyl 5-(2,4-difluorophenyl)-1,3,4-thiadiazole-2-carboxylate (0.070 g, 0.26 mmol, prepared as described in WO2019/014308) in 0.4 ml of toluene under argon atmosphere (gas evolution and exothermic addition). The resulting pale brown solution was stirred at room temperature for 15 minutes. A pale brown solution of 2-(6-chloro-2-pyridyl)-2-(1-methylpyrazol-4-yl)propan-1-amine (0.05 g, 0.20 mmol) in 0.4 ml of toluene was then added dropwise at room temperature (gas evolution). The resulting yellow solution was stirred for 2 hours at 90°C under argon. The reaction mixture was cooled to 0°C and quenched carefully by addition of HCl 1 M (0.25 mL). The mixture was diluted with saturated NaHCO₃ and extracted twice with ethyl acetate. The combined organic layers were washed once with brine, dried over anhydrous sodium sulfate, filtered and concentrated under vacuum to give the crude product which was purified by flash chromatography eluting with acetonitrile in water to give the title compound as a white powder. LC/MS (method A); 475 [M+H]⁺; retention time: 1.08 min. ¹H NMR (400 MHz, CDCl₃) δ ppm 1.75 (s, 3 H) 3.89 (s, 3 H) 4.05 - 4.20 (m, 2 H) 7.00 - 7.18 (m, 3 H) 7.24 - 7.30 (m, 3 H) 7.60 (t, *J*=7.81 Hz, 1 H) 8.31 (br t, *J*=6.18 Hz, 1 H) 8.45 (td, *J*=8.54, 6.54 Hz, 1 H).

### Example P3: Preparation of 5-(2,4-difluorophenyl)-N-[2-(1-methylpyrazol-4-yl)-2-pyrazin-2-yl-ethyllisoxazole-3-carboxamide (Compound P-8, Table T1)

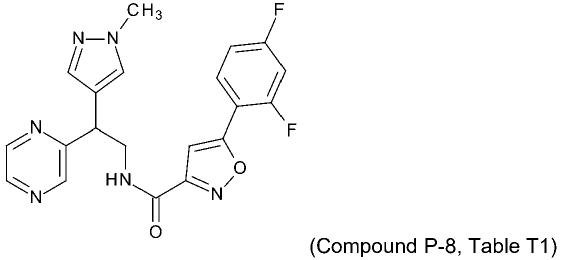

### Step 1: Preparation of 2-nitro-1-pyrazin-2-yl-ethanol

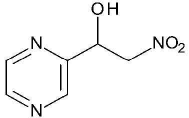

To a round bottom flask was added 2-pyrazinecarboxaldehyde (770 mg, 7.12 mmol) in 10 mL of nitro methane, followed by the addition of triethyl amine (721 mg, 7.12 mmol). The reaction mixture was stirred at room temperature for 1 hour. The reaction mixture was then concentrated under vacuum and the residue was loaded onto a flash chromatography column. The column was eluted with 0-5% methanol in CH₂Cl₂. The product fractions were collected and concentrated to afford 2-nitro-1-pyrazin-2-yl-ethanol. LC/MS (method A); 170 [M+H]⁺; retention time: 0.26 min. ¹H NMR (400 MHz, CDCl₃) δ ppm 3.81 (br s, 1 H) 4.69 - 4.82 (m, 1 H) 4.96 (dd, *J*=13.62, 3.45 Hz, 1 H) 5.61 (dd, *J*=8.36, 3.27 Hz, 1 H) 8.55 - 8.62 (m, 2 H) 8.88 (s, 1 H).

### Step 2: Preparation of 2-[(E)-2-nitrovinyl]pyrazine

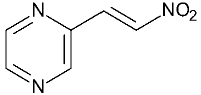

A solution of 2-nitro-1-pyrazin-2-yl-ethanol (0.80 g, 4.69 mmol) in anhydrous CH₂Cl₂ (10mL) was cooled to -20°C. Trifluoroacetic anhydride (0.38 mL, 5.85 mmol) was added keeping the temperature below -10°C. The solution was stirred for two minutes after the addition of the trifluoroacetic anhydride. Triethyl amine (TEA, 1.49 mL, 10.64 mmol) was then added dropwise with the solution being kept below -10° C. The solution became homogeneous and orange/brown upon addition of the base. The solution was stirred for 15 minutes after addition of TEA. The solution was diluted with more CH₂Cl₂ and washed with a saturated ammonium chloride solution. The aqueous layer was extracted with CH₂Cl₂ (2x50 mL) and the combined organic layers were washed with brine, dried over Na₂SO₄, filtered, and concentrated under vacuum. The crude brown solid residue was suspended in 300 mL of hot ethyl acetate and filtered. The filtrate was concentrated under vacuum. The residue was purified *via* flash chromatography and eluted with 50% ethyl acetate in cyclohexane to yield 2-[(E)-2-nitrovinyl]pyrazine as a bright yellow solid. LC/MS (method A);152 [M+H]⁺; retention time: 0.52 min. ¹H NMR (400 MHz, CDCl₃) δ ppm 7.98 (d, *J*=1.00 Hz, 1 H) 8.07 (d, *J*=1.00 Hz, 1 H) 8.68 (s, 2 H) 8.75 (s, 1 H).

### Step 3: Preparation of 2-[1-(1-methylpyrazol-4-yl)-2-nitro-ethyl]pyrazine

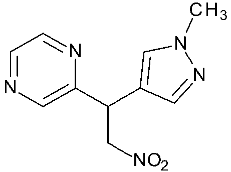

A round bottom flask equipped with a magnetic stirred bar was charged with 4-iodo-1-methyl-1h-pyrazole (0.19 g, 0.89 mmol) and tetrahydrofuran (0.89 mL) under argon to give a colourless solution. Isopropylmagnesium chloride lithium chloride complex (0.68 mL, 0.89 mmol) was added dropwise at 0°C. The resulting white cloudy solution was stirred at 0°C for 20 minutes under argon. To this solution was added 2-[(E)-2-nitrovinyl]pyrazine (0.13 g, 0.89 mmol) and the resulting pale-yellow solution was stirred at 0°C for 10 minutes, and then allowed to warm to room temperature and stirred for 1 hour under argon. The reaction mixture was poured into 60 ml of water and extracted twice with 30 ml of ethyl acetate. The combined organic layers were washed once with brine, dried over anhydrous sodium sulfate, filtered, and concentrated under vacuum. The crude product was purified by flash chromatography (silica gel, gradient: ethyl acetate in cyclohexane) to afford 2-[1-(1-methylpyrazol-4-yl)-2-nitro-ethyl]pyrazine. LC/MS (method A); 234 [M+H]⁺; retention time: 0.56 min. ¹H NMR (400 MHz, CDCl₃) δ ppm 3.88 (s, 3 H) 4.84 (dd, *J*=13.81, 6.54 Hz, 1 H) 5.00 (dd, *J*=8.17, 6.72 Hz, 1 H) 5.27 (dd, *J*=13.44, 8.36 Hz, 1 H) 7.30 (s, 1 H) 7.42 (s, 1 H) 8.49 - 8.60 (m, 3 H).

### Step 4: Preparation of 2-(1-methylpyrazol-4-yl)-2-pyrazin-2-yl-ethanamine

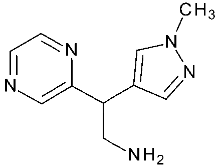

A mixture of 2-[1-(1-methylpyrazol-4-yl)-2-nitro-ethyl]pyrazine (0.11 g, 0.47 mmol), ammonium chloride (0.51 g, 9.43 mmol) and zinc (0.62 g, 9.43 mmol) in tetrahydrofuran (0.47 mL) and methanol (0.47 mL) was stirred at room temperature for 4.5 hours. The mixture was diluted with ethyl acetate (20 mL) and filtered through celite. The filtrate was concentrated under vacuum and used as crude in the next step. LC/MS (method A); 204 [M+H]⁺; retention time: 0.16 min.

### Step 5: Preparation of 5-(2,4-difluorophenyl)-N-[2-(1-methylpyrazol-4-yl)-2-pyrazin-2-yl-ethyl]isoxazole-3-carboxamide (Compound P-8, Table T1)

A round bottom flask, equipped with a magnetic stirrer bar, was charged with 2-[(E)-2-nitrovinyl]pyrazine (0.10 g, 0.49 mmol), ethyl acetate (4.92 mL), N,N-diisopropylethylamine (0.25 mL, 1.48 mmol) and 5-(2,4-difluorophenyl)isoxazole-3-carboxylic acid (0.12 g, 0.54 mmol). Propanephosphonic acid anhydride (0.87 mL, 1.48 mmol) was then added dropwise at room temperature and the mixture was stirred at room temperature for 1 hour. The reaction mixture was quenched with a saturated aqueous solution of NaHCO₃ and extracted with ethyl acetate (3x20mL). The combined organic layers were washed with brine, dried over sodium sulfate and concentrated under vacuum. The crude product was purified by flash chromatography using cyclohexane and ethyl acetate as eluent (gradient 1 to 100% ethyl acetate in cyclohexane) to yield the title compound as a yellowish solid. LC/MS (method A); 211 [M+H]⁺; retention time: 0.85 min. ¹H NMR (400 MHz, CDCl₃) δ ppm 3.89 (s, 3 H) 4.09 - 4.19 (m, 2 H) 4.51 (dd, *J*=7.81, 6.36 Hz, 1 H) 6.95 - 7.11 (m, 3 H) 7.35 (s, 1 H) 7.38 - 7.48 (m, 2 H) 7.93 (td, *J*=8.54, 6.18 Hz, 1 H) 8.51 (dd, *J*=8.90, 2.00 Hz, 2 H) 8.61 (dd, *J*=2.54, 1.45 Hz, 1 H).

### Example P4: Preparation of N-[2-(6-chloro-2-pyridyl)-2-(1-methylpyrazol-4-yl)propyl]-3-(2,4-difluorophenyl)isoxazole-5-carboxamide (Compound P-4, Table T1)

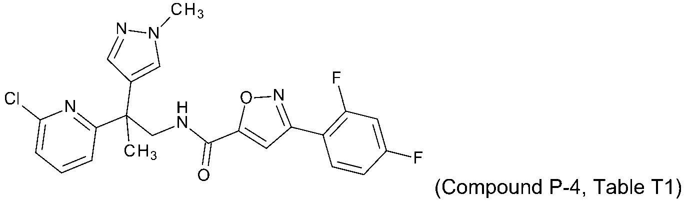

Steps 1-3 are the same as for Example 1.

### Step 4: Preparation of N-[2-(6-chloro-2-pyridyl)-2-(1-methylpyrazol-4-yl)propyl]-3-(2,4-difluorophenyl)isoxazole-5-carboxamide (Compound P-4, Table T1)

COMU (0.14 g, 0.32 mmol) was added to a colourless solution of 2-(6-chloro-2-pyridyl)-2-(1-methylpyrazol-4-yl)propan-1-amine (0.070 g, 0.28 mmol), 3-(2,4-difluorophenyl)-1,2-oxazole-5-carboxylic acid (0.073 g, 0.31 mmol) and N,N-diisopropylethylamine (0.073 g, 0.098 mL, 2.0, 0.56 mmol) in 2-MeTHF (2.8 mL) under argon. The resulting yellow suspension was stirred for 30 minutes at room temperature to give a light-yellow solution. The reaction mixture was diluted with ethyl acetate, and the separated organic phase washed with 10 ml of 0.05 M aqueous HCI, saturated aqueous sodium bicarbonate, and then dried over anhydrous Na₂SO₄, filtered and concentrated under vacuum to give a pale yellow oily powder. The crude product was purified by flash chromatography with a gradient of ethanol/ethyl acetate in cyclohexane, and then further purified by flash chromatography with a gradient of acetonitrile in water to give the title compound as a colourless oily powder. LC/MS (method A); 458 [M+H]⁺; retention time: 1.03 min. ¹H NMR (400 MHz, CDCl₃) δ ppm 1.73 (s, 3 H) 3.89 (s, 3 H) 4.00 - 4.11 (m, 2 H) 6.94 - 7.06 (m, 2 H) 7.13 (dd, *J*=7.63, 0.73 Hz, 1 H) 7.24 - 7.34 (m, 4 H) 7.62 (t, *J*=7.81 Hz, 1 H) 7.97 - 8.04 (m, 1 H) 8.08 (br t, *J*=5.99 Hz, 1 H).

### Example P5: Preparation of N-[2-(6-chloro-2-pyridyl)-2-(1-methylpyrazol-4-yl)ethyl]-5-(2,4-difluorophenyl)isoxazole-3-carboxamide (Compound P-13, Table T1)

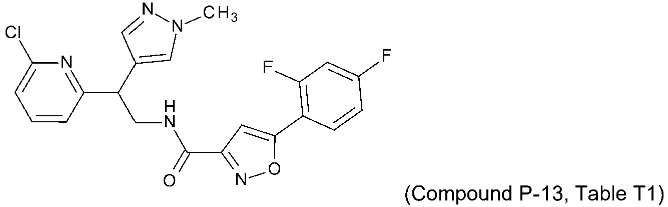

### Step 1: Preparation of (6-chloro-2-pyridyl)-(1-methylpyrazol-4-yl)methanol

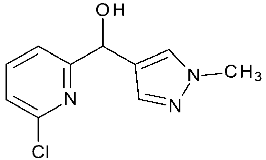

A 3-necked round bottom flask equipped with a magnetic stirred bar was charged with 4-iodo-1-methyl-1h-pyrazole (3.20 g, 15.1 mmol) and tetrahydrofuran (54.8 mL) under argon to give a colourless solution. Isopropylmagnesium chloride lithium chloride complex (17 mL, 21.9 mmol) was added dropwise at 0°C. The resulting white cloudy solution was stirred at 0°C for 20 minutes under argon. To this solution was added 6-chloropyridine-2-carbaldehyde (2.00 g, 13.7 mmol) and the resulting yellow suspension was stirred at 0°C for 10 minutes, and then allowed to warm to room temperature and stirred for 4 hours under argon. The reaction mixture was poured into 60 ml of water and extracted twice with 30 ml of ethyl acetate. The combined organic layers were washed once with brine, dried over anhydrous sodium sulfate, filtered, and concentrated under vacuum to give the crude compound. The crude product was purified by flash chromatography with a gradient of ethyl acetate in cyclohexane to give the title compound as a pale yellow oil. LC/MS (method A); 224 [M+H]⁺; retention time: 0.58 min. ¹H NMR (400 MHz, CDCl₃) δ ppm 3.45 - 3.79 (br s, 1 H) 3.90 (s, 3 H) 5.80 (s, 1 H) 7.25 - 7.30 (m, 2 H) 7.36 (s, 1 H) 7.46 (s, 1 H) 7.67 (t, *J*=7.81 Hz, 1 H).

### Step 2: Preparation of 2-(6-chloro-2-pyridyl)-2-(1-methylpyrazol-4-yl)acetonitrile

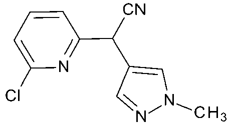

(6-chloro-2-pyridyl)-(1-methylpyrazol-4-yl)methanol (2.35 g, 10.5 mmol) was dissolved in dichloromethane (168 mL) and then treated with lithium carbonate (0.16 g, 2.10 mmol). Trimethylsilyl cyanide (6.10 mL, 47.3 mmol) and iodine (4.85 g, 18.9 mmol) were added successively at room temperature. The resulting dark light brown solution was stirred at 35°C for 2 hours. The reaction mixture was cooled to room temperature and poured into a saturated aqueous solution of Na₂S₂O₃ and extracted twice with dichloromethane. The combined organic layers were dried over anhydrous sodium sulfate, filtered and concentrated under vacuum to give 2.54 g of a brown oily solid. The crude product was purified by flash chromatography with an eluent of ethyl acetate to give the title compound as a pale brown oil. LC/MS (method A); 233 [M+H]+; retention time: 0.73 min. ¹H NMR (400 MHz, CDCl₃) δ ppm 3.94 (s, 3 H) 5.24 (s, 1 H) 7.34 (d, *J*=7.99 Hz, 1 H) 7.42 (d, *J*=7.63 Hz, 1 H) 7.52 (d, *J*=6.90 Hz, 2 H) 7.73 (t, *J*=7.81 Hz, 1 H).

### Step 3: Preparation of 2-(6-chloro-2-pyridyl)-2-(1-methylpyrazol-4-yl)ethanamine

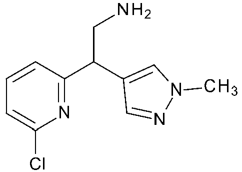

A solution of 2-(6-chloro-2-pyridyl)-2-(1-methylpyrazol-4-yl)acetonitrile (0.79 g, 3.38 mmol) in tetrahydrofuran (5 mL) was treated with BMS (5.07 mL, 10.1 mmol) at room temperature under argon and the resulting light brown solution was stirred for 1 hour at 65°C to give a light red suspension. The reaction mixture was cooled to 0°C before adding hydrochloric acid (2.26 mL, 13.6 mmol) dropwise. The resulting pale orange suspension was stirred for 1 hour at 65°C. Some water was added. It was then neutralised with NaOH 6 N to pH 12 and extracted three times with ethyl acetate. The combined organic layers were washed once with brine, dried over anhydrous sodium sulfate and concentrated under vacuum at 60°C to give 2-(6-chloro-2-pyridyl)-2-(1-methylpyrazol-4-yl)ethanamine (0.40 g, 50% yield) as a pale brown oil with small impurities. LC/MS (method A); 237 [M+H]⁺; retention time: 0.16 min and 0.26 min.

### Step 4: Preparation of N-[2-(6-chloro-2-pyridyl)-2-(1-methylpyrazol-4-yl)ethyl]-5-(2,4-difluorophenyl)isoxazole-3-carboxamide (Compound P-13, Table T1)

5-(2,4-difluorophenyl)isoxazole-3-carboxylic acid (0.024 g, 0.10 mmol) was added to a pale brown solution of 2-(6-chloro-2-pyridyl)-2-(1-methylpyrazol-4-yl)ethanamine (0.022 g, 1.0, 0.09 mmol) in N,N-dimethylformamide (0.46 mL). N,N-diisopropylethylamine (0.049 g, 0.065 mL, 0.37 mmol) and (1-[bis(dimethylamino)methylene]-1H-1,2,3-triazolo[4,5-b]pyridinium 3-oxide hexafluorophosphate (HATU) (0.043 g, 0.11 mmol) were then added slowly to give a light brown solution (slightly exothermic additions). It was stirred for 1 hour at room temperature. The reaction mixture was diluted with water and then extracted twice with ethyl acetate. The combined organic layers were washed once with brine, dried over anhydrous Na₂SO₄, filtered, and concentrated under vacuum to give the crude product as a pale brown oil. The crude was purified by flash chromatography eluting with ethyl acetate in cyclohexane to give N-[2-(6-chloro-2-pyridyl)-2-(1-methylpyrazol-4-yl)ethyl]-5-(2,4-difluorophenyl)isoxazole-3-carboxamide as a pale yellow oil. LC/MS (method A); 444 [M+H]⁺; retention time: 1.00 min.

### Example P6: Preparation of N-[2-(6-bromo-2-pyridyl)-2-(1-methylpyrazol-4-yl)propyll-5-(2,4-difluorophenyl)-1,3,4-thiadiazole-2-carboxamide (Compound P-5, Table T1)

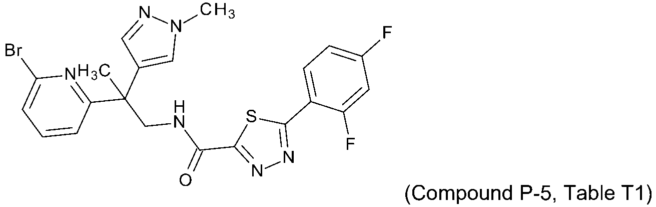

### Step 1: Preparation of 2-(6-bromo-2-pyridyl)-2-(1-methylpyrazol-4-yl)propan-1-amine

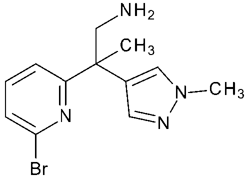

2-(1-methylpyrazol-4-yl)propanenitrile (0.10 g, 0.74 mmol, prepared as previously described in Example 1, step 1) was dissolved in tetrahydrofuran (3.00 mL) under argon and the pale yellow solution cooled to - 78°C. To this solution n-butyllithium (0.30 mL, 0.74 mmol) was added dropwise and the resulting pale brown suspension was stirred at this temperature for 10 minutes before adding 2,6-dibromopyridine (0.18 g, 0.74 mmol). The resulting brown suspension was stirred at -78°C for 5 minutes, allowed to warm to room temperature, and stirred for 30 minutes under argon to give 2-(6-bromo-2-pyridyl)-2-(1-methylpyrazol-4-yl)propanenitrile as a yellow solution in THF. LC/MS (method A); 291 [M+H]⁺; retention time: 0.83 min.

To this solution was added BMS (0.38 mL, 4.03 mmol) at room temperature under argon and the resulting colourless solution was stirred at 65°C for 1 hour. The reaction mixture was then cooled to 0°C and concentrated hydrochloric acid (0.90 mL, 5.40 mmol) was added dropwise. The reaction mixture was stirred for 30 minutes at 65°C. The reaction mixture was diluted with water and then treated with NaOH 6N to adjust the mixture to pH 10. The mixture was extracted three times with ethyl acetate and the combined organic layers were washed with brine, dried over anhydrous sodium sulfate and concentrated under vacuum to give 2-(6-bromo-2-pyridyl)-2-(1-methylpyrazol-4-yl)propan-1-amine as a colourless oil. LC/MS (method A); 295 [M+H]⁺; retention time: 0.47 min.

### Step 2: Preparation of N-[2-(6-bromo-2-pyridyl)-2-(1-methylpyrazol-4-yl)propyl]-5-(2,4-difluorophenyl)-1,3,4-thiadiazole-2-carboxamide (Compound P-5, Table T1)

Trimethylaluminum (0.86 mL, 1.73 mmol) was added dropwise to a light brown suspension of 2-(6-bromo-2-pyridyl)-2-(1-methylpyrazol-4-yl)propan-1-amine (0.26 g, 0.86 mmol) and ethyl 5-(2,4-difluorophenyl)-1,3,4-thiadiazole-2-carboxylate (0.30 g, 1.12 mmol) in toluene (3.46 mL) under argon. The resulting dark light brown solution was stirred for at 90°C for 1 hour under argon. The reaction mixture was cooled to 0°C and quenched carefully by addition of HCI 1M (1 mL). The mixture was diluted with some saturated NaHCO₃ and extracted twice ethyl acetate. The combined organic layers were washed once with brine, dried over anhydrous sodium sulfate, filtered and concentrated under vacuum to give the crude product as a light brown oil. This was purified by using flash chromatography with the eluent of ethanol/ethyl acetate in cyclohexane to give N-[2-(6-bromo-2-pyridyl)-2-(1-methylpyrazol-4-yl)propyl]-5-(2,4-difluorophenyl)-1,3,4-thiadiazole-2-carboxamide as a yellow powder. LC/MS (method A); 419 [M+H]⁺; retention time: 1.08 min. ¹H NMR (400 MHz, CDCl₃) δ ppm 1.75 (s, 3 H) 3.89 (s, 3 H) 4.04 - 4.20 (m, 2 H) 7.00 - 7.13 (m, 2 H) 7.17 (d, *J*=7.63 Hz, 1 H) 7.25 - 7.31 (m, 1 H) 7.35 (s, 1 H) 7.37 - 7.44 (m, 1 H) 7.46 - 7.53 (m, 1 H) 8.32 (br t, *J*=6.18 Hz, 1 H) 8.45 (td, *J*=8.45, 6.36 Hz, 1 H).

### Example P7: Preparation of 5-(2,4-difluorophenyl)-N-[2-(6-ethylsulfonyl-2-pyridyl)-2-(1-methylpyrazol-4-yl)propyl]-1,3,4-thiadiazole-2-carboxamide (Compound P-3, Table T1)

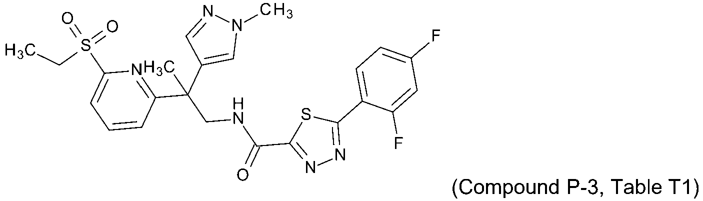

Sodium ethanesulfinate (0.04 g, 0.31 mmol) was added to a pale green-yellow solution of N-[2-(6-bromo-2-pyridyl)-2-(1-methylpyrazol-4-yl)propyl]-5-(2,4-difluorophenyl)-1,3,4-thiadiazole-2-carboxamide (0.04 g, 0.08 mmol) and copper(i) iodide (0.016 g, 0.09 mmol) in dimethyl sulfoxide (DMSO, 0.39 mL) under argon. The resulting pale brown-green solution was stirred for at 90°C under argon for 20 hours to give a light brown solution. The mixture was extracted with ethyl acetate and water. The organic layer was washed once with brine, dried over anhydrous Na₂SO₄, filtered and evaporated under vacuum at 60°C to give 40 mg of a pale brown oil. The crude was purified by using flash chromatography with eluent ethanol/ethyl acetate in cyclohexane to give 5-(2,4-difluorophenyl)-N-[2-(6-ethylsulfonyl-2-pyridyl)-2-(1-methylpyrazol-4-yl)propyl]-1,3,4-thiadiazole-2-carboxamide (5.5 mg, 13% yield) as a pale brown oil. LC/MS standard 533 [M+H]+; retention time: 0.94 min. ¹H NMR (400 MHz, CDCl₃) δ ppm 1.39 - 1.51 (t, 3 H) 1.80 (s, 3 H) 3.68 (q, *J*=7.63 Hz, 2 H) 3.91 (s, 3 H) 4.10 - 4.25 (m, 2 H) 7.01 - 7.14 (m, 2 H) 7.29 (s, 1 H) 7.34 (s, 1 H) 7.48 (dd, *J*=7.99, 0.73 Hz, 1 H) 7.87 - 7.97 (m, 1 H) 8.05 (dd, *J*=7.63, 1.09 Hz, 1 H) 8.25 (br t, *J*=6.54 Hz, 1 H) 8.43 (td, *J*=8.54, 6.18 Hz, 1 H).

Further examples of synthesized compounds are shown in Table T1.

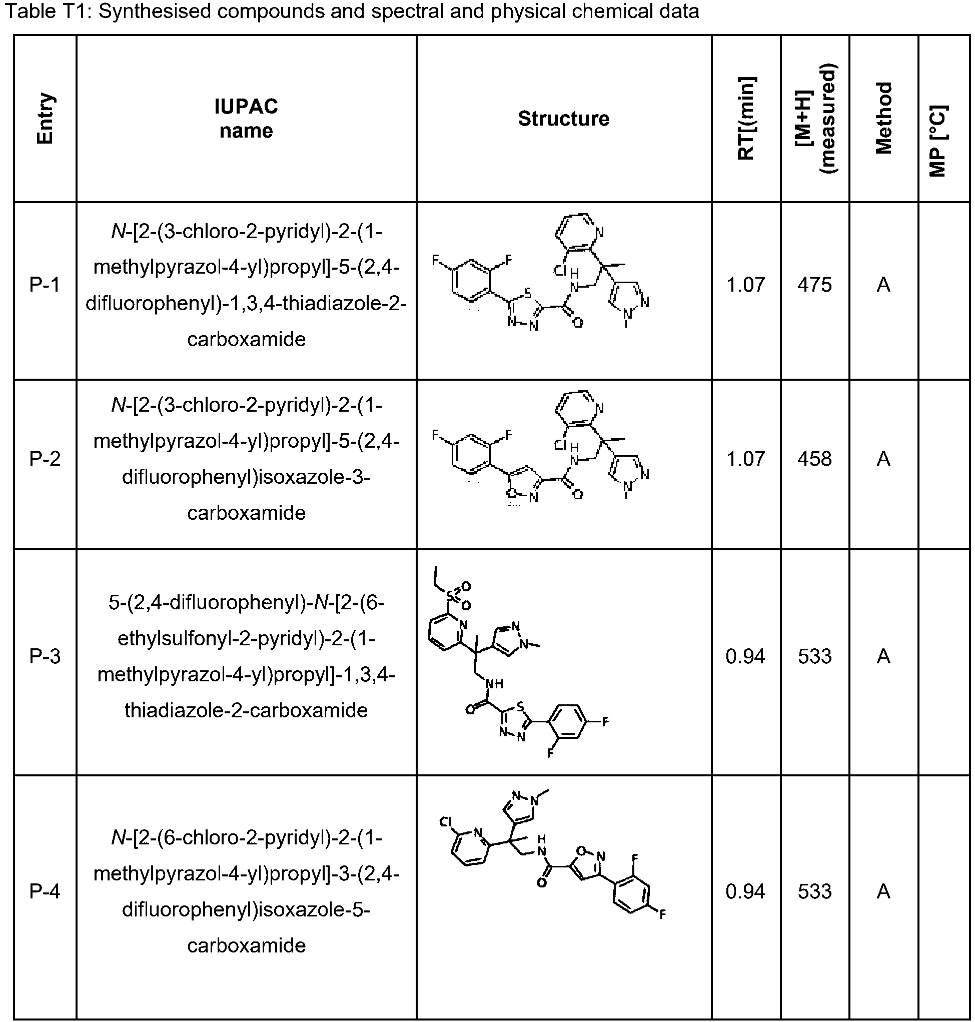

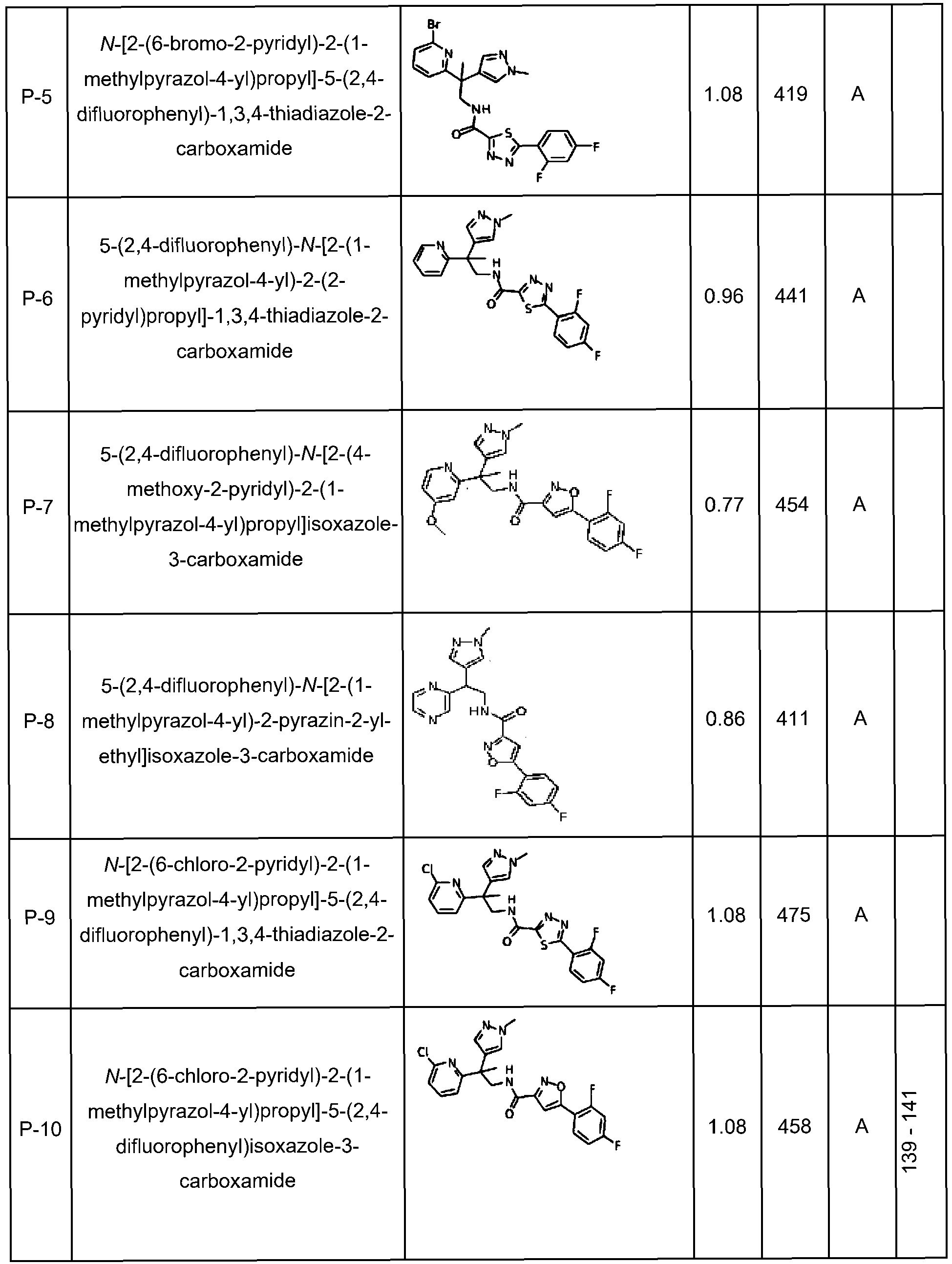

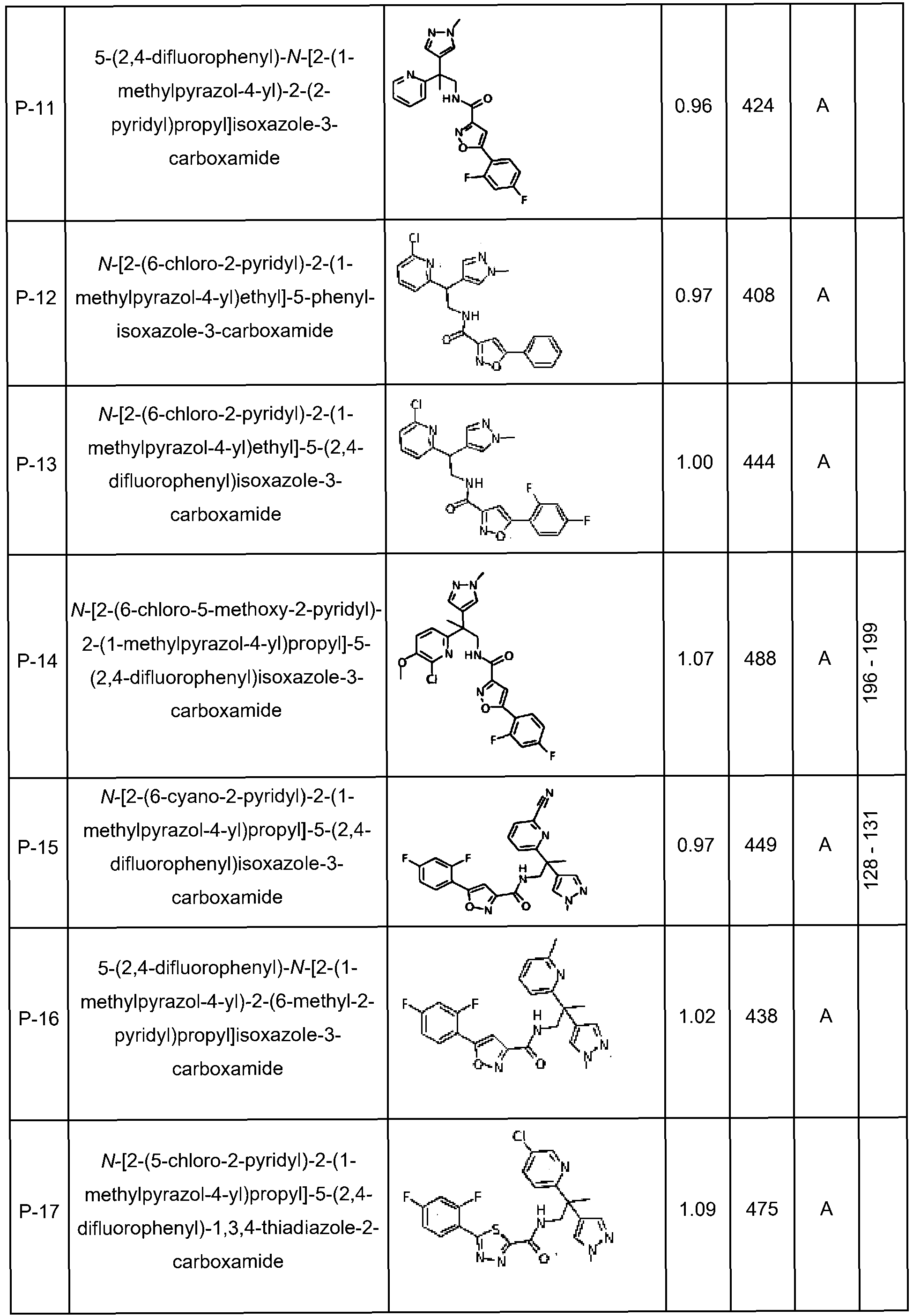

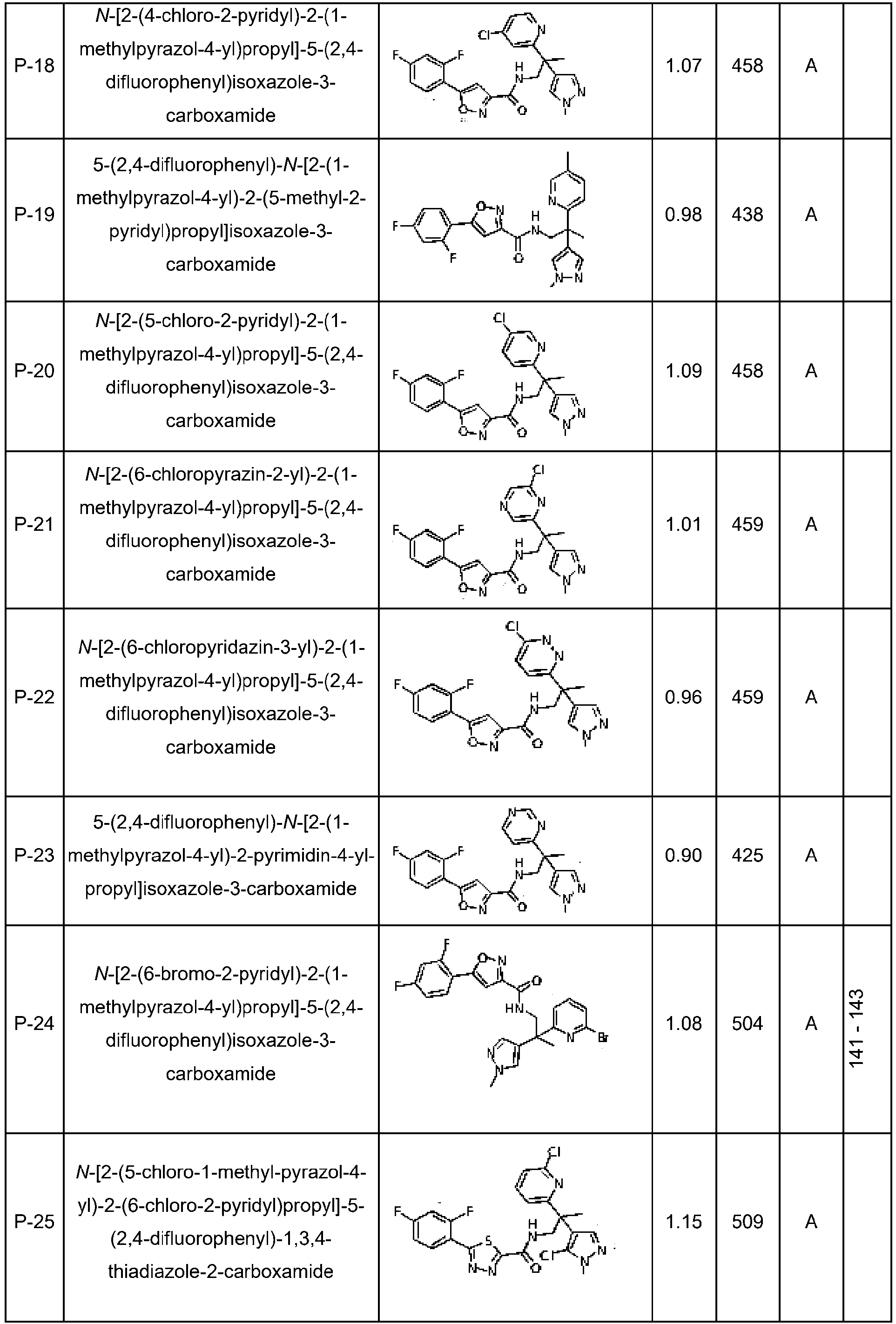

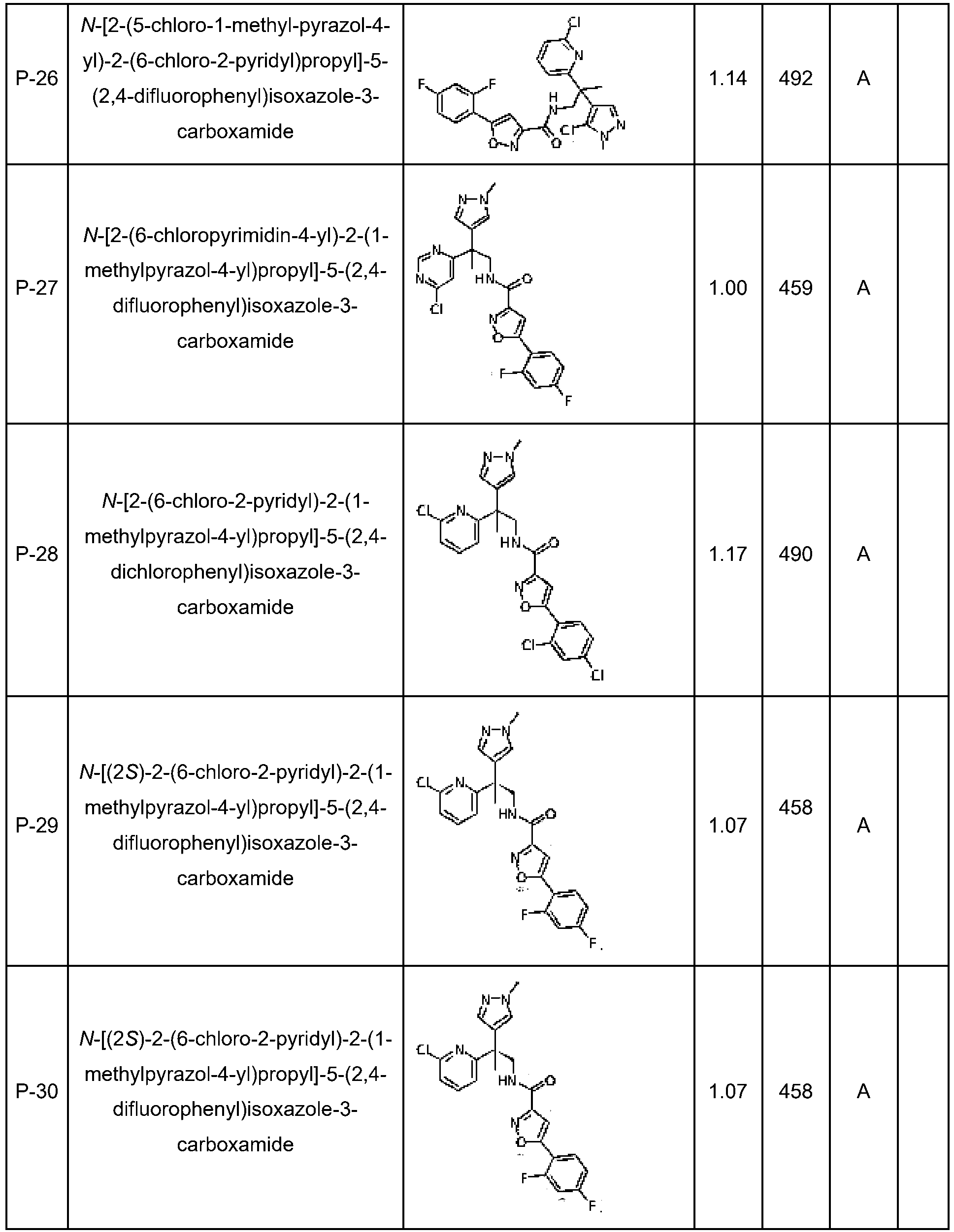

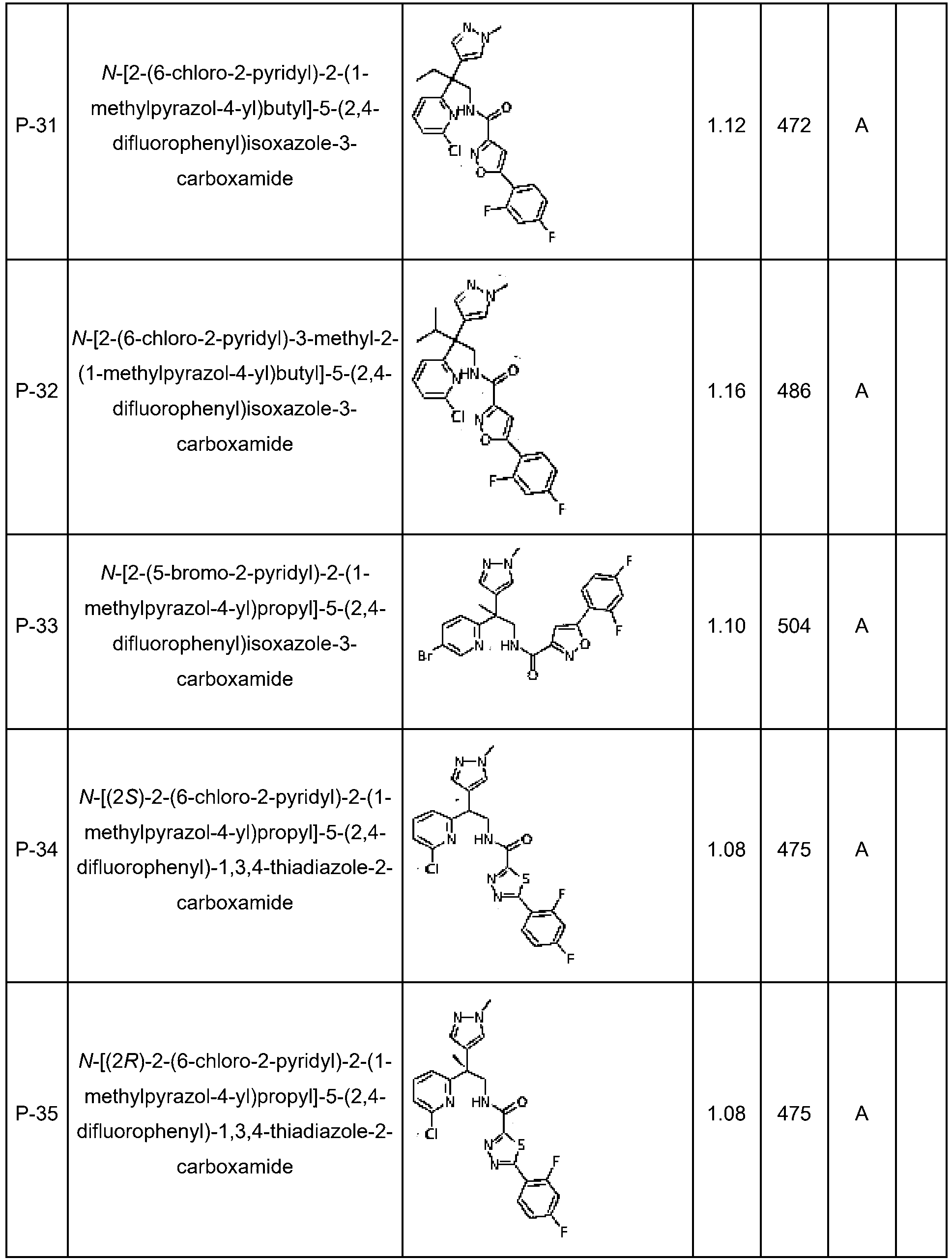

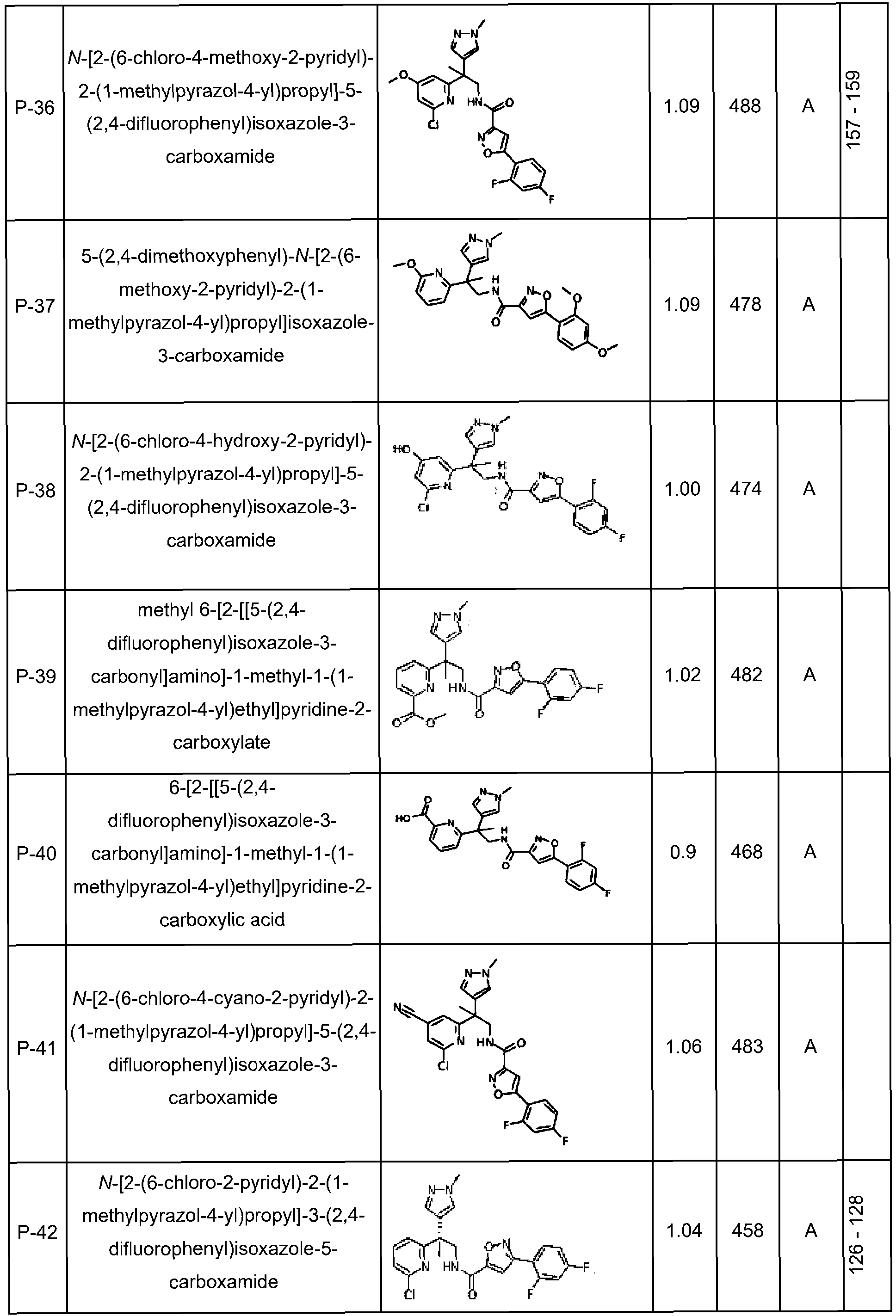

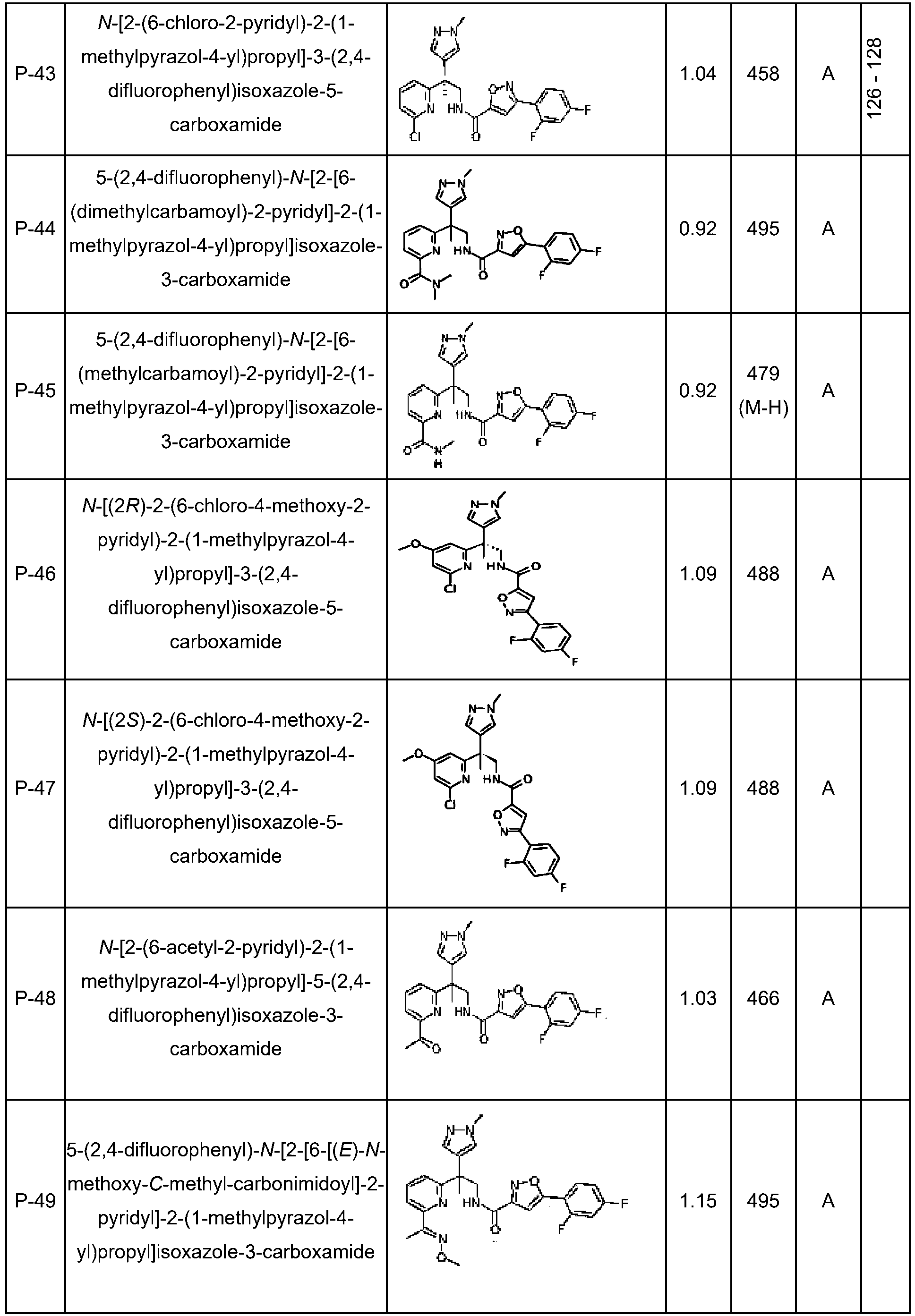

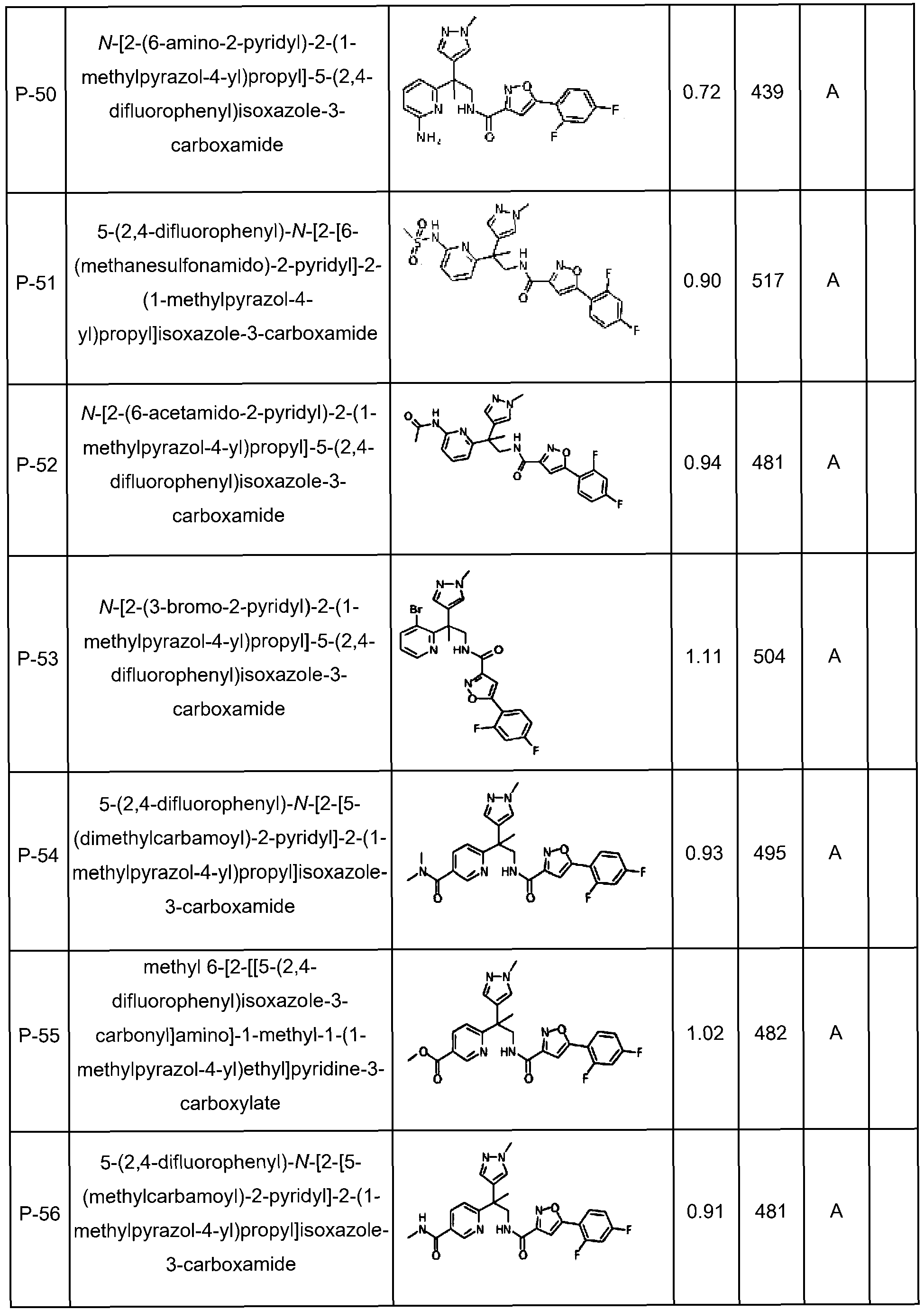

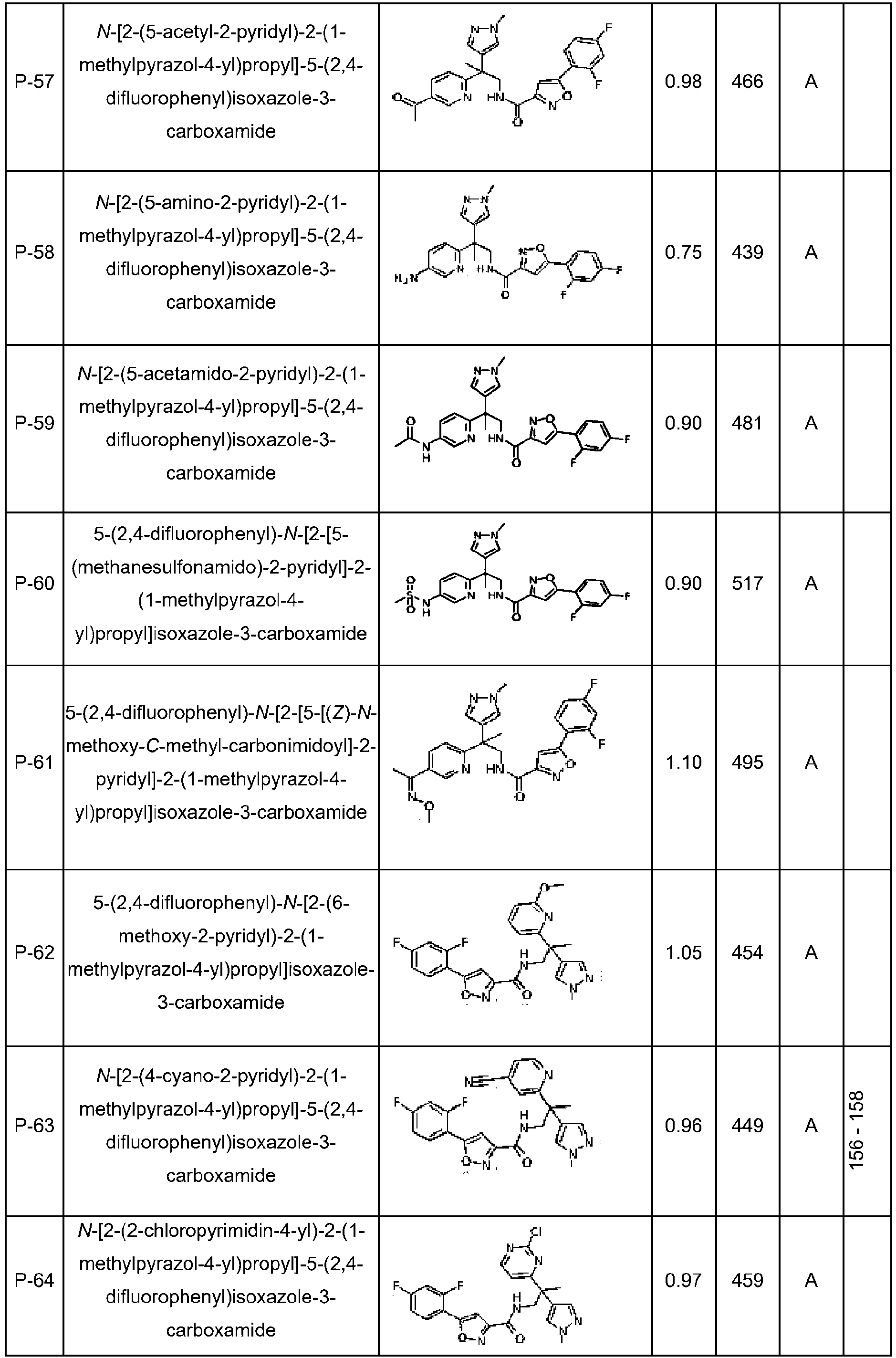

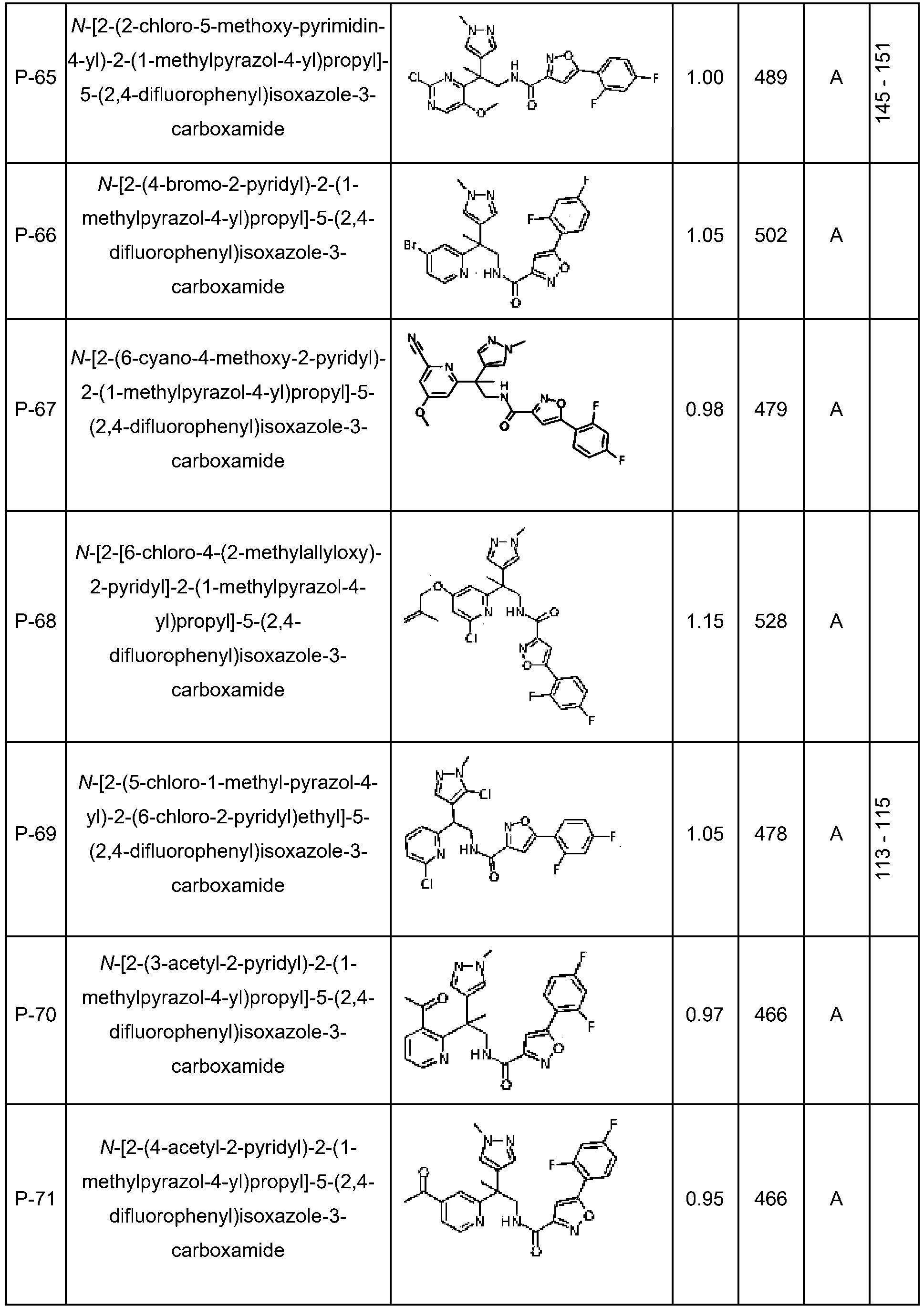

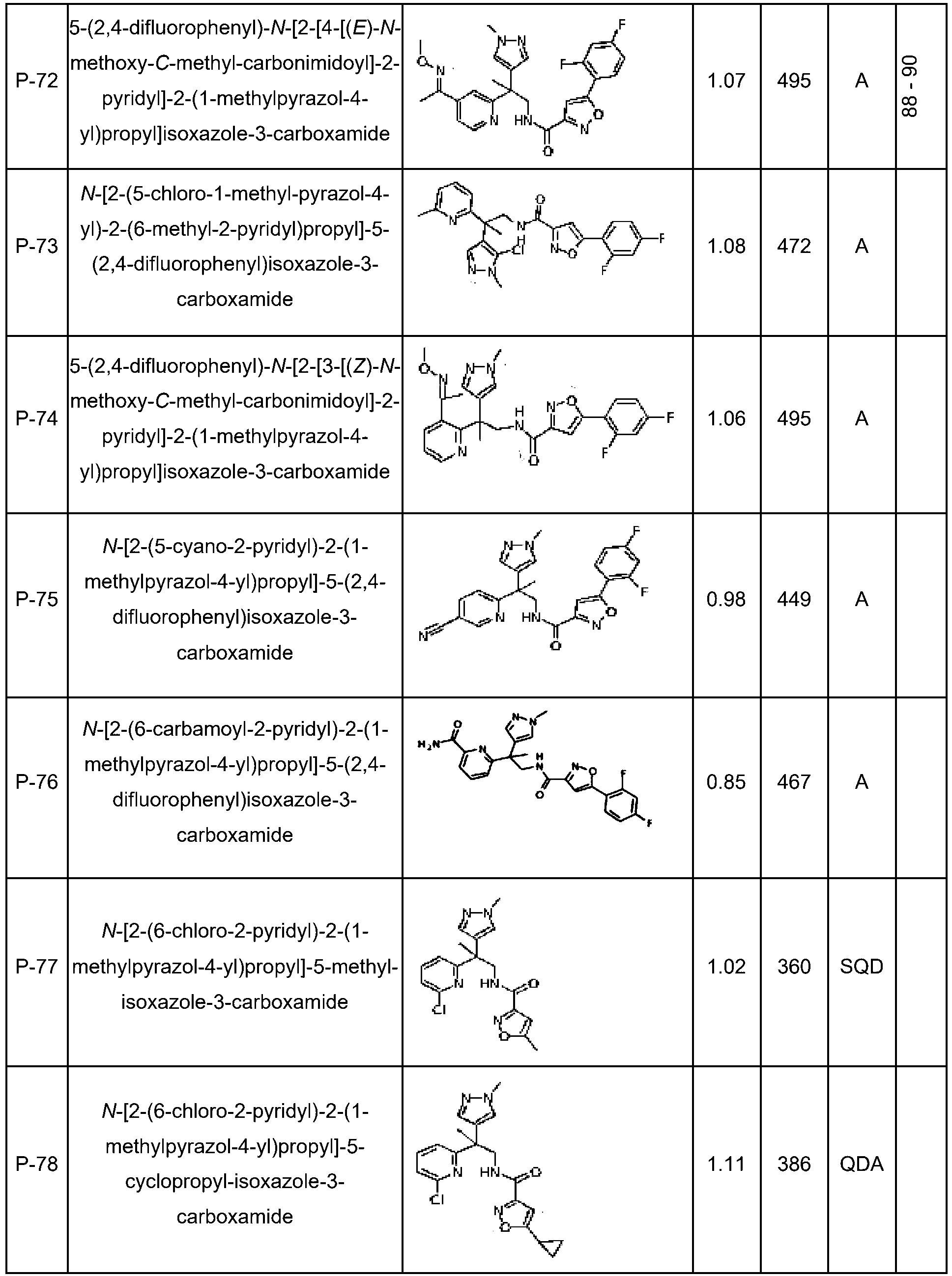

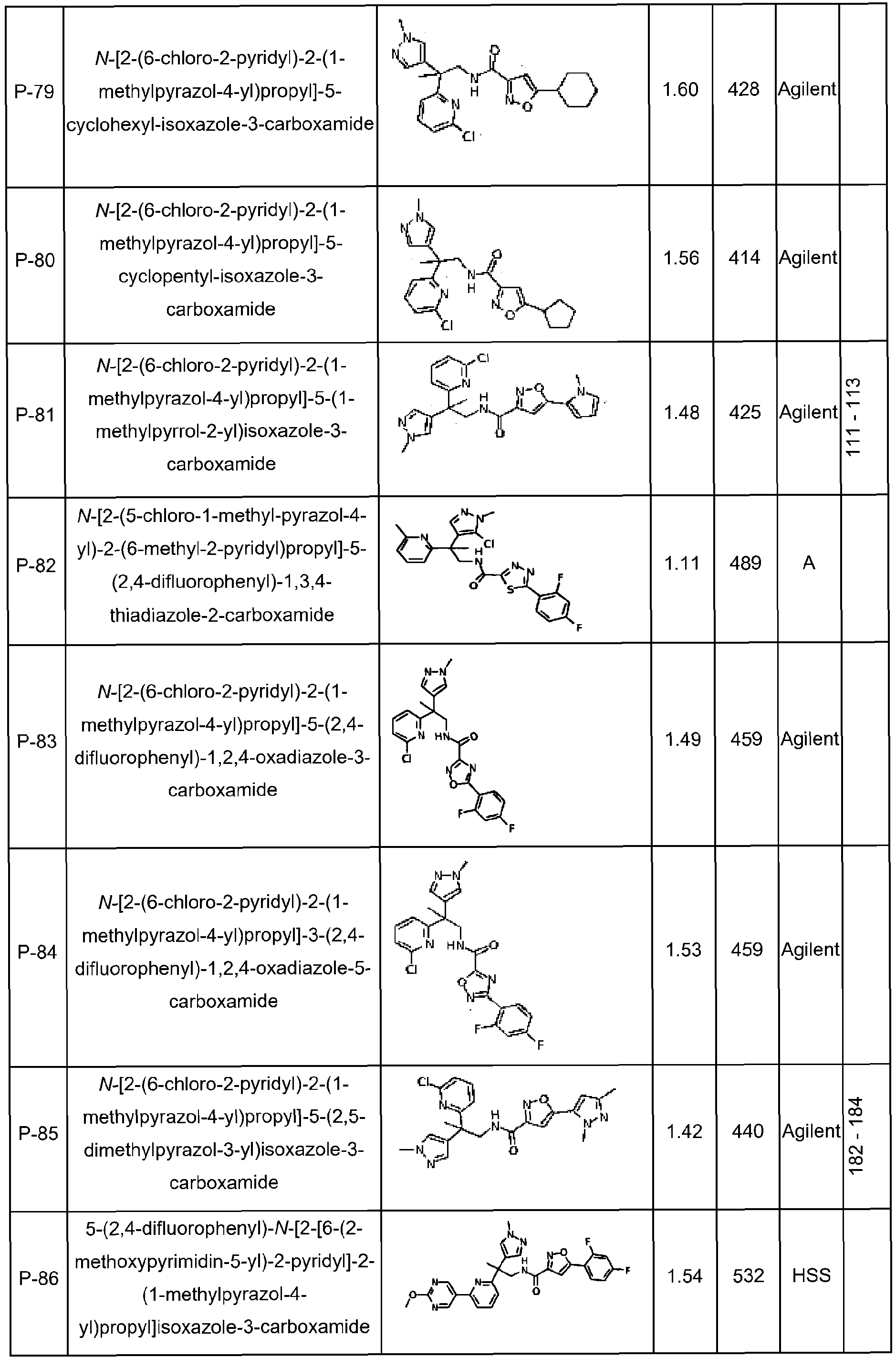

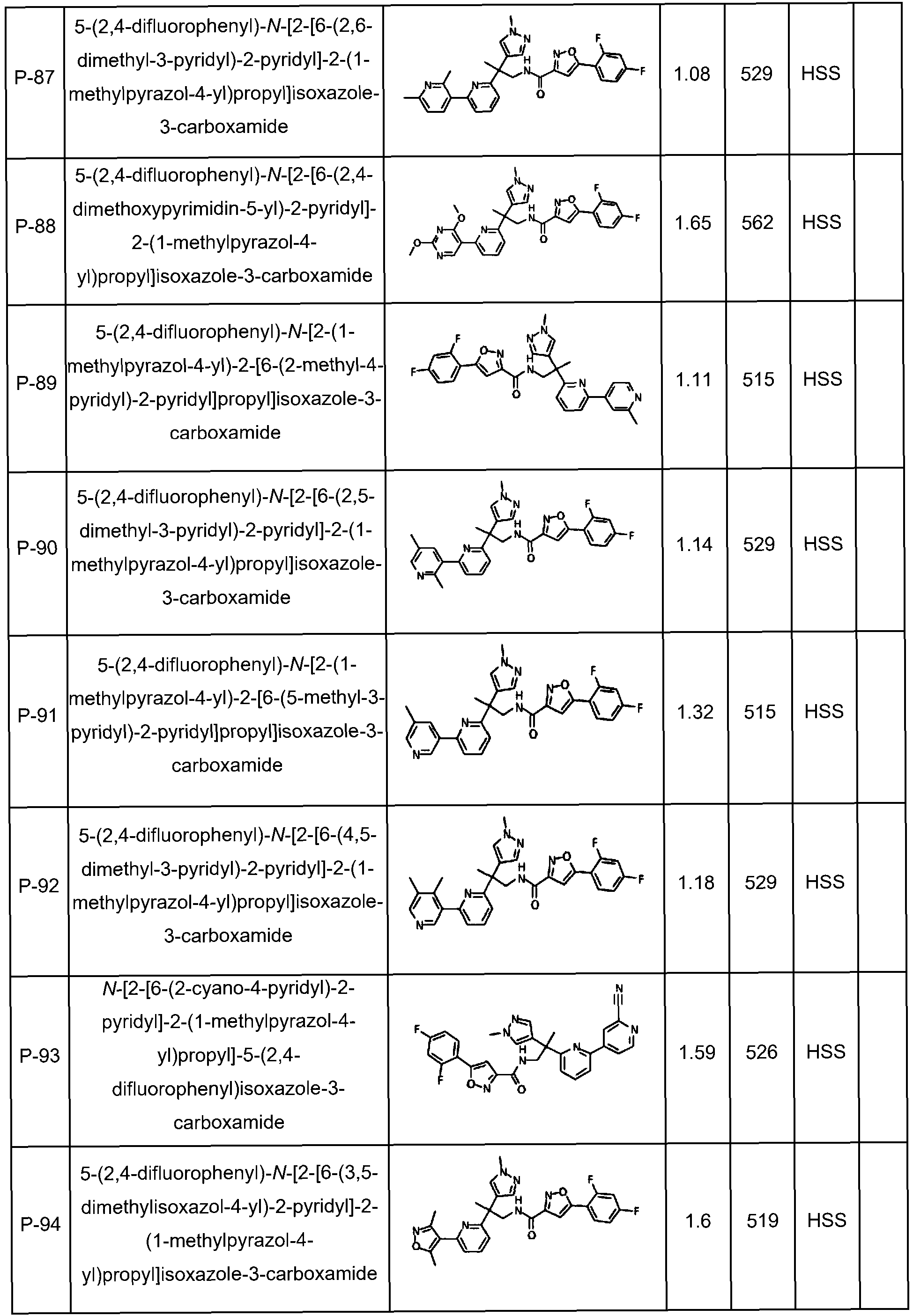

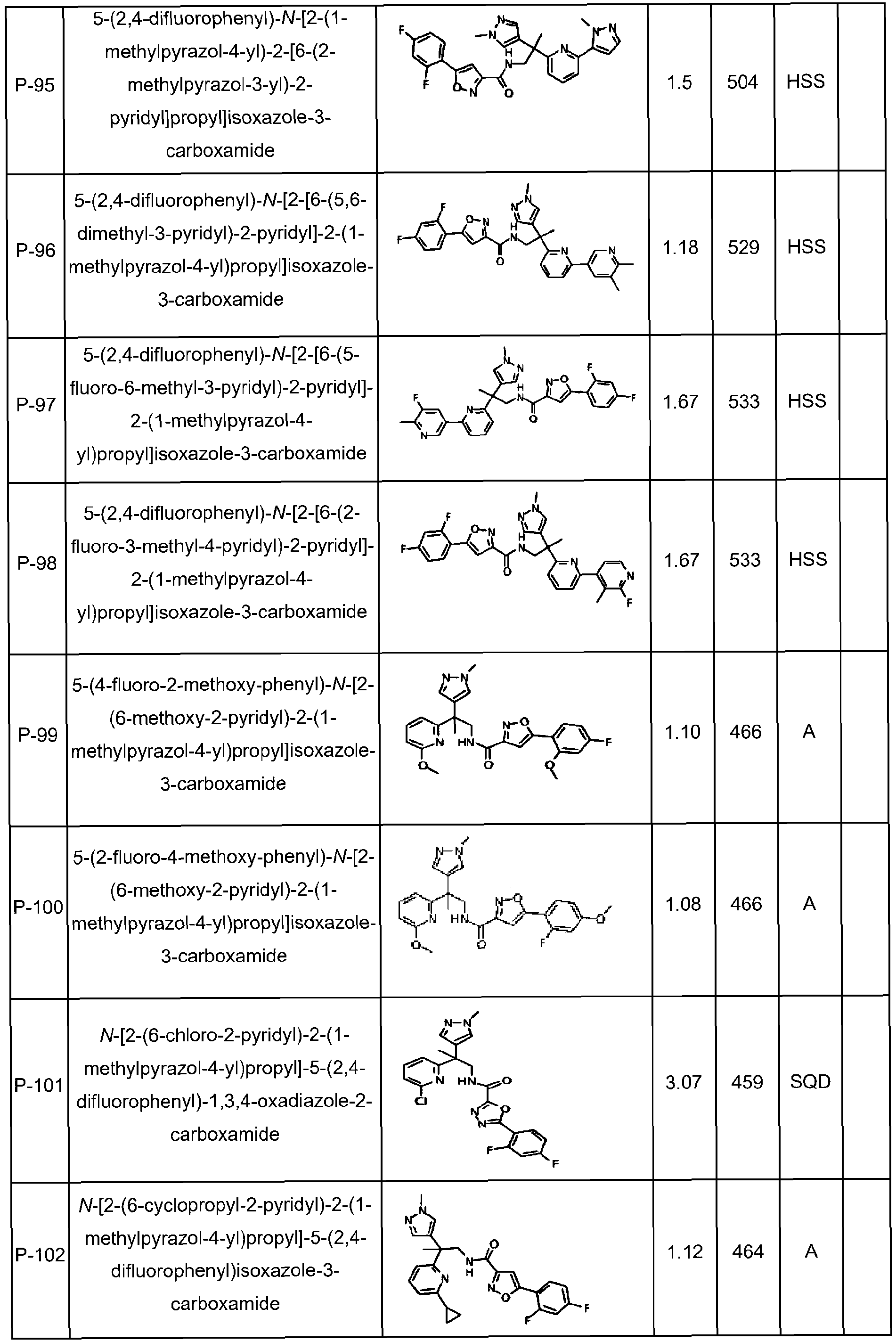

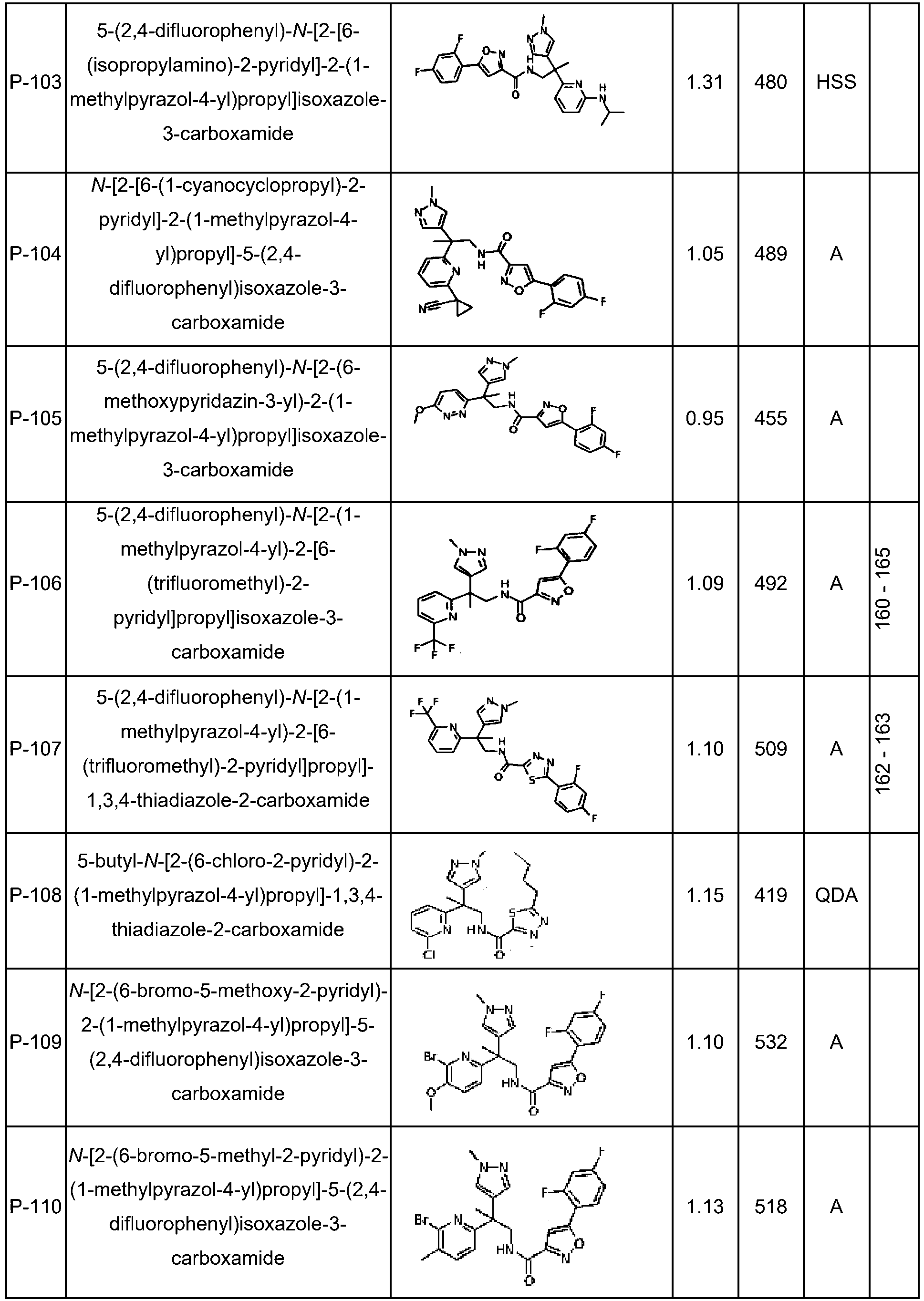

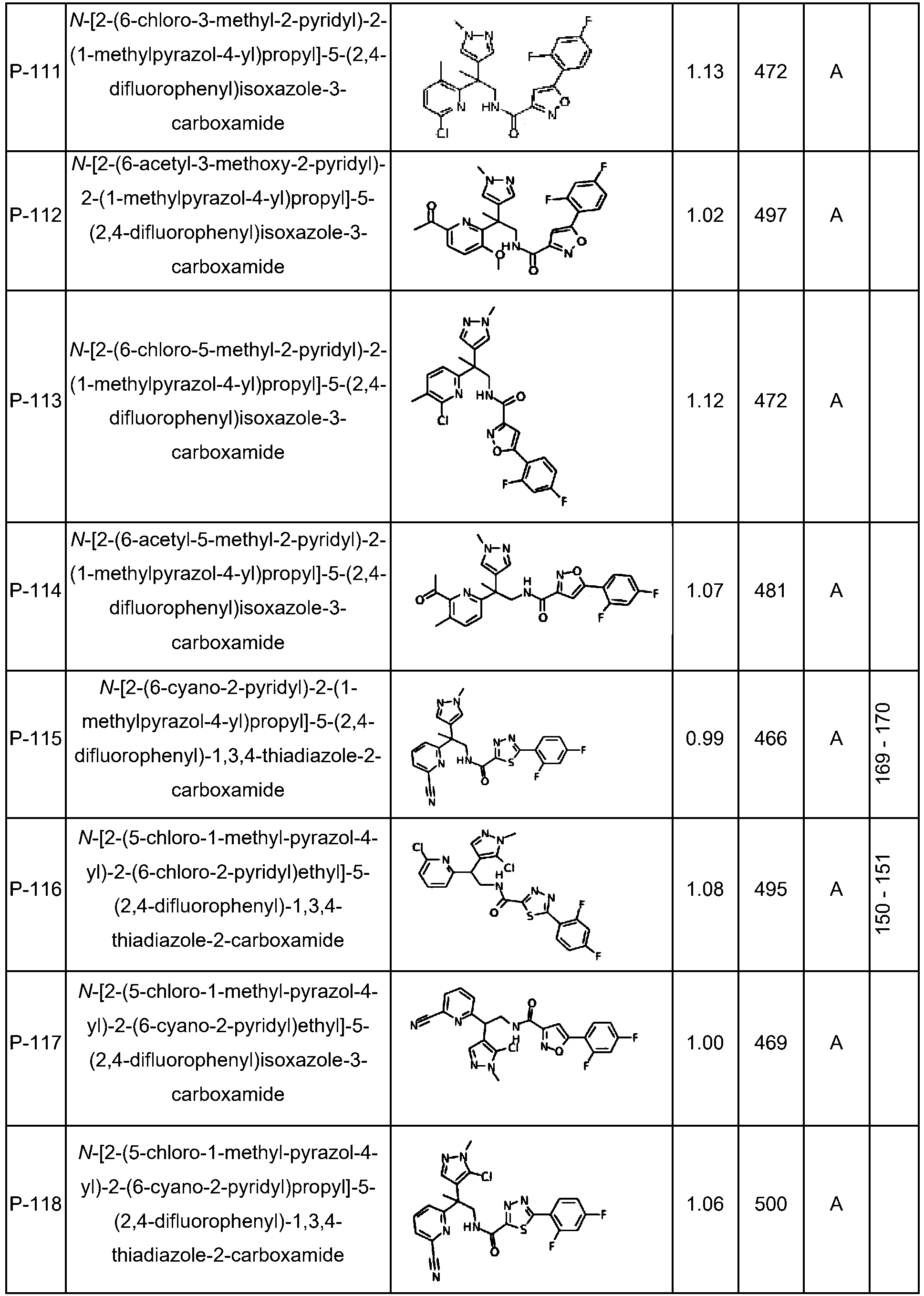

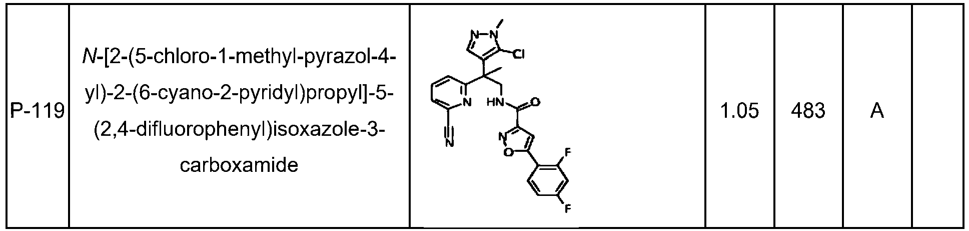

### BIOLOGICAL EXAMPLES

### Example B1: Alternaria solani / tomato / leaf disc (early blight)

Tomato leaf disks cv. Baby are placed on agar in multiwell plates (24-well format) and sprayed with the formulated test compound diluted in water. The leaf disks are inoculated with a spore suspension of the fungus 2 days after application. The inoculated leaf disks are incubated at 23 °C / 21°C (day/night) and 80% rh under a light regime of 12/12 h (light/dark) in a climate cabinet and the activity of a compound is assessed as percent disease control compared to untreated when an appropriate level of disease damage appears on untreated check disk leaf disks (5 - 7 days after application). The following compounds gave at least 80% control of Alternaria solani at 200 ppm when compared to untreated control under the same conditions, which showed extensive disease development: P-4, P-5, P-6, P-7, P-9, P-10, P-11, P-15, P-19, P-22, P-23, P-24, P-25, P-26, P-29, P-30, P-31, P-32, P-34, P-35, P-42, P-43, P-44, P-48, P-49, P-61, P-64, P-73, P-75, P-80, P-82, P-83, P-84, P-101, P-105, P-106, P-107, and P-110.

### Example B2: Botryotinia fuckeliana (Botrytis cinerea) / liquid culture (Gray mould)

Conidia of the fungus from cryogenic storage are directly mixed into nutrient broth (Vogels broth). After placing a (DMSO) solution of test compound into a microtiter plate (96-well format), the nutrient broth containing the fungal spores is added. The test plates are incubated at 24 °C and the inhibition of growth is determined photometrically 3-4 days after application. The following compounds gave at least 80% control of Botryotinia fuckeliana at 20 ppm when compared to untreated control under the same conditions, which showed extensive disease development: P-1, P-2, P-4, P-5, P-6, P-9, P-10, P-11, P-13, P-15, P-16, P-19, P-21, P-24, P-25, P-26, P-29, P-30, P-31, P-32, P-34, P-35, P-36, P-38, P-39, P-41, P-42, P-43, P-46, P-47, P-48, P-49, P-50, P-55, P-64, P-68, P-69, P-73, P-80, P-81, P-82, P-83, P-84, P-88, P-101, P-102, P-106, P-107, P-108, P-110, P-115, P-116, P-117, P-118, and P-119

### Example B3: Glomerella lagenarium (Colletotrichum lagenarium) / liquid culture (Anthracnose)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is measured photometrically 3-4 days after application. The following compounds gave at least 80% control of Glomerella lagenarium at 20 ppm when compared to untreated control under the same conditions, which showed extensive disease development: P-4, P-5, P-6, P-7, P-9, P-10, P-11, P-15, P-16, P-17, P-19, P-20, P-24, P-25, P-26, P-28, P-29, P-30, P-31, P-33, P-34, P-35, P-36, P-39, P-42, P-43, P-46, P-48, P-49, P-50, P-55, P-57, P-61, P-64, P-68, P-69, P-73, P-79, P-80, P-81, P-82, P-83, P-84, P-85, P-101, P-102, P-106, P-107, P-108, P-110, P-115, P-116, P-117, P-118, and P-119

### Example B4: Blumeria graminis f. sp. tritici (Erysiphe graminis f. sp. tritici) / wheat / leaf disc preventative (Powdery mildew on wheat)

Wheat leaf segments cv. Kanzler are placed on agar in a multiwell plate (24-well format) and sprayed with the formulated test compound diluted in water. The leaf disks are inoculated by shaking powdery mildew infected plants above the test plates 1 day after application. The inoculated leaf disks are incubated at 20 °C and 60% rh under a light regime of 24 h darkness followed by 12 h light / 12 h darkness in a climate chamber and the activity of a compound is assessed as percent disease control compared to untreated when an appropriate level of disease damage appears on untreated check leaf segments (6 - 8 days after application). The following compounds gave at least 80% control of Blumeria graminis f. sp. tritici at 200 ppm when compared to untreated control under the same conditions, which showed extensive disease development: P-4, P-5, P-6, P-8, P-9, P-10, P-11, P-15, P-16, P-19, P-21, P-24, P-25, P-26, P-29, P-30, P-31, P-34, P-35, P-36, P-42, P-43, P-49, P-59, P-64, P-69, P-73, P-82, P-83, P-84, P-85, P-101, P-107, and P-110

### Example B5: Fusarium culmorum / liquid culture (Head blight)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is determined photometrically 3-4 days after application. The following compounds gave at least 80% control of Fusarium culmorum at 20 ppm when compared to untreated control under the same conditions, which showed extensive disease development: P-4, P-5, P-9, P-10, P-15, P-16, P-24, P-25, P-26, P-29, P-30, P-31, P-34, P-35, P-36, P-42, P-43, P-46, P-49, P-73, P-80, P-82, P-83, P-84, P-102, P-106, P-107, P-108, P-110, P-115, P-116, P-118, P-119

### Example B6: Fusarium culmorum / wheat / spikelet preventative (Head blight)

Wheat spikelets cv. Monsun are placed on agar in multiwell plates (24-well format) and sprayed with the formulated test compound diluted in water. The spikelets are inoculated with a spore suspension of the fungus 1 day after application. The inoculated spikelets are incubated at 20 °C and 60% rh under a light regime of 72 h semi darkness followed by 12 h light / 12 h darkness in a climate chamber and the activity of a compound is assessed as percent disease control compared to untreated when an appropriate level of disease damage appears on untreated check spikelets (6 - 8 days after application). The following compounds gave at least 80% control of Fusarium culmorum at 200 ppm when compared to untreated control under the same conditions, which showed extensive disease development: P-4, P-5, P-10, P-16, P-29, P-31, P-34, P-35, P-36, P-42, P-46, P-49, and P-73

### Example B7: Gibberella zeae (Fusarium graminearum) / wheat / spikelet preventative (Head blight)

Wheat spikelets cv. Monsun are placed on agar in multiwell plates (24-well format) and sprayed with the formulated test compound diluted in water. One day after application, the spikelets are inoculated with a spore suspension of the fungus. The inoculated test leaf disks are incubated at 20 °C and 60% rh under a light regime of 72 h semi darkness followed by 12 h light / 12 h darkness in a climate chamber, the activity of a compound is assessed as percent disease control compared to untreated when an appropriate level of disease damage appears on untreated check spikelets (6 - 8 days after application). The following compounds gave at least 80% control of Gibberella zeae at 200 ppm when compared to untreated control under the same conditions, which showed extensive disease development: P-4, P-5, P-9, P-16, P-24, P-26, P-30, P-35, P-36, P-42, P-46, P-73, P-80, and P-82

### Example B8: Phaeosphaeria nodorum (Septoria nodorum) / wheat / leaf disc preventative (Glume blotch)

Wheat leaf segments cv. Kanzler are placed on agar in a multiwell plate (24-well format) and sprayed with the formulated test compound diluted in water. The leaf disks are inoculated with a spore suspension of the fungus 2 days after application. The inoculated test leaf disks are incubated at 20 °C and 75% rh under a light regime of 12 h light / 12 h darkness in a climate cabinet and the activity of a compound is assessed as percent disease control compared to untreated when an appropriate level of disease damage appears in untreated check leaf disks (5 - 7 days after application). The following compounds gave at least 80% control of Phaeosphaeria nodorum at 200 ppm when compared to untreated control under the same conditions, which showed extensive disease development: P-4, P-5, P-6, P-9, P-10, P-11, P-15, P-24, P-25, P-26, P-29, P-30, P-31, P-32, P-34, P-35, P-39, P-42, P-43, P-44, P-48, P-49, P-61, P-64, P-69, P-73, P-82, P-83, P-84, P-101, P-102, and P-110

### Example B9: Monographella nivalis (Microdochium nivale) / liquid culture (foot rot cereals)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is determined photometrically 4-5 days after application. The following compounds gave at least 80% control of Monographella nivalis at 20 ppm when compared to untreated control under the same conditions, which showed extensive disease development: P-1, P-2, P-4, P-5, P-6, P-7, P-9, P-10, P-11, P-13, P-15, P-16, P-17, P-18, P-19, P-20, P-22, P-24, P-25, P-26, P-28, P-29, P-30, P-31, P-32, P-33, P-34, P-35, P-36, P-39, P-42, P-43, P-46, P-47, P-48, P-49, P-50, P-53, P-55, P-57, P-58, P-61, P-64, P-67, P-68, P-69, P-72, P-73, P-75, P-78, P-79, P-80, P-81, P-82, P-83, P-84, P-88, P-89, P-91, P-94, P-95, P-101, P-102, P-103, P-104, P-106, P-107, P-108, P-110, P-115, P-116, P-117, P-118, and P-119

### Example B10: Mycosphaerella arachidis (Cercospora arachidicola) / liquid culture (early leaf spot)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is determined photometrically 4-5 days after application. The following compounds gave at least 80% control of Mycosphaerella arachidis at 20 ppm when compared to untreated control under the same conditions, which showed extensive disease development: P-4, P-5, P-9, P-10, P-15, P-16, P-17, P-24, P-25, P-26, P-29, P-30, P-31, P-34, P-35, P-36, P-42, P-43, P-46, P-48, P-49, P-50, P-61, P-68, P-69, P-73, P-78, P-79, P-80, P-81, P-82, P-83, P-84, P-101, P-102, P-106, P-107, P-108, P-110, P-115, P-116, P-117, P-118, and P-119

### Example B11: Puccinia recondita f. sp. tritici / wheat / leaf disc curative (Brown rust)

Wheat leaf segments cv. Kanzler are placed on agar in multiwell plates (24-well format). The leaf segments are inoculated with a spore suspension of the fungus. Plates are stored in darkness at 19 °C and 75% rh. The formulated test compound diluted in water is applied 1 day after inoculation. The leaf segments are incubated at 19 °C and 75% rh under a light regime of 12 h light / 12 h darkness in a climate cabinet and the activity of a compound is assessed as percent disease control compared to untreated when an appropriate level of disease damage appears in untreated check leaf segments (6 - 8 days after application). The following compounds gave at least 80% control of Puccinia recondita f. sp. tritici at 200 ppm when compared to untreated control under the same conditions, which showed extensive disease development: P-19

### Example B12: Puccinia recondita f. sp. tritici / wheat / leaf disc preventative (Brown rust)

Wheat leaf segments cv. Kanzler are placed on agar in multiwell plates (24-well format) and sprayed with the formulated test compound diluted in water. The leaf disks are inoculated with a spore suspension of the fungus 1 day after application. The inoculated leaf segments are incubated at 19 °C and 75% rh under a light regime of 12 h light / 12 h darkness in a climate cabinet and the activity of a compound is assessed as percent disease control compared to untreated when an appropriate level of disease damage appears in untreated check leaf segments (7 - 9 days after application). The following compounds gave at least 80% control of Puccinia recondita f. sp. tritici at 200 ppm when compared to untreated control under the same conditions, which showed extensive disease development: P-10, P-20

### Example B13: Magnaporthe grisea (Pyricularia oryzae) / rice / leaf disc preventative (Rice Blast)

Rice leaf segments cv. Ballila are placed on agar in a multiwell plate (24-well format) and sprayed with the formulated test compound diluted in water. The leaf segments are inoculated with a spore suspension of the fungus 2 days after application. The inoculated leaf segments are incubated at 22 °C and 80% rh under a light regime of 24 h darkness followed by 12 h light / 12 h darkness in a climate cabinet and the activity of a compound is assessed as percent disease control compared to untreated when an appropriate level of disease damage appears in untreated check leaf segments (5 - 7 days after application). The following compounds gave at least 80% control of Magnaporthe grisea at 200 ppm when compared to untreated control under the same conditions, which showed extensive disease development: P-8, P-10, P-15, P-26, P-39, P-42, P-49, P-103, P-110

### Example B14: Pyrenophora teres / barley / leaf disc preventative (Net blotch)

Barley leaf segments cv. Hasso are placed on agar in a multiwell plate (24-well format) and sprayed with the formulated test compound diluted in water. The leaf segmens are inoculated with a spore suspension of the fungus 2 days after application. The inoculated leaf segments are incubated at 20 °C and 65% rh under a light regime of 12 h light / 12 h darkness in a climate cabinet and the activity of a compound is assessed as disease control compared to untreated when an appropriate level of disease damage appears in untreated check leaf segments (5 - 7 days after application). The following compounds gave at least 80% control of Pyrenophora teres at 200 ppm when compared to untreated control under the same conditions, which showed extensive disease development: P-2, P-3, P-4, P-5, P-6, P-8, P-9, P-10, P-11, P-15, P-16, P-19, P-20, P-21, P-22, P-23, P-24, P-25, P-26, P-29, P-30, P-31, P-32, P-34, P-35, P-36, P-42, P-43, P-44, P-45, P-48, P-49, P-50, P-52, P-53, P-56, P-61, P-63, P-64, P-69, P-73, P-75, P-79, P-80, P-82, P-83, P-84, P-85, P-101, P-102, P-105, P-107, P-110

### Example B15: Sclerotinia sclerotiorum / liquid culture (cottony rot)

Mycelia fragments of a newly grown liquid culture of the fungus are directly mixed into nutrient broth (PDB potato dextrose broth). After placing a (DMSO) solution of test compound into a microtiter plate (96-well format) the nutrient broth containing the fungal material is added. The test plates are incubated at 24 °C and the inhibition of growth is determined photometrically 3-4 days after application. The following compounds gave at least 80% control of Sclerotinia sclerotiorum at 20 ppm when compared to untreated control under the same conditions, which showed extensive disease development: P-4, P-9, P-10, P-16, P-24, P-25, P-26, P-29, P-30, P-31, P-34, P-35, P-42, P-43, P-49, P-73, P-82, P-84, P-101, P-102, P-106, P-107, P-110, P-118, and P-119

### Example B16: Mycosphaerella graminicola (Septoria tritici) / liquid culture (Septoria blotch)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is determined photometrically 4-5 days after application. The following compounds gave at least 80% control of Mycosphaerella graminicola at 20 ppm when compared to untreated control under the same conditions, which showed extensive disease development: P-1, P-2, P-4, P-5, P-6, P-7, P-9, P-10, P-11, P-13, P-14, P-15, P-16, P-17, P-18, P-19, P-20, P-21, P-24, P-25, P-26, P-28, P-29, P-30, P-31, P-32, P-33, P-34, P-35, P-36, P-38, P-39, P-41, P-42, P-43, P-46, P-47, P-48, P-49, P-50, P-55, P-57, P-61, P-63, P-64, P-66, P-67, P-68, P-69, P-73, P-75, P-77, P-78, P-79, P-80, P-81, P-82, P-83, P-84, P-85, P-88, P-89, P-91, P-94, P-95, P-97, P-98, P-99, P-101, P-102, P-103, P-104, P-106, P-107, P-108, P-109, P-110, P-112, P-115, P-116, P-117, P-118, and P-119.

Embodiment 1 is a compound of formula (I): 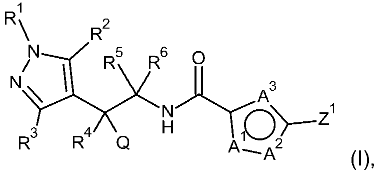 wherein R¹ is selected from the group consisting of hydrogen, C₁-C₄ alkyl, C₂-C₄ alkenyl, C₂-C₄ alkynyl and C₃-C₆ cycloalkyl; R² is selected from the group consisting of hydrogen, halogen, C₁-C₄ alkyl, C₂-C₄ alkenyl, C₂-C₄ alkynyl, C₁-C₄ haloalkyl, C₃-C₆ cycloalkyl, C₁-C₄ alkylcarbonyl, N-C₁-C₄ alkoxy-C₁-C₄ alkyl-carbonimidoyl, N-hydroxy-C₁-C₄ alkyl-carbonimidoyl and C₁-C₄ alkoxycarbonyl; R³ is selected from the group consisting of hydrogen, halogen and C₁-C₄ alkyl; R⁴ is selected from the group consisting of hydrogen, halogen, C₁-C₄ alkyl, cyano, C₁-C₄ alkylcarbonyl, C₁-C₄ alkoxycarbonyl, C₁-C₄ alkylaminocarbonyl and di(C₁-C₄ alkylamino)carbonyl; R⁵ and R⁶ are independently selected from the group consisting of hydrogen and C₁-C₄ alkyl; A¹, A² and A³ are independently selected from the group consisting of CR⁷, N, NR⁸, O and S, with the proviso that at least one of A¹, A² and A³ is selected from N, O and S, and that no more than one of A¹, A² and A³ is O or S; R⁷ and R⁸ are independently selected from the group consisting of hydrogen, C₁-C₄ alkyl, C₂-C₄ alkenyl and C₂-C₄ alkynyl; Q is selected from the group consisting of Q¹, Q², Q³, Q⁴ and Q⁵: 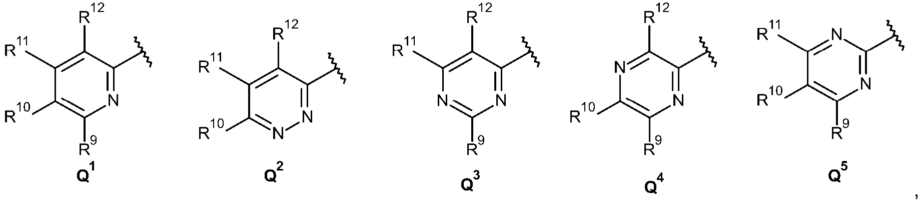 wherein R⁹, R¹⁰, R¹¹ and R¹² are independently selected from the group consisting of hydrogen, halogen, amino, hydroxy, carboxylic acid, cyano, C₁-C₄ alkyl, C₁-C₄ haloalkyl, C₁-C₄ alkoxy, C₁-C₄ haloalkoxy, C₂-C₄ alkenyloxy, C₂-C₄ alkynyloxy, C₁-C₄ alkylsulfanyl, C₁-C₄ alkylsulflnyl, C₁-C₄ alkylsulfonyl, C₁-C₄ alkoxy-C₁-C₄ alkyl, N-C₁₋₄alkylamino, N,N-diC₁₋₄alkylamino, C₁-C₆ alkoxycarbonyl, C₁-C₄ alkylcarbonyl, N-C₁-C₄ alkoxy-C₁-C₄alkyl-carbonimidoyl, N-hydroxy-C₁-C₄alkyl-carbonimidoyl, C₁-C₄alkylaminocarbonyl, di(C₁-C₄ alkylamino)carbonyl), C₁-C₄ alkylcarbonylamino, C₁-C₄ alkylsulfonylamino, trifluoromethylsulfonyloxy, phenyl, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl and C₁-C₄ alkoxy; and Z¹ is selected from the group consisting of C₁-C₄ alkyl, phenyl, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆ cycloalkyl are optionally substituted by 1, 2 or 3 substituents independently selected from halogen, cyano, C₁-C₄ alkyl, C₁-C₄ haloalkyl, C₁-C₄ alkoxy, C₁-C₄ haloalkoxy, C₁-C₄ alkylsulfanyl, C₁-C₄ alkylsulfinyl, C₁-C₄ alkylsulfonyl and C₂-C₄ alkynyl; or an agrochemically acceptable salt, stereoisomer, enantiomer, tautomer or N-oxide thereof.

Embodiment 2, wherein in the compound according to embodiment 1, R¹ is methyl, ethyl or isopropyl.

Embodiment 3, wherein in the compound according to embodiment 1 or embodiment 2, R² is selected from the group consisting of hydrogen, fluorine, chlorine and methyl.

Embodiment 4, wherein in the compound according to embodiment 1 to embodiment 3, R³ is selected from the group consisting of hydrogen, fluorine, chlorine and methyl.

Embodiment 5, wherein in the compound according to embodiment 1 to embodiment 4, R⁴ is selected from the group consisting of hydrogen, methyl, ethyl, isopropyl and cyano.

Embodiment 6, wherein in the compound according to embodiment 1 to embodiment 5, R⁵ and R⁶ are independently selected from the group consisting of hydrogen, methyl and ethyl.

Embodiment 7, wherein in the compound according to embodiment 1 to embodiment 6, Q is selected from Q¹, Q² and Q³:

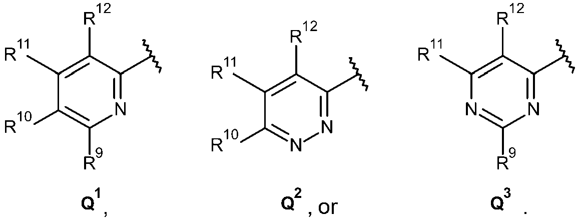

Embodiment 8, wherein in the compound according to embodiment 1 to 7, R⁹ and R¹⁰ are independently selected from the group consisting of hydrogen, halogen, amino, hydroxy, carboxylic acid, cyano, C₁-C₃ alkyl, C₁-C₂ haloalkyl, C₁-C₄ alkoxy, C₁-C₃ haloalkoxy, C₂-C₃ alkenyloxy, C₂-C₃ alkynyloxy, C₁-C₂ alkylsulfanyl, C₁-C₂ alkylsulflnyl, C₁-C₂ alkylsulfonyl, C₁-C₂ alkoxy-C₁-C₂ alkyl, N-C₁₋₄alkylamino, N,N-diC₁₋₄alkylamino, C₁-C₃ alkoxycarbonyl, C₁-C₂₋alkylcarbonyl, N-C₁-C₂ alkoxy-C₁-C₂ alkyl-carbonimidoyl, N-hydroxy-C₁-C₂ alkyl-carbonimidoyl, C₁-C₂ alkylaminocarbonyl, di(C₁-C₂ alkylamino)carbonyl, C₁-C₂-alkylcarbonylamino, C₁-C₂-alkylsulfonylamino, trifluoromethyl-sulfonyloxy, phenyl, 2-cyanophenyl, 3-cyanophenyl, 4-cyanophenyl, 2-methylphenyl, 3-methylphenyl, 4-methylphenyl, 3-chloropyrrol-1-yl, 4-(trifluoromethyl)pyrazol-1-yl, 3-(trifluoromethyl)pyrazol-1-yl, 3-cyanopyrazol-1-yl, 4-cyanopyrazol-1-yl, 5-chloropyrazol-1-yl, 4-chloropyrazol-1-yl, 3-chloropyrazol-1-yl, 5-fluoropyrazol-1-yl, 4-fluoropyrazol-1-yl, 3-fluoropyrazol-1-yl, 3,5-dimethylpyrazol-1-yl, 5-methylpyrazol-1-yl, 4-methylpyrazol-1-yl, 3-methylpyrazol-1-yl, pyrazol-1-yl, cyclopropyl and 1-cyanocyclopropyl; R¹¹ is selected from the group consisting of hydrogen, halogen, hydroxy, cyano, C₁-C₄ alkyl and C₁-C₄ alkoxy; and R¹² is selected from the group consisting of hydrogen, halogen and C₁-C₄ alkyl.

Embodiment 9, wherein in the compound according to embodiment 1 to 8, the compound of formula (I) is a compound of formula (II): 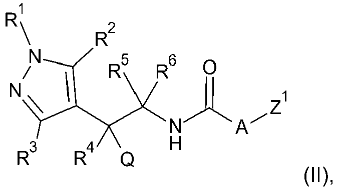 wherein R¹, R², R³, R⁴, R⁵, R⁶, Q and Z¹ correspond to the same definitions as for the compounds of formula (I) according to any one of embodiment 1 to 8, and A is selected from A-1, A-4, A-9, A-10, A-13, or A-15: 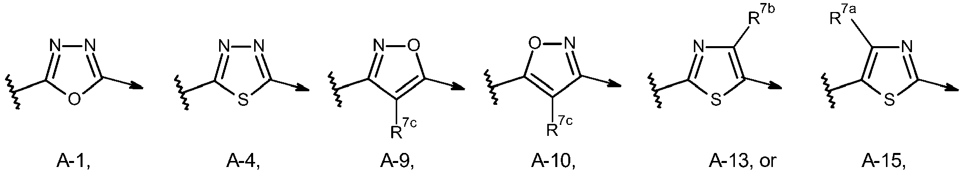 wherein the dotted line indicates the bond to the C(=O) group, and the arrow indicates the bond to the Z¹ group.

Embodiment 10, wherein in the compound according to embodiment 9, wherein A is selected from A-4, A-9 or A-10.

Embodiment 11, wherein in the compound according to embodiment 1 to 10, Z¹ is selected from 1-methylpyrazol-4-yl, 2,3,4-trifluorophenyl, 2,3-difluorophenyl, 3,4-difluorophenyl, 2,4,6-trifluorophenyl, 2,4-difluorophenyl, 2,5-difluorophenyl, 2-fluoro-4-methoxy-phenyl, 2-fluoro-4-methylsulfonyl-phenyl, 2-fluorophenyl, 3-fluorophenyl, 4-fluorophenyl, 2-furyl, 2-thienyl, 3-thienyl, 2-methylphenyl, 3-methylphenyl, 4-methylphenyl, 2-chlorophenyl, 3-chlorophenyl, 4-chlorophenyl, 3-methoxyphenyl, 4-ethynyl-2-fluorophenyl, 4-fluoro-2-methoxy-phenyl, cyclopropyl, 1-methylcyclopropyl, cyclobutyl, cyclohexyl, cyclopentyl, methyl, n-propyl and phenyl.

Embodiment 12 is an intermediate compound of formula (III) or a salt thereof: 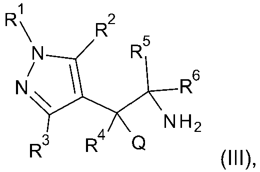 wherein R¹, R², R³, R⁴, R⁵, R⁶ and Q correspond to the same definitions as for the compounds according to any one of embodiments 1 to 8.

Embodiment 13 is an agrochemical composition comprising a fungicidally effective amount of a compound as defined in any one of embodiments 1 to 11.

Embodiment 14 is a method of controlling or preventing infestation of useful plants by phytopathogenic microorganisms, wherein a fungicidally effective amount of a compound as defined in any one of embodiments 1 to 11, is applied to the plants, to parts thereof or the locus thereof.

Embodiment 15 is a method of controlling or preventing infestation of useful plants by phytopathogenic microorganisms, wherein a composition comprising a compound as defined in any one of embodiments 1 to 11, is applied to the plants, to parts thereof or the locus thereof.

Embodiment 16 is the nontherapeutic use of a compound according to any one of embodiments 1 to 11 as a fungicide.

## Claims

1. A compound of formula (II): 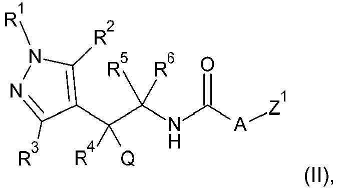 wherein
R¹ is methyl;
R² is selected from hydrogen, chloro or methyl;
R⁴ is selected from hydrogen, methyl or ethyl;
R³, R⁵ and R⁶ are hydrogen:
Q is Q1: 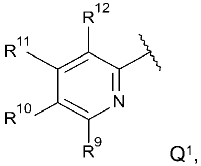
wherein the dotted line indicates the connection to the rest of the molecule: wherein
R⁹ is selected from chloro, bromo, cyano, -C(CH₃)=NOCH₃, or methyl;
R¹¹ is selected from hydrogen or cyano;
R¹⁰ is selected from hydrogen or methyl; and
R¹² is hydrogen;
A is selected from A-1, A-4, A-6, A-7, A-9 or A-10: 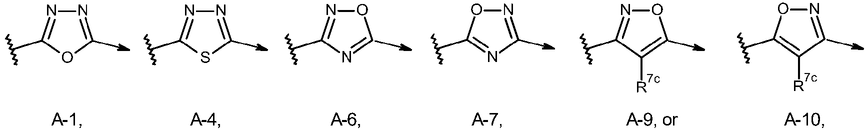
wherein the dotted line indicates the bond to the C(=O) group, and the arrow indicates the bond to the Z¹ group, and wherein R^{7c} is hydrogen; and
Z¹ is selected from 2,4-difluorophenyl, 2-fluorophenyl, 4-fluorophenyl or phenyl;
or an agrochemically acceptable salt, stereoisomer, enantiomer, tautomer or N-oxide thereof.

2. The compound according to claim 1, wherein R² is hydrogen, or chloro; R⁴ is hydrogen, or methyl; and Z¹ is 2,4-difluorophenyl.

3. The compound according the claim 1 or claim 2 wherein the compound is selected from N-[2-(6-chloro-2-pyridyl)-2-(1-methylpyrazol-4-yl)propyl]-3-(2,4-difluorophenyl)isoxazole-5-carboxamide (P-4), N-[2-(6-bromo-2-pyridyl)-2-(1-methylpyrazol-4-yl)propyl]-5-(2,4-difluorophenyl)-1,3,4-thiadiazole-2-carboxamide (P-5), N-[2-(6-chloro-2-pyridyl)-2-(1-methylpyrazol-4-yl)propyl]-5-(2,4-difluorophenyl)-1,3,4-thiadiazole-2-carboxamide (P-9), N-[2-(6-chloro-2-pyridyl)-2-(1-methyl-pyrazol-4-yl)propyl]-5-(2,4-difluorophenyl)isoxazole-3-carboxamide (P-10), N-[2-(6-chloro-2-pyridyl)-2-(1-methylpyrazol-4-yl)ethyl]-5-(2,4-difluorophenyl)isoxazole-3-carboxamide (P-13), N-[2-(6-cyano-2-pyridyl)-2-(1-methylpyrazol-4-yl)propyl]-5-(2,4-difluorophenyl)isoxazole-3-carboxamide (P-15), 5-(2,4-difluoro-phenyl)-N-[2-(1-methylpyrazol-4-yl)-2-(6-methyl-2-pyridyl)-propyl]isoxazole-3-carboxamide (P-16), N-[2-(6-bromo-2-pyridyl)-2-(1-methylpyrazol-4-yl)propyl]-5-(2,4-difluorophenyl)isoxazole-3-carboxamide (P-24), N-[2-(5-chloro-1-methyl-pyrazol-4-yl)-2-(6-chloro-2-pyridyl)propyl]-5-(2,4-difluorophenyl)-1,3,4-thiadiazole-2-carboxamide (P-25), N-[2-(5-chloro-1-methyl-pyrazol-4-yl)-2-(6-chloro-2-pyridyl)propyl]-5-(2,4-difluorophenyl)-isoxazole-3-carboxamide (P-26), N-[(2S)-2-(6-chloro-2-pyridyl)-2-(1-methylpyrazol-4-yl)propyl]-5-(2,4-difluorophenyl)isoxazole-3-carboxamide (P-29), N-[(2S)-2-(6-chloro-2-pyridyl)-2-(1-methylpyrazol-4-yl)propyl]-5-(2,4-difluorophenyl)isoxazole-3-carboxamide (P-30), N-[2-(6-chloro-2-pyridyl)-2-(1-methylpyrazol-4-yl)butyl]-5-(2,4-difluorophenyl)-isoxazole-3-carboxamide (P-31), N-[(2S)-2-(6-chloro-2-pyridyl)-2-(1-methylpyrazol-4-yl)propyl]-5-(2,4-difluoro-phenyl)-1,3,4-thiadiazole-2-carboxamide (P-34), N-[(2R)-2-(6-chloro-2-pyridyl)-2-(1-methylpyrazol-4-yl)propyl]-5-(2,4-difluorophenyl)-1,3,4-thiadiazole-2-carboxamide (P-35), N-[2-(6-chloro-4-cyano-2-pyridyl)-2-(1-methyl-pyrazol-4-yl)propyl]-5-(2,4-difluorophenyl)-isoxazole-3-carboxamide (P-41), N-[2-(6-chloro-2-pyridyl)-2-(1-methylpyrazol-4-yl)propyl]-3-(2,4-difluorophenyl)-isoxazole-5-carboxamide (P-42), N-[2-(6-chloro-2-pyridyl)-2-(1-methylpyrazol-4-yl)propyl]-3-(2,4-difluorophenyl)isoxazole-5-carboxamide (P-43), 5-(2,4-difluorophenyl)-N-[2-[6-[(E)-N-methoxy-C-methyl-carbonimidoyl]-2-pyridyl]-2-(1-methylpyrazol-4-yl)propyl]isoxazole-3-carboxamide (P-49), N-[2-(5-chloro-1-methyl-pyrazol-4-yl)-2-(6-chloro-2-pyridyl)ethyl]-5-(2,4-difluorophenyl)isoxazole-3-carboxamide (P-69), N-[2-(5-chloro-1-methyl-pyrazol-4-yl)-2-(6-methyl-2-pyridyl)propyl]-5-(2,4-difluorophenyl)isoxazole-3-carboxamide (P-73), N-[2-(5-chloro-1-methyl-pyrazol-4-yl)-2-(6-methyl-2-pyridyl)propyl]-5-(2,4-difluorophenyl)-1,3,4-thiadiazole-2-carboxamide (P-82), N-[2-(6-chloro-2-pyridyl)-2-(1-methyl-pyrazol-4-yl)propyl]-5-(2,4-difluorophenyl)-1,2,4-oxadiazole-3-carboxamide (P-83), N-[2-(6-chloro-2-pyridyl)-2-(1-methylpyrazol-4-yl)propyl]-3-(2,4-difluorophenyl)-1,2,4-oxadiazole-5-carboxamide (P-84), N-[2-(6-chloro-2-pyridyl)-2-(1-methylpyrazol-4-yl)propyl]-5-(2,4-difluorophenyl)-1,3,4-oxadiazole-2-carboxamide (P-101), N-[2-(6-bromo-5-methyl-2-pyridyl)-2-(1-methylpyrazol-4-yl)propyl]-5-(2,4-difluorophenyl)isoxazole-3-carboxamide (P-110), N-[2-(5-chloro-1-methyl-pyrazol-4-yl)-2-(6-chloro-2-pyridyl)ethyl]-5-(2,4-difluorophenyl)-1,3,4-thiadiazole-2-carboxamide (P-116), N-[2-(5-chloro-1-methyl-pyrazol-4-yl)-2-(6-cyano-2-pyridyl)ethyl]-5-(2,4-difluorophenyl)-isoxazole-3-carboxamide (P-117),N-[2-(5-chloro-1-methyl-pyrazol-4-yl)-2-(6-cyano-2-pyridyl)-propyl]-5-(2,4-difluorophenyl)-1,3,4-thiadiazole-2-carboxamide (P-118), or N-[2-(5-chloro-1-methyl-pyrazol-4-yl)-2-(6-cyano-2-pyridyl)propyl]-5-(2,4-difluorophenyl)isoxazole-3-carboxamide (P-119).

4. The compound according to claim 1 or 2, wherein the compound is selected from N-[2-(6-chloro-2-pyridyl)-2-(1-methylpyrazol-4-yl)propyl]-3-(2,4-difluorophenyl)isoxazole-5-carboxamide (P-4), N-[2-(6-bromo-2-pyridyl)-2-(1-methylpyrazol-4-yl)propyl]-5-(2,4-difluorophenyl)-1,3,4-thiadiazole-2-carboxamide (P-5), N-[2-(6-chloro-2-pyridyl)-2-(1-methylpyrazol-4-yl)propyl]-5-(2,4-difluorophenyl)-1,3,4-thiadiazole-2-carboxamide (P-9), N-[2-(6-chloro-2-pyridyl)-2-(1-methylpyrazol-4-yl)propyl]-5-(2,4-difluorophenyl)isoxazole-3-carboxamide (P-10), N-[2-(6-cyano-2-pyridyl)-2-(1-methylpyrazol-4-yl)propyl]-5-(2,4-difluorophenyl)isoxazole-3-carboxamide (P-15), 5-(2,4-difluorophenyl)-N-[2-(1-methylpyrazol-4-yl)-2-(6-methyl-2-pyridyl)propyl]isoxazole-3-carboxamide (P-16), N-[2-(5-chloro-1-methyl-pyrazol-4-yl)-2-(6-chloro-2-pyridyl)propyl]-5-(2,4-difluorophenyl)-1,3,4-thiadiazole-2-carboxamide (P-25), N-[2-(5-chloro-1-methyl-pyrazol-4-yl)-2-(6-chloro-2-pyridyl)propyl]-5-(2,4-difluorophenyl)isoxazole-3-carboxamide (P-26), N-[(2S)-2-(6-chloro-2-pyridyl)-2-(1-methylpyrazol-4-yl)propyl]-5-(2,4-difluorophenyl)isoxazole-3-carboxamide (P-29), N-[(2S)-2-(6-chloro-2-pyridyl)-2-(1-methylpyrazol-4-yl)propyl]-5-(2,4-difluorophenyl)-isoxazole-3-carboxamide (P-30), N-[2-(6-chloro-2-pyridyl)-2-(1-methylpyrazol-4-yl)butyl]-5-(2,4-difluorophenyl)-isoxazole-3-carboxamide (P-31), N-[(2S)-2-(6-chloro-2-pyridyl)-2-(1-methylpyrazol-4-yl)propyl]-5-(2,4-difluorophenyl)-1,3,4-thiadiazole-2-carboxamide (P-34), N-[(2R)-2-(6-chloro-2-pyridyl)-2-(1-methylpyrazol-4-yl)propyl]-5-(2,4-difluoro-phenyl)-1,3,4-thiadiazole-2-carboxamide (P-35), N-[2-(6-chloro-2-pyridyl)-2-(1-methylpyrazol-4-yl)propyl]-3-(2,4-difluoro-phenyl)isoxazole-5-carboxamide (P-42), N-[2-(6-chloro-2-pyridyl)-2-(1-methylpyrazol-4-yl)propyl]-3-(2,4-difluorophenyl)isoxazole-5-carboxamide (P-43), 5-(2,4-difluoro-phenyl)-N-[2-[6-[(E)-N-methoxy-C-methyl-carbonimidoyl]-2-pyridyl]-2-(1-methylpyrazol-4-yl)propyl]-isoxazole-3-carboxamide (P-49), N-[2-(5-chloro-1-methyl-pyrazol-4-yl)-2-(6-methyl-2-pyridyl)propyl]-5-(2,4-difluorophenyl)isoxazole-3-carboxamide (P-73), N-[2-(5-chloro-1-methyl-pyrazol-4-yl)-2-(6-methyl-2-pyridyl)propyl]-5-(2,4-difluorophenyl)-1,3,4-thiadiazole-2-carboxamide (P-82), N-[2-(6-chloro-2-pyridyl)-2-(1-methylpyrazol-4-yl)propyl]-3-(2,4-difluorophenyl)-1,2,4-oxadiazole-5-carboxamide (P-84), or N-[2-(6-bromo-5-methyl-2-pyridyl)-2-(1-methylpyrazol-4-yl)propyl]-5-(2,4-difluorophenyl)isoxazole-3-carboxamide (P-110).

5. The compound according to claim 1 or 2, wherein the compound is selected from N-[2-(6-chloro-2-pyridyl)-2-(1-methylpyrazol-4-yl)propyl]-3-(2,4-difluorophenyl)isoxazole-5-carboxamide (P-4), N-[2-(6-chloro-2-pyridyl)-2-(1-methylpyrazol-4-yl)propyl]-5-(2,4-difluorophenyl)isoxazole-3-carboxamide (P-10), N-[2-(5-chloro-1-methyl-pyrazol-4-yl)-2-(6-chloro-2-pyridyl)propyl]-5-(2,4-difluorophenyl)-isoxazole-3-carboxamide (P-26), N-[(2R)-2-(6-chloro-2-pyridyl)-2-(1-methyl-pyrazol-4-yl)propyl]-5-(2,4-difluorophenyl)-1,3,4-thiadiazole-2-carboxamide (P-35), N-[2-(6-chloro-2-pyridyl)-2-(1-methyl-pyrazol-4-yl)propyl]-3-(2,4-difluorophenyl)isoxazole-5-carboxamide (P-42), 5-(2,4-difluorophenyl)-N-[2-[6-[(E)-N-methoxy-C-methyl-carbonimidoyl]-2-pyridyl]-2-(1-methyl-pyrazol-4-yl)propyl]isoxazole-3-carboxamide (P-49), or N-[2-(5-chloro-1-methyl-pyrazol-4-yl)-2-(6-methyl-2-pyridyl)propyl]-5-(2,4-difluorophenyl)isoxazole-3-carboxamide (P-73).

6. An agrochemical composition comprising a fungicidally effective amount of a compound of formula (I) as defined in any one of claims 1 to 5.

7. A method of controlling or preventing infestation of useful plants by phytopathogenic microorganisms, wherein a fungicidally effective amount of a compound of formula (I) as defined in any one of claims 1 to 5, or a composition comprising the compound of formula (I), is applied to the plants, to parts thereof or the locus thereof.

8. Use of a compound according to any one of claims 1 to 5 as a fungicide.
